# EUROPEAN PATENT APPLICATION

(11) **EP 2 650 367 A1**
(43) Date of publication of application: **16.10.2013**
(21) Application number: 11846511.1
(22) Date of filing: 05.12.2011
(51) Int. Cl.: C12N 15/09, A61K 39/395, A61P 1/04, A61P 17/06, A61P 25/00, A61P 35/00, C07K 16/28, C12N 1/15, C12N 1/19, C12N 1/21, C12N 5/10, C12P 21/08

(54) **HUMAN MONOCLONAL ANTIBODY**

(30) Priority: 06.12.2010 JP 2010272046
(71) Applicant: Dainippon Sumitomo Pharma Co., Ltd., Osaka 541-8524 (JP)
(72) Inventor: WATANABE, Takamasa, Osaka-shi Osaka 554-0022 (JP); YOSHIMA, Tadahiko, Osaka-shi Osaka 554-0022 (JP); MATTSSON, Mikael, SE-246 50 Loddekopinge (SE); SARNEFALT, Anna, SE-212 22 Malmo (SE); HASEZAKI, Takuya, Osaka-shi Osaka 554-0022 (JP)
(74) Representative: Vossius & Partner
(86) International application number: PCT/JP2011/078110
(87) International publication number: WO 2012/077649

(57) **Abstract**

The present invention provides an anti-CD81 antibody that is useful as a pharmaceutical product for human use. Specifically, the present invention provides an anti-human CD81 antibody that can bind to a peptide domain consisting of an amino acid sequence represented by amino acid Nos. 80 to 175 in the amino acid sequence represented by SEQ ID NO: 22.

## Description

### Technical Field

The present invention relates to a human monoclonal antibody molecule. Specifically, it relates to a human antibody molecule against human CD81 and a pharmaceutical composition containing the molecule as an active ingredient.

### Background Art

Bowels are organs which digest and absorb nutrients and water essential for activities of life of organisms. Meanwhile, they are also organs which have an immunodefense performance for excluding foreign matters such as pathogens and keep life conservation by controlling contradictory qualities in a well-balanced manner. It is however known that when the balance of these functions becomes abnormal, this dynamic equilibrium is broken to induce various bowel diseases. Especially, inflammatory bowel diseases (abbreviated as IBD), of which patients have been increased in number in recent years, are associated with abnormalities in digestive organs such as abdominal pain, diarrhea, mucous and bloody stool and the like and, in view of pathogenic states thereof, grouped into ulcerative colitis and Crohn's disease.

Ulcerative colitis is a disease mainly showing diffuse bowel mucosal inflammation restricted to the large intestine, where repeated inflammation leads to the onset of colorectal cancer, surgery is often necessary, and postoperative problems of increased frequency of defecation, stool leakage and onset of pouchitis are caused. Crohn's disease is a disease showing lesion spreading from the small intestine to the large intestine, and intense, discontinuous all layer inflammation starting from the submucosal layer, where repeated inflammation leads to the intestinal complications (stenosis, fistula, abscess) that require operation (Non-patent document 1).

In recent years, it has been known that an anti-TNF-α antibody is effective as a therapeutic agent of Crohn's disease and ulcerative colitis (Non-patent document 2). Also, an anti-α4 integrin antibody Natalizumab has been reported to be effective as a therapeutic agent of Crohn's disease (Non-patent document 3). Nevertheless, in the current therapies including the antibodies, 40-60% of IBD patients has not yet received a satisfactory medical treatment. Accordingly, the development of an effective therapeutic agent has been in high demand in a medical care (Non-patent document 4).

CD81 is a cell surface molecule of 26 kDa, which is expressed in wide-ranging cells. It has an activity of decreasing a threshold of B cell activation by forming a complex with CD21, CD19 and Leu 13 in a B cell. In a T cell, it is associated with CD4 and CD8 to transduce stimulatory signal into cells. In view of these matters, CD81 is considered to have a significant role in an immune response to a heterologous antigen. Moreover, it is involved in various integrins physiologically and functionally to activate VLA-4 (α4β1 integrin) in a B cell or LFA-1 (αLβ2 integrin) in a thymocyte.

As a disease associated with CD81, hepatitis C is known (Non-patent document 5).

In recent years, it has been reported that anti-CD81 antibody is useful for the treatment of IBD (Patent document 1). IBD associated with T cell migration (Non-patent documents 3, 6 and 7). As other diseases associated with T cell migration, multiple sclerosis and psoriasis are known (Non-patent documents 3, 8 and 9).

To be specific, it has been reported that bowel mucosa layer T cells or peripheral blood T cells of a patient suffering from IBD such as Crohn's disease or ulcerative colitis highly express a chemokine receptor CXCR4 and exhibit a strong chemotactic response to a chemokine CXCL12 (Non-patent document 6), and that colitis is cured by administering a CXCR4 inhibitor to an IBD model, dextran sulfate-induced mouse colitis model (Non-patent document 7), and that an anti-α4 integrin antibody Natalizumab, which treats IBD by suppressing T cell migration, has been approved as a pharmaceutical product (Non-patent document 3).

It has also been reported that T cell migration is important for the pathology of an animal model of multiple sclerosis, experimental autoimmune encephalomyelitis (EAE) mouse (Non-patent document 8). Natalizumab is thought to exert its therapeutic efficacy by blocking the α4 integrin-mediated adhesion of circulating T cells to the blood-brain barrier in EAE mice (Non-patent document 3). Natalizumab is also effective for the treatment of multiple sclerosis.

Furthermore, it has been reported that T cells abundantly accumulate in psoriatic skin and that an anti-LFA-1 antibody Efalizumab (trade name: Raptiva), which suppresses T cell migration, is effective for the treatment of psoriasis (Non-patent document 9).

There arise various problems based on the species difference when clinical application of an anti-CD81 antibody to human is desired. For example, administration of a mouse antibody to human may be limited by short serum half-life, failure to trigger certain kinds of human effector function and induction of undesirable human immune response to the mouse antibody ("human anti-mouse antibody" (HAMA) reaction) (Non-patent documents 10 and 11). Moreover, even an anti-TNFα antibody (Remicade), which is a chimeric molecule obtained by binding the variable (V) region of a rodent antibody with the constant (C) region of a human antibody, may induce a human anti-chimeric antibody (HACA) and cause an infusion reaction or loss of drug efficacy (Non-patent document 12).

### Document List

### Patent Document

Patent document 1: WO 2005/021792

### Non-patent Documents

Non-patent document 1: Inflamm. Bowel. Dis., 8, 244-250, 2002
Non-patent document 2: N. Engl. J. Med., 353, 2462-2476, 2005
Non-patent document 3: J. Clin. Invest. , 118, 825-826, 2008
Non-patent document 4: J. Clin. Gastroenterol., 41, 799-809, 2007
Non-patent document 5: Science, 282, 938-941, 1998
Non-patent document 6: Inflamm. Bowel Dis., 16(4), 583-592, 2010
Non-patent document 7: J. Pharmacol. Exp. Ther., 327(2), 383-392, 2008
Non-patent document 8: J. Neuroimmunol., 60, 17-28, 1995
Non-patent document 9: Expert Opinion on Biological Therapy, 3(2), 361-370, 2003
Non-patent document 10: Blood, 62, 988-995, 1983
Non-patent document 11: Cancer Res., 45, 879-885, 1985
Non-patent document 12: Current Gastroenterology Reports, 5(6), 501-505, 2003

### Summary of the Invention

### Problems to be Solved by the Invention

Under the circumstances, an anti-CD81 antibody that can be used as a pharmaceutical product is desired. However, such antibody is not known, and therefore, the problem to be solved by the present invention is provision of an anti-CD81 antibody usable as a pharmaceutical product for human.

### Means of Solving the Problems

In an attempt to solve the above-mentioned problem, the present inventors have prepared fully human anti-CD81 antibodies from a human complementarity-determining region (CDR) library by a phage library method, evaluated the region of human CD81 to which the antibodies bind, and found that antibodies bound to a certain region of CD81 show superior efficacy as well as high safety for human body, which resulted in the completion of the present invention. The present invention provides a human monoclonal antibody to human CD81. Furthermore, the present inventors obtained new findings that the anti-CD81 antibodies suppressed T cell migration, which revealed that the antibody of the present invention was useful for the prophylaxis, improvement or treatment of not only inflammatory bowel diseases such as Crohn's disease and ulcerative colitis but also diseases associated with T cell migration such as multiple sclerosis and psoriasis. Furthermore, the present inventors found that the antibody of the present invention is not only capable of merely binding to CD81-expressing cancer cells, but also has a cytotoxic effect, due to its complement-dependent cytotoxicity (CDC), on cancer cells to which it has bound, and is therefore also useful in preventing, ameliorating or treating cancers caused by CD81-expressing cancer cells, including hematological cancers (hematologic cancers, blood cancers, hematologic(al) malignancies).

Accordingly, the present invention is as follows.
[1] An anti-human CD81 antibody capable of binding to a peptide region consisting of the amino acid sequence of the amino acid numbers 80 to 175 in the amino acid sequence shown in SEQ ID NO:22.
[2] The antibody of [1], wherein the peptide region consists of the amino acid sequence of the amino acid numbers 113 to 175.
[3] The antibody of [1] or [2], wherein the binding affinity of the antibody to at least one human CD81 variant selected from the group consisting of the following (1) to (13) is less than 40% of that to the human CD81 having the amino acid sequence shown in SEQ ID NO:22.
   (1) CD81 variant having the amino acid sequence shown in SEQ ID NO:22 wherein tyrosine at the amino acid number 127 is substituted with phenylalanine or tryptophan;
   (2) CD81 variant having the amino acid sequence shown in SEQ ID NO:22 wherein alanine at the amino acid number 130 is substituted with threonine or valine;
   (3) CD81 variant having the amino acid sequence shown in SEQ ID NO:22 wherein valine at the amino acid number 135 is substituted with alanine or leucine;
   (4) CD81 variant having the amino acid sequence shown in SEQ ID NO:22 wherein aspartic acid at the amino acid number 137 is substituted with alanine or glutamic acid;
   (5) CD81 variant having the amino acid sequence shown in SEQ ID NO:22 wherein alanine at the amino acid number 143 is substituted with threonine or valine;
   (6) CD81 variant having the amino acid sequence shown in SEQ ID NO:22 wherein histidine at the amino acid number 151 is substituted with alanine or arginine;
   (7) CD81 variant having the amino acid sequence shown in SEQ ID NO:22 wherein leucine at the amino acid number 154 is substituted with alanine or isoleucine;
   (8) CD81 variant having the amino acid sequence shown in SEQ ID NO:22 wherein glycine at the amino acid number 158 is substituted with alanine or serine;
   (9) CD81 variant having the amino acid sequence shown in SEQ ID NO:22 wherein alanine at the amino acid number 164 is substituted with threonine or valine;
   (10) CD81 variant having the amino acid sequence shown in SEQ ID NO:22 wherein serine at the amino acid number 168 is substituted with alanine or threonine;
   (11) CD81 variant having the amino acid sequence shown in SEQ ID NO:22 wherein valine at the amino acid number 169 is substituted with alanine or leucine;
   (12) CD81 variant having the amino acid sequence shown in SEQ ID NO:22 wherein leucine at the amino acid number 170 is substituted with alanine or isoleucine; and
   (13) CD81 variant having the amino acid sequence shown in SEQ ID NO:22 wherein asparagine at the amino acid number 172 is substituted with alanine or glutamine.
[4] The antibody of any one of [1] to [3], wherein the binding affinity of the antibody to each of the above-identified human CD81 variants (9) and (11) is less than 40% of that to the human CD81 having the amino acid sequence shown in SEQ ID NO:22.
[5] An antibody having a binding property equivalent to that of the antibody of any one of [1] to [4], or binding to the human CD81 having the amino acid sequence shown in SEQ ID NO:22 competitively with the antibody of any one of [1] to [4].
[6] An antibody binding to the human CD81 having the amino acid sequence shown in SEQ ID NO:22 competitively with the antibody of any one of [1] to [4], and having a suppressive effect of T cell migration.
[7] An anti-human CD81 antibody, which comprises all 6 CDRs described in any one of the following groups 1 to 24.
   Group 1
      (a-1) a CDR comprising the amino acid sequence shown in SEQ ID NO:1,
      (b-1) a CDR comprising the amino acid sequence shown in SEQ ID NO:2,
      (c-1) a CDR comprising the amino acid sequence shown in SEQ ID NO:3,
      (d-1) a CDR comprising the amino acid sequence shown in SEQ ID NO:4,
      (e-1) a CDR comprising the amino acid sequence shown in SEQ ID NO:5, and
      (f-1) a CDR comprising the amino acid sequence shown in SEQ ID NO:6
   Group 2
      (a-2) a CDR comprising the amino acid sequence shown in SEQ ID NO:1,
      (b-2) a CDR comprising the amino acid sequence shown in SEQ ID NO:2,
      (c-2) a CDR comprising the amino acid sequence shown in SEQ ID NO:37,
      (d-2) a CDR comprising the amino acid sequence shown in SEQ ID NO:4,
      (e-2) a CDR comprising the amino acid sequence shown in SEQ ID NO:5, and
      (f-2) a CDR comprising the amino acid sequence shown in SEQ ID NO:6
   Group 3
      (a-3) a CDR comprising the amino acid sequence shown in SEQ ID NO:1,
      (b-3) a CDR comprising the amino acid sequence shown in SEQ ID NO:2,
      (c-3) a CDR comprising the amino acid sequence shown in SEQ ID NO:40,
      (d-3) a CDR comprising the amino acid sequence shown in SEQ ID NO:4,
      (e-3) a CDR comprising the amino acid sequence shown in SEQ ID NO:5, and
      (f-3) a CDR comprising the amino acid sequence shown in SEQ ID NO:6
   Group 4
      (a-4) a CDR comprising the amino acid sequence shown in SEQ ID NO:1,
      (b-4) a CDR comprising the amino acid sequence shown in SEQ ID NO:2,
      (c-4) a CDR comprising the amino acid sequence shown in SEQ ID NO:43,
      (d-4) a CDR comprising the amino acid sequence shown in SEQ ID NO:4,
      (e-4) a CDR comprising the amino acid sequence shown in SEQ ID NO:5, and
      (f-4) a CDR comprising the amino acid sequence shown in SEQ ID NO:6
   Group 5
      (a-5) a CDR comprising the amino acid sequence shown in SEQ ID NO:1,
      (b-5) a CDR comprising the amino acid sequence shown in SEQ ID NO:2,
      (c-5) a CDR comprising the amino acid sequence shown in SEQ ID NO:46,
      (d-5) a CDR comprising the amino acid sequence shown in SEQ ID NO:4,
      (e-5) a CDR comprising the amino acid sequence shown in SEQ ID NO:5, and
      (f-5) a CDR comprising the amino acid sequence shown in SEQ ID NO:6
   Group 6
      (a-6) a CDR comprising the amino acid sequence shown in SEQ ID NO:1,
      (b-6) a CDR comprising the amino acid sequence shown in SEQ ID NO:2,
      (c-6) a CDR comprising the amino acid sequence shown in SEQ ID NO:49,
      (d-6) a CDR comprising the amino acid sequence shown in SEQ ID NO:4,
      (e-6) a CDR comprising the amino acid sequence shown in SEQ ID NO:5, and
      (f-6) a CDR comprising the amino acid sequence shown in SEQ ID NO:6
   Group 7
      (a-7) a CDR comprising the amino acid sequence shown in SEQ ID NO:1,
      (b-7) a CDR comprising the amino acid sequence shown in SEQ ID NO:2,
      (c-7) a CDR comprising the amino acid sequence shown in SEQ ID NO:52,
      (d-7) a CDR comprising the amino acid sequence shown in SEQ ID NO:4,
      (e-7) a CDR comprising the amino acid sequence shown in SEQ ID NO:5, and
      (f-7) a CDR comprising the amino acid sequence shown in SEQ ID NO:6
   Group 8
      (a-8) a CDR comprising the amino acid sequence shown in SEQ ID NO:1,
      (b-8) a CDR comprising the amino acid sequence shown in SEQ ID NO:2,
      (c-8) a CDR comprising the amino acid sequence shown in SEQ ID NO:43,
      (d-8) a CDR comprising the amino acid sequence shown in SEQ ID NO:4,
      (e-8) a CDR comprising the amino acid sequence shown in SEQ ID NO:5, and
      (f-8) a CDR comprising the amino acid sequence shown in SEQ ID NO:55
   Group 9
      (a-9) a CDR comprising the amino acid sequence shown in SEQ ID NO:60,
      (b-9) a CDR comprising the amino acid sequence shown in SEQ ID NO:2,
      (c-9) a CDR comprising the amino acid sequence shown in SEQ ID NO:3,
      (d-9) a CDR comprising the amino acid sequence shown in SEQ ID NO:4,
      (e-9) a CDR comprising the amino acid sequence shown in SEQ ID NO:5, and
      (f-9) a CDR comprising the amino acid sequence shown in SEQ ID NO:61
   Group 10
      (a-10) a CDR comprising the amino acid sequence shown in SEQ ID NO:1,
      (b-10) a CDR comprising the amino acid sequence shown in SEQ ID NO:2,
      (c-10) a CDR comprising the amino acid sequence shown in SEQ ID NO:66,
      (d-10) a CDR comprising the amino acid sequence shown in SEQ ID NO:4,
      (e-10) a CDR comprising the amino acid sequence shown in SEQ ID NO:5, and
      (f-10) a CDR comprising the amino acid sequence shown in SEQ ID NO:6
   Group 11
      (a-11) a CDR comprising the amino acid sequence shown in SEQ ID NO:1,
      (b-11) a CDR comprising the amino acid sequence shown in SEQ ID NO:2,
      (c-11) a CDR comprising the amino acid sequence shown in SEQ ID NO:3,
      (d-11) a CDR comprising the amino acid sequence shown in SEQ ID NO:4,
      (e-11) a CDR comprising the amino acid sequence shown in SEQ ID NO:69, and
      (f-11) a CDR comprising the amino acid sequence shown in SEQ ID NO:70
   Group 12
      (a-12) a CDR comprising the amino acid sequence shown in SEQ ID NO:60,
      (b-12) a CDR comprising the amino acid sequence shown in SEQ ID NO:2,
      (c-12) a CDR comprising the amino acid sequence shown in SEQ ID NO:66,
      (d-12) a CDR comprising the amino acid sequence shown in SEQ ID NO:4,
      (e-12) a CDR comprising the amino acid sequence shown in SEQ ID NO:5, and
      (f-12) a CDR comprising the amino acid sequence shown in SEQ ID NO:6
   Group 13
      (a-13) a CDR comprising the amino acid sequence shown in SEQ ID NO:1,
      (b-13) a CDR comprising the amino acid sequence shown in SEQ ID NO:2,
      (c-13) a CDR comprising the amino acid sequence shown in SEQ ID NO:3,
      (d-13) a CDR comprising the amino acid sequence shown in SEQ ID NO:77,
      (e-13) a CDR comprising the amino acid sequence shown in SEQ ID NO:5, and
      (f-13) a CDR comprising the amino acid sequence shown in SEQ ID NO:6
   Group 14
      (a-14) a CDR comprising the amino acid sequence shown in SEQ ID NO:80,
      (b-14) a CDR comprising the amino acid sequence shown in SEQ ID NO:2,
      (c-14) a CDR comprising the amino acid sequence shown in SEQ ID NO:3,
      (d-14) a CDR comprising the amino acid sequence shown in SEQ ID NO:4,
      (e-14) a CDR comprising the amino acid sequence shown in SEQ ID NO:81, and
      (f-14) a CDR comprising the amino acid sequence shown in SEQ ID NO:6
   Group 15
      (a-15) a CDR comprising the amino acid sequence shown in SEQ ID NO:1,
      (b-15) a CDR comprising the amino acid sequence shown in SEQ ID NO:2,
      (c-15) a CDR comprising the amino acid sequence shown in SEQ ID NO:66,
      (d-15) a CDR comprising the amino acid sequence shown in SEQ ID NO:4,
      (e-15) a CDR comprising the amino acid sequence shown in SEQ ID NO:5, and
      (f-15) a CDR comprising the amino acid sequence shown in SEQ ID NO:6
   Group 16
      (a-16) a CDR comprising the amino acid sequence shown in SEQ ID NO:1,
      (b-16) a CDR comprising the amino acid sequence shown in SEQ ID NO:2,
      (c-16) a CDR comprising the amino acid sequence shown in SEQ ID NO:90,
      (d-16) a CDR comprising the amino acid sequence shown in SEQ ID NO:4,
      (e-16) a CDR comprising the amino acid sequence shown in SEQ ID NO:5, and
      (f-16) a CDR comprising the amino acid sequence shown in SEQ ID NO:6
   Group 17
      (a-17) a CDR comprising the amino acid sequence shown in SEQ ID NO:1,
      (b-17) a CDR comprising the amino acid sequence shown in SEQ ID NO:2,
      (c-17) a CDR comprising the amino acid sequence shown in SEQ ID NO:52,
      (d-17) a CDR comprising the amino acid sequence shown in SEQ ID NO:4,
      (e-17) a CDR comprising the amino acid sequence shown in SEQ ID NO:5, and
      (f-17) a CDR comprising the amino acid sequence shown in SEQ ID NO:93
   Group 18
      (a-18) a CDR comprising the amino acid sequence shown in SEQ ID NO:98,
      (b-18) a CDR comprising the amino acid sequence shown in SEQ ID NO:2,
      (c-18) a CDR comprising the amino acid sequence shown in SEQ ID NO:3,
      (d-18) a CDR comprising the amino acid sequence shown in SEQ ID NO:4,
      (e-18) a CDR comprising the amino acid sequence shown in SEQ ID NO:5, and
      (f-18) a CDR comprising the amino acid sequence shown in SEQ ID NO:99
   Group 19
      (a-19) a CDR comprising the amino acid sequence shown in SEQ ID NO:60,
      (b-19) a CDR comprising the amino acid sequence shown in SEQ ID NO:2,
      (c-19) a CDR comprising the amino acid sequence shown in SEQ ID NO:3,
      (d-19) a CDR comprising the amino acid sequence shown in SEQ ID NO:4,
      (e-19) a CDR comprising the amino acid sequence shown in SEQ ID NO:5, and
      (f-19) a CDR comprising the amino acid sequence shown in SEQ ID NO:99
   Group 20
      (a-20) a CDR comprising the amino acid sequence shown in SEQ ID NO:1,
      (b-20) a CDR comprising the amino acid sequence shown in SEQ ID NO:2,
      (c-20) a CDR comprising the amino acid sequence shown in SEQ ID NO:90,
      (d-20) a CDR comprising the amino acid sequence shown in SEQ ID NO:4,
      (e-20) a CDR comprising the amino acid sequence shown in SEQ ID NO:5, and
      (f-20) a CDR comprising the amino acid sequence shown in SEQ ID NO:6
   Group 21
      (a-21) a CDR comprising the amino acid sequence shown in SEQ ID NO:1,
      (b-21) a CDR comprising the amino acid sequence shown in SEQ ID NO:2,
      (c-21) a CDR comprising the amino acid sequence shown in SEQ ID NO:3,
      (d-21) a CDR comprising the amino acid sequence shown in SEQ ID NO:4,
      (e-21) a CDR comprising the amino acid sequence shown in SEQ ID NO:5, and
      (f-21) a CDR comprising the amino acid sequence shown in SEQ ID NO:55
   Group 22
      (a-22) a CDR comprising the amino acid sequence shown in SEQ ID NO:1,
      (b-22) a CDR comprising the amino acid sequence shown in SEQ ID NO:2,
      (c-22) a CDR comprising the amino acid sequence shown in SEQ ID NO:66,
      (d-22) a CDR comprising the amino acid sequence shown in SEQ ID NO:110,
      (e-22) a CDR comprising the amino acid sequence shown in SEQ ID NO:5, and
      (f-22) a CDR comprising the amino acid sequence shown in SEQ ID NO:6
   Group 23
      (a-23) a CDR comprising the amino acid sequence shown in SEQ ID NO:1,
      (b-23) a CDR comprising the amino acid sequence shown in SEQ ID NO:2,
      (c-23) a CDR comprising the amino acid sequence shown in SEQ ID NO:3,
      (d-23) a CDR comprising the amino acid sequence shown in SEQ ID NO:4,
      (e-23) a CDR comprising the amino acid sequence shown in SEQ ID NO:5, and
      (f-23) a CDR comprising the amino acid sequence shown in SEQ ID NO:115
   Group 24
      (a-24) a CDR comprising the amino acid sequence shown in SEQ ID NO:1,
      (b-24) a CDR comprising the amino acid sequence shown in SEQ ID NO:2,
      (c-24) a CDR comprising the amino acid sequence shown in SEQ ID NO:90,
      (d-24) a CDR comprising the amino acid sequence shown in SEQ ID NO:4,
      (e-24) a CDR comprising the amino acid sequence shown in SEQ ID NO:120, and
      (f-24) a CDR comprising the amino acid sequence shown in SEQ ID NO:6.
[8] The antibody of [7], which comprises the combination of the light chain variable region and the heavy chain variable region described in any one of the following groups 25 to 48.
   Group 25
      (g-1) a light chain variable region comprising the above-identified CDRs (a-1) to (c-1); and
      (h-1) a heavy chain variable region comprising the above-identified CDRs (d-1) to (f-1),
   Group 26
      (g-2) a light chain variable region comprising the above-identified CDRs (a-2) to (c-2); and
      (h-2) a heavy chain variable region comprising the above-identified CDRs (d-2) to (f-2),
   Group 27
      (g-3) a light chain variable region comprising the above-identified CDRs (a-3) to (c-3); and
      (h-3) a heavy chain variable region comprising the above-identified CDRs (d-3) to (f-3),
   Group 28
      (g-4) a light chain variable region comprising the above-identified CDRs (a-4) to (c-4); and
      (h-4) a heavy chain variable region comprising the above-identified CDRs (d-4) to (f-4),
   Group 29
      (g-5) a light chain variable region comprising the above-identified CDRs (a-5) to (c-5); and
      (h-5) a heavy chain variable region comprising the above-identified CDRs (d-5) to (f-5),
   Group 30
      (g-6) a light chain variable region comprising the above-identified CDRs (a-6) to (c-6); and
      (h-6) a heavy chain variable region comprising the above-identified CDRs (d-6) to (f-6),
   Group 31
      (g-7) a light chain variable region comprising the above-identified CDRs (a-7) to (c-7); and
      (h-7) a heavy chain variable region comprising the above-identified CDRs (d-7) to (f-7),
   Group 32
      (g-8) a light chain variable region comprising the above-identified CDRs (a-8) to (c-8); and
      (h-8) a heavy chain variable region comprising the above-identified CDRs (d-8) to (f-8),
   Group 33
      (g-9) a light chain variable region comprising the above-identified CDRs (a-9) to (c-9); and
      (h-9) a heavy chain variable region comprising the above-identified CDRs (d-9) to (f-9),
   Group 34
      (g-10) a light chain variable region comprising the above-identified CDRs (a-10) to (c-10); and
      (h-10) a heavy chain variable region comprising the above-identified CDRs (d-10) to (f-10),
   Group 35
      (g-11) a light chain variable region comprising the above-identified CDRs (a-11) to (c-11); and
      (h-11) a heavy chain variable region comprising the above-identified CDRs (d-11) to (f-11),
   Group 36
      (g-12) a light chain variable region comprising the above-identified CDRs (a-12) to (c-12); and
      (h-12) a heavy chain variable region comprising the above-identified CDRs (d-12) to (f-12),
   Group 37
      (g-13) a light chain variable region comprising the above-identified CDRs (a-13) to (c-13); and
      (h-13) a heavy chain variable region comprising the above-identified CDRs (d-13) to (f-13),
   Group 38
      (g-14) a light chain variable region comprising the above-identified CDRs (a-14) to (c-14); and
      (h-14) a heavy chain variable region comprising the above-identified CDRs (d-14) to (f-14),
   Group 39
      (g-15) a light chain variable region comprising the above-identified CDRs (a-15) to (c-15); and
      (h-15) a heavy chain variable region comprising the above-identified CDRs (d-15) to (f-15),
   Group 40
      (g-16) a light chain variable region comprising the above-identified CDRs (a-16) to (c-16); and
      (h-16) a heavy chain variable region comprising the above-identified CDRs (d-16) to (f-16),
   Group 41
      (g-17) a light chain variable region comprising the above-identified CDRs (a-17) to (c-17); and
      (h-17) a heavy chain variable region comprising the above-identified CDRs (d-17) to (f-17),
   Group 42
      (g-18) a light chain variable region comprising the above-identified CDRs (a-18) to (c-18); and
      (h-18) a heavy chain variable region comprising the above-identified CDRs (d-18) to (f-18),
   Group 43
      (g-19) a light chain variable region comprising the above-identified CDRs (a-19) to (c-19); and
      (h-19) a heavy chain variable region comprising the above-identified CDRs (d-19) to (f-19),
   Group 44
      (g-20) a light chain variable region comprising the above-identified CDRs (a-20) to (c-20); and
      (h-20) a heavy chain variable region comprising the above-identified CDRs (d-20) to (f-20),
   Group 45
      (g-21) a light chain variable region comprising the above-identified CDRs (a-21) to (c-21); and
      (h-21) a heavy chain variable region comprising the above-identified CDRs (d-21) to (f-21),
   Group 46
      (g-22) a light chain variable region comprising the above-identified CDRs (a-22) to (c-22); and
      (h-22) a heavy chain variable region comprising the above-identified CDRs (d-22) to (f-22),
   Group 47
      (g-23) a light chain variable region comprising the above-identified CDRs (a-23) to (c-23); and
      (h-23) a heavy chain variable region comprising the above-identified CDRs (d-23) to (f-23),
   Group 48
      (g-24) a light chain variable region comprising the above-identified CDRs (a-24) to (c-24); and
      (h-24) a heavy chain variable region comprising the above-identified CDRs (d-24) to (f-24).
[9] The antibody of [8], which comprises the combination of the light chain variable region and the heavy chain variable region described in any one of the following groups 49 to 72.
   Group 49
      (i-1) a light chain variable region comprising the amino acid sequence shown in SEQ ID NO:8; and
      (j-1) a heavy chain variable region comprising amino acid sequence shown in SEQ ID NO:10,
   Group 50
      (i--2) a light chain variable region comprising the amino acid sequence shown in SEQ ID NO:38; and
      (j-2) a heavy chain variable region comprising amino acid sequence shown in SEQ ID NO:10,
   Group 51
      (i-3) a light chain variable region comprising the amino acid sequence shown in SEQ ID NO:41; and
      (j-3) a heavy chain variable region comprising amino acid sequence shown in SEQ ID NO:10,
   Group 52
      (i-4) a light chain variable region comprising the amino acid sequence shown in SEQ ID NO:44; and
      (j-4) a heavy chain variable region comprising amino acid sequence shown in SEQ ID NO:10,
   Group 53
      (i-5) a light chain variable region comprising the amino acid sequence shown in SEQ ID NO:47; and
      (j-5) a heavy chain variable region comprising amino acid sequence shown in SEQ ID NO:10,
   Group 54
      (i-6) a light chain variable region comprising amino acid sequence shown in SEQ ID NO:50; and
      (j-6) a heavy chain variable region comprising amino acid sequence shown in SEQ ID NO:10,
   Group 55
      (i-7) a light chain variable region comprising amino acid sequence shown in SEQ ID NO:53; and
      (j-7) a heavy chain variable region comprising amino acid sequence shown in SEQ ID NO:10,
   Group 56
      (i-8) a light chain variable region comprising amino acid sequence shown in SEQ ID NO:56; and
      (j-8) a heavy chain variable region comprising amino acid sequence shown in SEQ ID NO:57,
   Group 57
      (i-9) a light chain variable region comprising amino acid sequence shown in SEQ ID NO:62; and
      (j-9) a heavy chain variable region comprising amino acid sequence shown in SEQ ID NO:63,
   Group 58
      (i-10) a light chain variable region comprising amino acid sequence shown in SEQ ID NO:67; and
      (j-10) a heavy chain variable region comprising amino acid sequence shown in SEQ ID NO:10,
   Group 59
      (i-11) a light chain variable region comprising amino acid sequence shown in SEQ ID NO:71; and
      (j-11) a heavy chain variable region comprising amino acid sequence shown in SEQ ID NO:72,
   Group 60
      (i-12) a light chain variable region comprising amino acid sequence shown in SEQ ID NO:75; and
      (j-12) a heavy chain variable region comprising amino acid sequence shown in SEQ ID NO:10,
   Group 61
      (i-13) a light chain variable region comprising amino acid sequence shown in SEQ ID NO:8; and
      (j-13) a heavy chain variable region comprising amino acid sequence shown in SEQ ID NO:78,
   Group 62
      (i-14) a light chain variable region comprising amino acid sequence shown in SEQ ID NO:82; and
      (j-14) a heavy chain variable region comprising amino acid sequence shown in SEQ ID NO:83,
   Group 63
      (i-15) a light chain variable region comprising amino acid sequence shown in SEQ ID NO:86; and
      (j-15) a heavy chain variable region comprising amino acid sequence shown in SEQ ID NO:87,
   Group 64
      (i-16) a light chain variable region comprising amino acid sequence shown in SEQ ID NO:91; and
      (j-16) a heavy chain variable region comprising amino acid sequence shown in SEQ ID NO:10,
   Group 65
      (i-17) a light chain variable region comprising amino acid sequence shown in SEQ ID NO:94; and
      (j-17) a heavy chain variable region comprising amino acid sequence shown in SEQ ID NO:95,
   Group 66
      (i-18) a light chain variable region comprising amino acid sequence shown in SEQ ID NO:100; and
      (j-18) a heavy chain variable region comprising amino acid sequence shown in SEQ ID NO:101,
   Group 67
      (i-19) a light chain variable region comprising amino acid sequence shown in SEQ ID NO:104; and
      (j-19) a heavy chain variable region comprising amino acid sequence shown in SEQ ID NO:101,
   Group 68
      (i-20) a light chain variable region comprising amino acid sequence shown in SEQ ID NO:106; and
      (j-20) a heavy chain variable region comprising amino acid sequence shown in SEQ ID NO:10,
   Group 69
      (i-21) a light chain variable region comprising amino acid sequence shown in SEQ ID NO:108; and
      (j-21) a heavy chain variable region comprising amino acid sequence shown in SEQ ID NO:57,
   Group 70
      (i-22) a light chain variable region comprising amino acid sequence shown in SEQ ID NO:111; and
      (j-22) a heavy chain variable region comprising amino acid sequence shown in SEQ ID NO:112,
   Group 71
      (i-23) a light chain variable region comprising amino acid sequence shown in SEQ ID NO:116; and
      (j-23) a heavy chain variable region comprising amino acid sequence shown in SEQ ID NO:117,
   Group 72
      (i-24) a light chain variable region comprising amino acid sequence shown in SEQ ID NO:121; and
      (j-24) a heavy chain variable region comprising amino acid sequence shown in SEQ ID NO:122.
[10] The antibody of [8] or [9], which comprises the combination of the light chain and the heavy chain described in any one of the following groups 73 to 96.
   Group 73
      (k-1) a light chain comprising the amino acid sequence shown in SEQ ID NO:26; and
      (1-1) a heavy chain comprising the amino acid sequence shown in SEQ ID NO:28,
   Group 74
      (k-2) a light chain comprising the amino acid sequence shown in SEQ ID NO:39; and
      (1-2) a heavy chain comprising the amino acid sequence shown in SEQ ID NO:28,
   Group 75
      (k-3) a light chain comprising the amino acid sequence shown in SEQ ID NO:42; and
      (1-3) a heavy chain comprising the amino acid sequence shown in SEQ ID NO:28,
   Group 76
      (k-4) a light chain comprising the amino acid sequence shown in SEQ ID NO:45; and
      (1-4) a heavy chain comprising the amino acid sequence shown in SEQ ID NO:28,
   Group 77
      (k-5) a light chain comprising the amino acid sequence shown in SEQ ID NO:48; and
      (1-5) a heavy chain comprising the amino acid sequence shown in SEQ ID NO:28,
   Group 78
      (k-6) a light chain comprising the amino acid sequence shown in SEQ ID NO:51; and
      (1-6) a heavy chain comprising the amino acid sequence shown in SEQ ID NO:28,
   Group 79
      (k-7) a light chain comprising the amino acid sequence shown in SEQ ID NO:54; and
      (1-7) a heavy chain comprising the amino acid sequence shown in SEQ ID NO:28,
   Group 80
      (k-8) a light chain comprising the amino acid sequence shown in SEQ ID NO:58; and
      (1-8) a heavy chain comprising the amino acid sequence shown in SEQ ID NO:59,
   Group 81
      (k-9) a light chain comprising the amino acid sequence shown in SEQ ID NO:64; and
      (1-9) a heavy chain comprising the amino acid sequence shown in SEQ ID NO:65,
   Group 82
      (k-10) a light chain comprising the amino acid sequence shown in SEQ ID NO:68; and
      (1-10) a heavy chain comprising the amino acid sequence shown in SEQ ID NO:28,
   Group 83
      (k-11) a light chain comprising the amino acid sequence shown in SEQ ID NO:73; and
      (1-11) a heavy chain comprising the amino acid sequence shown in SEQ ID NO:74,
   Group 84
      (k-12) a light chain comprising the amino acid sequence shown in SEQ ID NO:76; and
      (1-12) a heavy chain comprising the amino acid sequence shown in SEQ ID NO:28,
   Group 85
      (k-13) a light chain comprising the amino acid sequence shown in SEQ ID NO:26; and
      (1-13) a heavy chain comprising the amino acid sequence shown in SEQ ID NO:79,
   Group 86
      (k-14) a light chain comprising the amino acid sequence shown in SEQ ID NO:84; and
      (1-14) a heavy chain comprising the amino acid sequence shown in SEQ ID NO:85,
   Group 87
      (k-15) a light chain comprising the amino acid sequence shown in SEQ ID NO:88; and
      (1-15) a heavy chain comprising the amino acid sequence shown in SEQ ID NO:89,
   Group 88
      (k-16) a light chain comprising the amino acid sequence shown in SEQ ID NO:92; and
      (1-16) a heavy chain comprising the amino acid sequence shown in SEQ ID NO:28,
   Group 89
      (k-17) a light chain comprising the amino acid sequence shown in SEQ ID NO:96; and
      (1-17) a heavy chain comprising the amino acid sequence shown in SEQ ID NO:97,
   Group 90
      (k-18) a light chain comprising the amino acid sequence shown in SEQ ID NO:102; and
      (1-18) a heavy chain comprising the amino acid sequence shown in SEQ ID NO:103,
   Group 91
      (k-19) a light chain comprising the amino acid sequence shown in SEQ ID NO:105; and
      (1-19) a heavy chain comprising the amino acid sequence shown in SEQ ID NO:103,
   Group 92
      (k-20) a light chain comprising the amino acid sequence shown in SEQ ID NO:107; and
      (1-20) a heavy chain comprising the amino acid sequence shown in SEQ ID NO:28,
   Group 93
      (k-21) a light chain comprising the amino acid sequence shown in SEQ ID NO:109; and
      (1-21) a heavy chain comprising the amino acid sequence shown in SEQ ID NO:59,
   Group 94
      (k-22) a.light chain comprising the amino acid sequence shown in SEQ ID NO:113; and
      (1-22) a heavy chain comprising the amino acid sequence shown in SEQ ID NO:114,
   Group 95
      (k-23) a light chain comprising the amino acid sequence shown in SEQ ID NO:118; and
      (1-23) a heavy chain comprising the amino acid sequence shown in SEQ ID NO:119,
   Group 96
      (k-24) a light chain comprising the amino acid sequence shown in SEQ ID NO:123; and
      (1-24) a heavy chain comprising the amino acid sequence shown in SEQ ID NO:124.
[11] An antibody binding to the human CD81 having the amino acid sequence shown in SEQ ID NO:22 competitively with the antibody of any one of [7] to [10].
[12] The antibody of [11], which has a suppressive effect of T cell migration.
[13] An anti-human CD81 antibody, wherein the antibody comprises one or more of CDRs described in any one of the groups 1 to 24 in [7] and binds to the human CD81 having the amino acid sequence shown in SEQ ID NO:22 competitively with the antibody comprising all 6 CDRs described in said group.
[14] An anti-human CD81 antibody, wherein the antibody has a 90% sequence homology with any one of antibodies of [7] and binds to the human CD81 having the amino acid sequence shown in SEQ ID NO:22 competitively with said antibody.
[15] An anti-human CD81 antibody comprising:
   (a-25) a CDR comprising the amino acid sequence shown in SEQ ID NO:11;
   (b-25) a CDR comprising the amino acid sequence shown in SEQ ID NO:12;
   (c-25) a CDR comprising the amino acid sequence shown in SEQ ID NO:13;
   (d-25) a CDR comprising the amino acid sequence shown in SEQ ID NO:14;
   (e-25) a CDR comprising the amino acid sequence shown in SEQ ID NO:15; and
   (f-25) a CDR comprising the amino acid sequence shown in SEQ ID NO:16.
[16] The antibody of [15], which comprises:
   (g-25) a light chain variable region comprising the above-identified CDRs (a-25) to (c-25); and
   (h-25) a heavy chain variable region comprising the above-identified CDRs (d-25) to (f-25).
[17] The antibody of [16], which comprises:
   (i-25) a light chain variable region comprising the amino acid sequence shown in SEQ ID NO:18; and
   (j-25) a heavy chain variable region comprising the amino acid sequence shown in SEQ ID NO:20.
[18] The antibody of [16] or [17], which comprises:
   (k-25) a light chain comprising the amino acid sequence shown in SEQ ID NO:30; and
   (1-25) a heavy chain comprising the amino acid sequence shown in SEQ ID NO:32
[19] An antibody binding to the human CD81 having the amino acid sequence shown in SEQ ID NO:22 competitively with the antibody of any one of [15] to [18].
[20] The antibody of [19], which has a suppressive effect of T cell migration.
[21] An anti-human CD81 antibody, wherein the antibody comprises one or more of CDRs in [15] and binds to the human CD81 having the amino acid sequence shown in SEQ ID NO:22 competitively with the antibody described in [15].
[22] An anti-human CD81 antibody, wherein the antibody has a 90% sequence homology with the antibody described in [15] and binds to the human CD81 having the amino acid sequence shown in SEQ ID NO:22 competitively with said antibody.
[23] The antibody of any one of [1] to [6], [11] to [14] and [19] to [22] which is a humanized or human antibody.
[24] The antibody of any one of [7] to [10] and [15] to [18] which is a humanized or human antibody.
[25] The polynucleotide comprising a nucleotide sequence that encodes a heavy chain variable region and a light chain variable region of the antibody of any one of [1] to [24].
[26] A combination of the polynucleotide comprising a nucleotide sequence that encodes a heavy chain variable region of the antibody of any one of [1] to [6], [11] to [14] and [19] to [23], and the polynucleotide comprising a nucleotide sequence that encodes a light chain variable region of the antibody of any one of [1] to [6], [11] to [14] and [19] to [23].
[27] A combination of the polynucleotide comprising a nucleotide sequence that encodes a heavy chain variable region of the antibody of any one of [7] to [10], [15] to [18] and [24], and the polynucleotide comprising a nucleotide sequence that encodes the corresponding light chain variable region of the antibody of any one of [7] to [10], [15] to [18] and [24].
[28] The polynucleotide comprising a nucleotide sequence that encodes a heavy chain and a light chain of the antibody of any one of [1] to [24].
[29] A combination of the polynucleotide comprising a nucleotide sequence that encodes a heavy chain of the antibody of any one of [1] to [6], [11] to [14] and [19] to [23], and the polynucleotide comprising a nucleotide sequence that encodes a light chain of the antibody of any one of [1] to [6], [11] to [14] and [19] to [23].
[30] A combination of the polynucleotide comprising a nucleotide sequence that encodes a heavy chain of the antibody of any one of [7] to [10], [15] to [18] and [24], and the polynucleotide comprising a nucleotide sequence that encodes the corresponding light chain of the antibody of any one of [7] to [10], [15] to [18] and [24].
[31] An expression vector comprising the polynucleotide of [25] or [28].
[32] A recombinant cell transformed with the expression vector of [31].
[33] A recombinant cell transformed with the expression vector comprising the polynucleotide comprising a nucleotide sequence that encodes a heavy chain of the antibody of any one of [1] to [6], [11] to [14] and [19] to [23], and with the expression vector comprising the polynucleotide comprising a nucleotide sequence that encodes a light chain of the antibody of any one of [1] to [6], [11] to [14] and [19] to [23].
[34] A recombinant cell transformed with the expression vector comprising the polynucleotide comprising a nucleotide sequence that encodes the heavy chain of the antibody of any one of [7] to [10] [15] to [18] and [24], and with the expression vector comprising the polynucleotide comprising a nucleotide sequence that encodes the corresponding light chain of the antibody of any one of [7] to [10], [15] to [18] and [24].
[35] A method of producing an anti-human CD81 antibody, comprising culturing the recombinant cell of any one of [32] to [34], and recovering the antibody from the culture obtained.
[36] A pharmaceutical composition comprising the antibody of any one of [1] to [24].
[37] An agent for the prophylaxis, improvement or treatment of a disease selected from inflammatory bowel disease, multiple sclerosis, psoriasis and hematological cancer comprising the antibody of any one of [1] to [24].
[38] The antibody of any one of [1] to [3], wherein the binding affinity of the antibody to each of the above-identified human CD81 variants (3), (4), (8), (11) and (12) is less than 40% of that to the human CD81 having the amino acid sequence shown in SEQ ID NO:22.
[39] The antibody of any one of [1] to [3], wherein the binding affinity of the antibody to each of the above-identified human CD81 variants (3), (4), (6) and (8) to (13) is less than 40% of that to the human CD81 having the amino acid sequence shown in SEQ ID NO:22.
[40] The antibody of any one of [1] to [3], wherein the binding affinity of the antibody to each of the above-identified human CD81 variants (1) to (5), (7), (8), (11), and (12) is less than 40% of that to the human CD81 having the amino acid sequence shown in SEQ ID NO:22.

### Effect of the Invention

The present invention can provide a human monoclonal antibody against human CD81 with a superior drug efficacy and low immunogenicity to human. Since the present inventors obtained new findings that the anti-CD81 antibodies suppressed T cell migration, and moreover, exhibited cytotoxic effect on cancer cells, the antibody of the present invention is also useful for the prophylaxis, improvement or treatment of inflammatory diseases including inflammatory bowel diseases, and diseases associated with T cell migration including autoimmune diseases such as multiple sclerosis and psoriasis, as well as cancers caused by CD81-expressing cancer cells, including hematological cancers.

### Description of Embodiments

In the present specification, the indication using abbreviations such as amino acid, (poly)peptide, (poly)nucleotide and the like follows the definitions of IUPAC-IUB [IUPAC-IUB Communication on Biological Nomenclature, Eur. J. Biochem., 138: 9 (1984)], "Guideline for preparing specification and the like containing nucleotide sequence or amino acid sequence" (ed. Japan Patent Office), and conventional marks used in the field.
Herein, the "gene" or "DNA" is used in the meaning that it includes not only a double-stranded DNA but also respective single-stranded DNAs, a sense strand and an antisense strand constituting the double-stranded DNA. It is not particularly limited by the length thereof. Accordingly, the gene (DNA) in the specification includes, unless otherwise instructed, a double-stranded DNA including a human genomic DNA, a single-stranded DNA (plus strand) including a cDNA, a single-stranded DNA having a sequence complementary to the plus strand (complementary strand) and fragments thereof.
Herein, the term "CD81 gene" means a human CD81 gene (DNA) shown by SEQ ID NO:21, or naturally occurring mutants or polymorphic variants thereof (except those encoding any of the mutant proteins of (1) to (13) described below, as a result of the mutation or polymorphism). Such mutants or polymorphic variants include, for example, those registered in the SNP database available form NCBI.
Herein, the term "CD81 protein" or simply "CD81" means a human CD81 protein shown by SEQ ID NO:22, or a protein encoded by the naturally occurring mutant or polymorphic variant DNAs mentioned above.
The "antibody," used herein encompasses a polyclonal antibody, a monoclonal antibody, a chimeric antibody, a single-stranded antibody, or a part thereof capable of binding with its antigen such as an Fab fragment or a fragment generated from an Fab expression library.
Herein, the term "epitope" is a region of an antigen to which an antibody binds. In certain embodiments, it includes any site on an antigen that is capable of specific binding to an immunoglobulin or T cell receptor or B cell receptor. Antigen determinants include chemically active surface groupings of molecules such as amino acids, sugar side chains, phosphoryl groups, or sulfonyl groups, and, in certain embodiments, may have specific three-dimensional structural characteristics, and/or specific charge characteristics. In certain embodiments, it can be mentioned that an antibody specifically binds to its target antigen when it preferentially recognizes the antigen in a complex mixture of proteins and/or macromolecules.

### Structure of antibody

The basic structure of an antibody molecule is shared by all classes, and consists of both a heavy chain having a molecular weight of 50000 to 70000 and a light chain having a molecular weight of 20000 to 30000 (Immunology 4th ed., I. Roitt, J. Brostoff, D. Male eds., Mosby-Year Book, 1996). A heavy chain usually consists of a polypeptide chain comprising about 440 amino acids; heavy chains have characteristic structures in each different classes, and are called the γ, µ, α, δ, and ε chains corresponding to IgG, IgM, IgA, IgD, and IgE. Furthermore, IgG occurs as IgG1, IgG2, IgG3, and IgG4, and corresponding chains are called γ1, γ2, γ3, and γ4, respectively. A light chain usually consists of a polypeptide chain comprising about 220 amino acids; two types, type L and type K, are known, and are called the λ and K chains, respectively. Regarding the peptide configuration of the basic structure of an antibody molecule, two homologous heavy chains and two homologous light chains are bound via disulfide bonds (S-S bonds) and non-covalent bonds, and the molecular weight is 150000 to 190000. The two kinds of light chains are capable of paring with any heavy chain. Each antibody molecule always consists of two identical light chains and two identical heavy chains.

There are four intra-molecular S-S bonds in a heavy chain (five bonds for **µ** and **ε** chains) and two in a light chain; one loop is formed per 100 to 110 amino acid residues, and this steric structure is alike among the loops, and is called a structural unit or domain. For both heavy chains and light chains, the amino acid sequence of the domain located at the N terminus thereof is inconstant, even in preparations from the same class (subclass) of the same animal species, and this domain is called a variable region (V region) (the heavy chain variable region domain is called as V_{H} and the light chain variable region domain is called as V_{L}). The amino acid sequence on the C-terminal side therefrom is nearly constant in each class or subclass, and is called a constant region (C region) (the domains are expressed as C_{H}1, C_{H}2, C_{H}3 and C_{L}, respectively).

The antigen determination site of an antibody consists of V_{H} and V_{L}, and the binding specificity depends on the amino acid sequence of this site. On the other hand, biological activities such as binding to complements or various cells reflect the differences in C region structure among the various classes of Ig. The variability of the variable regions of light chain and heavy chain has been found to be nearly limited to three small hypervariable regions existing in both chains, and these regions are called complementarity determining region (CDR). Several numbering systems for identifying CDRs are in common use. The Kabat definition is based on sequence variability, and the Chothia definition is based on the location of the structural loop regions. The AbM definition is a compromise between the Kabat and Chothia approaches. The CDRs of the light chain and heavy chain variable regions are bounded according to the Kabat, Chothia, or AbM algorithm (Martin et al. (1989) Proc. Natl. Acad. Sci. USA 86: 9268-9272; Martin et al. (1991) Methods Enzymol. 203: 121-153; Pedersen et al. (1992) Immunomethods 1: 126; and Rees et al. (1996) In Sternberg M.J.E. (ed.), Protein Structure Prediction, Oxford University Press, Oxford, pp. 141-172).
In the case of 002-A07 antibody, the CDRs in the heavy chain variable region are bounded by the residues at amino acid Nos. 29-42 (CDR1-H), 49-67 (CDR2-H) and 97-108 (CDR3-H) of the amino acid sequence shown by SEQ ID NO:10, and the CDRs in the light chain variable region are bounded by the residues at amino acid Nos. 22-36 (CDR1-L), 52-58 (CDR2-L) and 90-101 (CDR3-L) of the amino acid sequence shown by SEQ ID NO:8. In the case of 005-C01 antibody, the CDRs in the heavy chain variable region are bounded by the residues at amino acid Nos. 29-42 (CDR1-H), 49-67 (CDR2-H) and 97-102 (CDR3-H) of the amino acid sequence shown by SEQ ID NO:20, and the CDRs in the light chain variable region are bounded by the residues at amino acid Nos. 22-35 (CDR1-L), 51-57 (CDR2-L) and 89-99 (CDR3-L) of the amino acid sequence shown by SEQ ID NO:18.

The portion other than CDRs of the variable region is called a framework region (FR), and is relatively constant. The framework region employs a β sheet conformation, and CDRs can form a loop connecting the β sheet structure. CDRs in each chain are maintained in the three dimensional structure thereof by the framework regions and form an antigen binding site together with CDRs from the other chain.

### Binding assay of antibody

Antibody binding can be confirmed by any known assay method, such as direct and indirect sandwich assays, flow cytometry and immunoprecipitation assays (Zola, Monoclonal Antibodies: A Manual of Techniques, CRC Press, Inc., 1987, pp. 147-158). In the present invention, the binding of an anti-human CD81 monoclonal antibody with a human CD81 polypeptide or a cell presenting same on its surface can be measured, for example, by the following method.

As a typical method, exemplified is a method comprising adsorbing a human CD81 polypeptide (antigen) onto a solid phase, blocking the solid phase with a protein that is not involved in the subsequent antigen-antibody reaction or enzyme reaction (e.g., skim milk, albumin etc.), contacting and incubating a human anti-human CD81 monoclonal antibody (test antibody) with the solid phase, removing an unreacted antibody by B/F separation, and adding a labeled secondary antibody specifically reacting with the test antibody (e.g., anti-human IgG, etc.) to the solid phase to determine the amount of the label on the solid phase. As the solid phase, for example, insoluble polysaccharides such as agarose, dextran and cellulose, synthetic resins such as plastic, polystylene, polyacrylamide and silicone (e.g., tube, microplate, etc.), or glass (beads, tube, etc.) can be used. The membrane fraction of a cell expressing a human CD81 polypeptide may be used as an antigen to be adsorbed onto the solid phase, as shown in Experimental Example 7(2) described below. As a means for immobilization, an antigen may be recombinantly expressed as a fusion protein with a peptide (e.g., His-tag, GST, MBP, etc.) capable of binding with a solid phase (Ni-, glutathione-, maltose-carrier, etc.) and bound to the solid phase with affinity thereto, as shown in Experimental Example 7(2). As the labeling agent, radioisotopes, enzymes, fluorescent substances, luminescent substances and the like can be used. As examples of the radioisotopes, [¹²⁵I], [¹³¹I], [³H], [¹⁴C] and the like can be mentioned. As examples of the enzymes, stable enzymes with high specific activity are preferred; for example, β-galactosidase, β-glucosidase, alkaline phosphatase, peroxidase, malate dehydrogenase and the like can be mentioned. As examples of the fluorescent substances, fluorescamine, fluorescein isothiocyanate and the like can be mentioned. As examples of the luminescent substances, luminol, luminol derivatives, luciferin, lucigenin and the like can be mentioned.

In the sandwich method, an immobilized anti-human CD81 antibody, which binds to human CD81 at a site different from the site to which the antibody of the present invention binds, is reacted with a human CD81 polypeptide (antigen), and further reacted with an anti-human CD81 monoclonal antibody (test antibody). After removing an unreacted antibody by B/F separation, a labeled secondary antibody specifically reacting with the test antibody (e.g., anti-human IgG, etc.) is added to determine the amount of the label on the solid phase. The labeling agent and the solid phase may be the same as mentioned above.

To examine binding of anti-human CD81 monoclonal antibodies to live cells expressing a human CD81 polypeptide, flow cytometry can be used. Briefly, a cell expressing human CD81 (grown under standard growth conditions) can be mixed with an anti-human CD81 antibody (test antibody) in, for example, PBS containing 0.1% BSA, and incubated at 37°C for 1 hour. After washing, the cells are reacted with a secondary antibody (e.g., anti-human IgG antibody) labeled with a fluorescent such as fluorescein or phycoerythrin (PE) under the same conditions as the reaction with test antibody. The samples can be analyzed by a flow cytometer using light and side scatter properties to gate on single cells. For example, when the percentage of the cells possessing a fluorescent intensity greater than that when nonspecific antibody (e.g., human IgG) is used is 90% or more, preferably 95% or more, more preferably 97% or more, the test antibody can be confirmed to specifically bind to the antigen. An alternative assay using fluorescence microscopy may be used in addition to or instead of the flow cytometry assay. Cells can be stained exactly as described above and examined by fluorescence microscopy. This method allows visualization of individual cells.

### Competitive assay

Competitive assays such as competitive ELISA can be used to determine the binding constant (Ka) of an anti-human CD81 antibody or identify another antibody of the present invention, which binds to a human CD81 competitively with the antibody of the present invention already obtained (known antibody). The competitive assays are carried out by adding a free antigen or known antibody to the reaction system of the solid phase and the test antibody in the binding assay using antigen-immobilized solid phase mentioned above. For example, a given concentration of test antibody solution and mixtures in which various concentrations of antigen are added to the test antibody solution are contacted and incubated with an antigen-immobilized solid phase, respectively, and the amounts of label on the respective solid phases are measured. The binding constant can be calculated as the gradient of graph showing the results of Scatchard analysis based on the measured values for respective antigen concentrations. On the other hand, an antibody binding to a human CD81 competitively with the antibody of the present invention can be identified by reacting a labeled known antibody (the inventive antibody) and various concentrations of test antibody with the antigen-immobilized solid phase, and selecting the test antibody that reduced the amount of label on the solid phase in a dose-dependent manner.

### Antibody of the present invention

The antibody of the present invention is an anti-human CD81 antibody capable of binding to a peptide region consisting of the amino acid sequence of the amino acid numbers 80 to 175, preferably 113 to 175, in the amino acid sequence shown in SEQ ID NO:22, or an antibody binding to the human CD81 consisting of the amino acid sequence shown in SEQ ID NO:22 competitively with said anti-human CD81 antibody. The amino acid sequence shown in SEX ID NO:22 is a reported amino acid sequence of human CD81 protein (EMBO J., 20: 12-18, 2001), and registered in NCBI database as Refseq ID: NP_004347. Hereinafter, the protein consisting of the amino acid sequence is also referred to as "naturally occurring human CD81", "wild type CD81" or "wild type human CD81".

The present invention is based on the findings that anti-human CD81 antibodies that recognize the peptide region mentioned above as an epitope exert their therapeutic effect such as suppression of T cell migration with no or few side effects.

The antibody of the present invention can be any monoclonal antibody, as long as it binds to the particular peptide region of the wild type human CD81 mentioned above, or binds to the wild type human CD81 competitively with an antibody binding to the peptide region.

### Epitope analysis

Epitope analysis can be carried out according to various known methods (Epitope Mapping Protocols/Second Edition, Mike Schutkowski, Ulrich Reineke, Ann N Y Acad Sci. 2010 Jan; 1183: 267-87). More detailed epitope analysis for an antibody can be performed by binding inhibition assay, homolog scanning and/or alanine scanning (for example, see JP 2009-159948 A, Science, 244: 1081-1085 (1989)).

Alanine scanning is a method to determine whether each amino acid residue of human CD81 is necessary for binding of its antibody thereto by preparing mutants wherein each of the amino acid residues of wild type human CD81 is substituted with alanine, and examining differences in binding activity of the antibody between against CD81 mutants and against wild type human CD81.
Homolog scanning is a method to determine which amino acid residues can be inserted, substituted or deleted without adverse effect on activity by substituting at least one amino acid residue of wild type human CD81 polypeptide with other homologous amino acid(s). In this method, the amino acid sequence of human CD81 polypeptide is compared to those of known homologous protein molecules and the number of amino acid changes generated within the region having a high homology is minimized (Protein Science (2005), 14:2405).

The homolog scanning and alanine scanning mutagenesis carried out in the present invention (see Experimental examples 8 and 9 etc.) has revealed that the substitutions of the particular amino acid residues in the peptide region mentioned above with other amino acids remarkably reduce the binding affinity of the anti-human CD81 antibodies to the human CD81 mutants. These findings show that the substituted amino acid residues mainly contribute to the binding of the antibodies to wild type human CD81.

Accordingly, in a preferable embodiment, the antibody of the present invention is characterized in that its binding affinity to at least one CD81 variant selected from the following (1) to (13) is less than 40% when its binding affinity to the above-mentioned wild type human CD81 is 100%:
(1) CD81 variant in which the 127th tyrosine (Y) of wild type human CD81 is substituted with phenylalanine or tryptophan (Y127F or Y127W);
(2) CD81 variant in which the 130th alanine (A) of wild type human CD81 is substituted with threonine or valine (A130T or A130V);
(3) CD81 variant in which the 135th valine (V) of wild type human CD81 is substituted with alanine or leucine (V135A or V135L);
(4) CD81 variant in which the 137th aspartic acid (D) of wild type human CD81 is substituted with alanine or glutamic acid (D137A or D137E);
(5) CD81 variant in which the 143rd alanine (A) of wild type human CD81 is substituted with threonine or valine (A143T or A143V);
(6) CD81 variant in which the 151st histidine (H) of wild type human CD81 is substituted with alanine or arginine (H151A or H151R);
(7) CD81 variant in which the 154th leucine (L) of wild type human CD81 is substituted with alanine or isoleucine (L154A or L154I);
(8) CD81 variant in which the 158th glycine (G) of wild type human CD81 is substituted with alanine or serine (G158A or G158S);
(9) CD81 variant in which the 164th alanine (A) of wild type human CD81 is substituted with threonine or valine (A164T or A164V);
(10) CD81 variant in which the 168th serine (S) of wild type human CD81 is substituted with alanine or threonine (S168A or S168T);
(11) CD81 variant in which the 169th valine (V) of wild type human CD81 is substituted with alanine or leucine (V169A or V169L);
(12) CD81 variant in which the 170th leucine (L) of wild type human CD81 is substituted with alanine or isoleucine (L170A or L170I); and
(13) CD81 variant in which the 172nd asparagine (N) of wild type human CD81 is substituted with alanine or glutamine (N172A or N172Q);
wherein the positions of the amino acid residues to be substituted are identified as the amino acid numbers of the amino acid sequence shown in SEQ ID NO:22.

Here, the above-mentioned CD81 variants can be produced by the description of the following Experimental Example or a known method, for example, introducing a suitable nucleotide change causing an amino acid substitution into a DNA encoding the wild type human CD81 polypeptide, or chemically synthesizing a desired variant polypeptide. The mutation of human CD81 polypeptide described here can be formed by, for example, the technique or guideline relating to the preservative or non-preservative mutation shown in US Patent No. 5,364,934 (for example, see Experimental examples 8 and 9 of the present invention).

The binding affinity of an antibody to the wild type or a mutant human CD81 can be determined by various binding assays described above. The binding constant (Ka) of the antibody of the present invention against wild type human CD81 is at least 1 x 10⁷ M⁻¹, preferably 1 x 10⁸ M⁻¹, more preferably 1 x 10⁹ M⁻¹, even more preferably 1 x 10¹⁰ M⁻¹. The binding constant of the antibody can be determined by the competitive assay described above or other well-known methods such as surface plasmon resonance (SPR).

More preferably, the antibody of the present invention is such that the binding affinity to each of the human CD81 variants (9) and (11) above is less than 40% of that to wild type human CD81. The antibody of the present invention is also such that the binding affinity to each of the human CD81 variants (3), (4), (9) and (11) above is less than 40% of that to wild type human CD81, that the binding affinity to each of the human CD81 variants (3), (4), (9), (10) and (11) above is less than 40%, that the binding affinity to each of the human CD81 variants (3), (4), (8), (9), (10) and (11) above is less than 40%, or the binding affinity to each of the human CD81 variants (3), (4), (6), (8), (9), (10), (11) and (12) above is less than 40%. The series of antibodies possessing the binding characteristic are described in detail in (i) below. In the case of an antibody comprising all six CDRs belonging to the group 1, which represents a specific mode of embodiment of the present invention described in (i) below, the binding affinity to each of the human CD81 variants (3), (4), (6) and (8) to (13) above is less than 40%.
In another preferred embodiment, the antibody of the present invention is such that the binding affinity to each of the human CD81 variants (1) to (5), (7), (8), (11) and (12) above is less than 40% of that to wild type human CD81. The series of antibodies possessing the binding characteristic are described in detail in (ii) below. Human CD81 variants shared by this antibody and the aforementioned antibody in the specific mode of embodiment described in (i) below, whose binding affinity is less than 40% of that to wild type human CD81, are the human CD81 variants (3), (4), (8), (11) and (12) above, respectively.

The present invention also provides an antibody binding to wild type human CD81 competitively with any antibody of the present invention described above. The antibody can bind to wild type human CD81 antibody in a region containing amino acid(s) outside the amino acid region of amino acid Nos. 80-175 in the amino acid sequence shown in SEQ ID NO:22, as long as it has a suppressive effect on T cell migration and/or a cytotoxic effect on CD81-expressing cancer cells. While it is desirable that the suppressive effect on T cell migration and the cytotoxic activity on CD81-expressing cancer cells be equivalent (e.g., 0.5-2 fold) to those of an antibody binding to wild type human CD81 in the amino acid region of amino acid Nos. 80-175 or Nos. 113-175 in the amino acid sequence shown in SEQ ID NO:22, the extent of the activity may be different, as long as the antibody exerts a prophylactic, ameliorating or therapeutic effect on inflammatory bowel diseases, multiple sclerosis psoriasis, or hematological cancers of humans. The "competitive binding" of antibodies to their antigen can be examined by the competitive assay described above.

### (i) One specific embodiment

One preferable embodiment is an antibody having a binding affinity of less than 40% to each of the human CD81 variants described in (9) and (11) when its binding affinity to wild type human CD81 is 100%.

A preferable example of the antibodies having the binding properties mentioned above is:
Group 1
   (a-1) a CDR comprising the amino acid sequence shown in SEQ ID NO:1,
   (b-1) a CDR comprising the amino acid sequence shown in SEQ ID NO:2,
   (c-1) a CDR comprising the amino acid sequence shown in SEQ ID NO:3,
   (d-1) a CDR comprising the amino acid sequence shown in SEQ ID NO:4,
   (e-1) a CDR comprising the amino acid sequence shown in SEQ ID NO:5, and
   (f-1) a CDR comprising the amino acid sequence shown in SEQ ID NO:6
Group 2
   (a-2) a CDR comprising the amino acid sequence shown in SEQ ID NO:1,
   (b-2) a CDR comprising the amino acid sequence shown in SEQ ID NO:2,
   (c-2) a CDR comprising the amino acid sequence shown in SEQ ID NO:37,
   (d-2) a CDR comprising the amino acid sequence shown in SEQ ID NO:4,
   (e-2) a CDR comprising the amino acid sequence shown in SEQ ID NO:5, and
   (f-2) a CDR comprising the amino acid sequence shown in SEQ ID NO:6
Group 3
   (a-3) a CDR comprising the amino acid sequence shown in SEQ ID NO:1,
   (b-3) a CDR comprising the amino acid sequence shown in SEQ ID NO:2,
   (c-3) a CDR comprising the amino acid sequence shown in SEQ ID NO:40,
   (d-3) a CDR comprising the amino acid sequence shown in SEQ ID NO:4,
   (e-3) a CDR comprising the amino acid sequence shown in SEQ ID NO:5, and
   (f-3) a CDR comprising the amino acid sequence shown in SEQ ID NO:6
Group 4
   (a-4) a CDR comprising the amino acid sequence shown in SEQ ID NO:1,
   (b-4) a CDR comprising the amino acid sequence shown in SEQ ID NO:2,
   (c-4) a CDR comprising the amino acid sequence shown in SEQ ID NO:43,
   (d-4) a CDR comprising the amino acid sequence shown in SEQ ID NO:4,
   (e-4) a CDR comprising the amino acid sequence shown in SEQ ID NO:5, and
   (f-4) a CDR comprising the amino acid sequence shown in SEQ ID NO:6
Group 5
   (a-5) a CDR comprising the amino acid sequence shown in SEQ ID NO:1,
   (b-5) a CDR comprising the amino acid sequence shown in SEQ ID NO:2,
   (c-5) a CDR comprising the amino acid sequence shown in SEQ ID NO:46,
   (d-5) a CDR comprising the amino acid sequence shown in SEQ ID NO:4,
   (e-5) a CDR comprising the amino acid sequence shown in SEQ ID NO:5, and
   (f-5) a CDR comprising the amino acid sequence shown in SEQ ID NO:6
Group 6
   (a-6) a CDR comprising the amino acid sequence shown in SEQ ID NO:1,
   (b-6) a CDR comprising the amino acid sequence shown in SEQ ID NO:2,
   (c-6) a CDR comprising the amino acid sequence shown in SEQ ID NO:49,
   (d-6) a CDR comprising the amino acid sequence shown in SEQ ID NO:4,
   (e-6) a CDR comprising the amino acid sequence shown in SEQ ID NO:5, and
   (f-6) a CDR comprising the amino acid sequence shown in SEQ ID NO:6
Group 7
   (a-7) a CDR comprising the amino acid sequence shown in SEQ ID NO:1,
   (b-7) a CDR comprising the amino acid sequence shown in SEQ ID NO:2,
   (c-7) a CDR comprising the amino acid sequence shown in SEQ ID NO:52,
   (d-7) a CDR comprising the amino acid sequence shown in SEQ ID NO:4,
   (e-7) a CDR comprising the amino acid sequence shown in SEQ ID NO:5, and
   (f-7) a CDR comprising the amino acid sequence shown in SEQ ID NO:6
Group 8
   (a-8) a CDR comprising the amino acid sequence shown in SEQ ID NO:1,
   (b-8) a CDR comprising the amino acid sequence shown in SEQ ID NO:2,
   (c-8) a CDR comprising the amino acid sequence shown in SEQ ID NO:43,
   (d-8) a CDR comprising the amino acid sequence shown in SEQ ID NO:4,
   (e-8) a CDR comprising the amino acid sequence shown in SEQ ID NO:5, and
   (f-8) a CDR comprising the amino acid sequence shown in SEQ ID NO:55
Group 9
   (a-9) a CDR comprising the amino acid sequence shown in SEQ ID NO:60,
   (b-9) a CDR comprising the amino acid sequence shown in SEQ ID NO:2,
   (c-9) a CDR comprising the amino acid sequence shown in SEQ ID NO:3,
   (d-9) a CDR comprising the amino acid sequence shown in SEQ ID NO:4,
   (e-9) a CDR comprising the amino acid sequence shown in SEQ ID NO:5, and
   (f-9) a CDR comprising the amino acid sequence shown in SEQ ID NO:61
Group 10
   (a-10) a CDR comprising the amino acid sequence shown in SEQ ID NO:1,
   (b-10) a CDR comprising the amino acid sequence shown in SEQ ID NO:2,
   (c-10) a CDR comprising the amino acid sequence shown in SEQ ID NO:66,
   (d-10) a CDR comprising the amino acid sequence shown in SEQ ID NO:4,
   (e-10) a CDR comprising the amino acid sequence shown in SEQ ID NO:5, and
   (f-10) a CDR comprising the amino acid sequence shown in SEQ ID NO:6
Group 11
   (a-11) a CDR comprising the amino acid sequence shown in SEQ ID NO:1,
   (b-11) a CDR comprising the amino acid sequence shown in SEQ ID NO:2,
   (c-11) a CDR comprising the amino acid sequence shown in SEQ ID NO:3,
   (d-11) a CDR comprising the amino acid sequence shown in SEQ ID NO:4,
   (e-11) a CDR comprising the amino acid sequence shown in SEQ ID NO:69, and
   (f-11) a CDR comprising the amino acid sequence shown in SEQ ID NO:70
Group 12
   (a-12) a CDR comprising the amino acid sequence shown in SEQ ID NO:60,
   (b-12) a CDR comprising the amino acid sequence shown in SEQ ID NO:2,
   (c-12) a CDR comprising the amino acid sequence shown in SEQ ID NO:66,
   (d-12) a CDR comprising the amino acid sequence shown in SEQ ID NO:4,
   (e-12) a CDR comprising the amino acid sequence shown in SEQ ID NO:5, and
   (f-12) a CDR comprising the amino acid sequence shown in SEQ ID NO:6
Group 13
   (a-13) a CDR comprising the amino acid sequence shown in SEQ ID NO:1,
   (b-13) a CDR comprising the amino acid sequence shown in SEQ ID NO:2,
   (c-13) a CDR comprising the amino acid sequence shown in SEQ ID NO:3,
   (d-13) a CDR comprising the amino acid sequence shown in SEQ ID NO:77,
   (e-13) a CDR comprising the amino acid sequence shown in SEQ ID NO:5, and
   (f-13) a CDR comprising the amino acid sequence shown in SEQ ID NO:6
Group 14
   (a-14) a CDR comprising the amino acid sequence shown in SEQ ID NO:80,
   (b-14) a CDR comprising the amino acid sequence shown in SEQ ID NO:2,
   (c-14) a CDR comprising the amino acid sequence shown in SEQ ID NO:3,
   (d-14) a CDR comprising the amino acid sequence shown in SEQ ID NO:4,
   (e-14) a CDR comprising the amino acid sequence shown in SEQ ID NO:81, and
   (f-14) a CDR comprising the amino acid sequence shown in SEQ ID NO:6
Group 15
   (a-15) a CDR comprising the amino acid sequence shown in SEQ ID NO:1,
   (b-15) a CDR comprising the amino acid sequence shown in SEQ ID NO:2,
   (c-15) a CDR comprising the amino acid sequence shown in SEQ ID NO:66,
   (d-15) a CDR comprising the amino acid sequence shown in SEQ ID NO:4,
   (e-15) a CDR comprising the amino acid sequence shown in SEQ ID NO:5, and
   (f-15) a CDR comprising the amino acid sequence shown in SEQ ID NO:6
Group 16
   (a-16) a CDR comprising the amino acid sequence shown in SEQ ID NO:1,
   (b-16) a CDR comprising the amino acid sequence shown in SEQ ID NO:2,
   (c-16) a CDR comprising the amino acid sequence shown in SEQ ID NO:90,
   (d-16) a CDR comprising the amino acid sequence shown in SEQ ID NO:4,
   (e-16) a CDR comprising the amino acid sequence shown in SEQ ID NO:5, and
   (f-16) a CDR comprising the amino acid sequence shown in SEQ ID NO:6
Group 17
   (a-17) a CDR comprising the amino acid sequence shown in SEQ ID NO:1,
   (b-17) a CDR comprising the amino acid sequence shown in SEQ ID NO:2,
   (c-17) a CDR comprising the amino acid sequence shown in SEQ ID NO:52,
   (d-17) a CDR comprising the amino acid sequence shown in SEQ ID NO:4,
   (e-17) a CDR comprising the amino acid sequence shown in SEQ ID NO:5, and
   (f-17) a CDR comprising the amino acid sequence shown in SEQ ID NO:93
Group 18
   (a-18) a CDR comprising the amino acid sequence shown in SEQ ID NO:98,
   (b-18) a CDR comprising the amino acid sequence shown in SEQ ID NO:2,
   (c-18) a CDR comprising the amino acid sequence shown in SEQ ID NO:3,
   (d-18) a CDR comprising the amino acid sequence shown in SEQ ID NO:4,
   (e-18) a CDR comprising the amino acid sequence shown in SEQ ID NO:5, and
   (f-18) a CDR comprising the amino acid sequence shown in SEQ ID NO:99
Group 19
   (a-19) a CDR comprising the amino acid sequence shown in SEQ ID NO:60,
   (b-19) a CDR comprising the amino acid sequence shown in SEQ ID NO:2,
   (c-19) a CDR comprising the amino acid sequence shown in SEQ ID NO:3,
   (d-19) a CDR comprising the amino acid sequence shown in SEQ ID NO:4,
   (e-19) a CDR comprising the amino acid sequence shown in SEQ ID NO:5, and
   (f-19) a CDR comprising the amino acid sequence shown in SEQ ID NO:99
Group 20
   (a-20) a CDR comprising the amino acid sequence shown in SEQ ID NO:1,
   (b-20) a CDR comprising the amino acid sequence shown in SEQ ID NO:2,
   (c-20) a CDR comprising the amino acid sequence shown in SEQ ID NO:90,
   (d-20) a CDR comprising the amino acid sequence shown in SEQ ID NO:4,
   (e-20) a CDR comprising the amino acid sequence shown in SEQ ID NO:5, and
   (f-20) a CDR comprising the amino acid sequence shown in SEQ ID NO:6
Group 21
   (a-21) a CDR comprising the amino acid sequence shown in SEQ ID NO:1,
   (b-21) a CDR comprising the amino acid sequence shown in SEQ ID NO:2,
   (c-21) a CDR comprising the amino acid sequence shown in SEQ ID NO:3,
   (d-21) a CDR comprising the amino acid sequence shown in SEQ ID NO:4,
   (e-21) a CDR comprising the amino acid sequence shown in SEQ ID NO:5, and
   (f-21) a CDR comprising the amino acid sequence shown in SEQ ID NO:55
Group 22
   (a-22) a CDR comprising the amino acid sequence shown in SEQ ID NO:1,
   (b-22) a CDR comprising the amino acid sequence shown in SEQ ID NO:2,
   (c-22) a CDR comprising the amino acid sequence shown in SEQ ID NO:66,
   (d-22) a CDR comprising the amino acid sequence shown in SEQ ID NO:110,
   (e-22) a CDR comprising the amino acid sequence shown in SEQ ID NO:5, and
   (f-22) a CDR comprising the amino acid sequence shown in SEQ ID NO:6
Group 23
   (a-23) a CDR comprising the amino acid sequence shown in SEQ ID NO:1,
   (b-23) a CDR comprising the amino acid sequence shown in SEQ ID NO:2,
   (c-23) a CDR comprising the amino acid sequence shown in SEQ ID NO:3,
   (d-23) a CDR comprising the amino acid sequence shown in SEQ ID NO:4,
   (e-23) a CDR comprising the amino acid sequence shown in SEQ ID NO: 5, and
   (f-23) a CDR comprising the amino acid sequence shown in SEQ ID NO:115
Group 24
   (a-24) a CDR comprising the amino acid sequence shown in SEQ ID NO:1,
   (b-24) a CDR comprising the amino acid sequence shown in SEQ ID NO:2,
   (c-24) a CDR comprising the amino acid sequence shown in SEQ ID NO:90,
   (d-24) a CDR comprising the amino acid sequence shown in SEQ ID NO:4,
   (e-24) a CDR comprising the amino acid sequence shown in SEQ ID NO:120, and
   (f-24) a CDR comprising the amino acid sequence shown in SEQ ID NO:6;
or
(2) an antibody comprising CDRs of (a-X) to (f-X) (X is 1 to 24; the same applies below) above, wherein one or more, preferably one to several (e.g., 1, 2, 3, 4 or 5) amino acid residues are substituted and/or deleted and/or added and/or inserted in one or more (e.g., 1, 2, 3, 4, 5 or 6) amino acid sequences selected from the amino acid sequences shown in (a-X) to (f-X), and wherein the antibody has a binding property equivalent to that of any one of the above-mentioned antibodies, or binds to wild type human CD81 competitively with any one of the above-mentioned antibodies. Namely, the "equivalent binding property" means at least binding to a peptide region consisting of the 80th to 175th or 113th to 175th amino acid residues of wild type human CD81 polypeptide, preferably further binding to at least one human CD81 variant selected from (1) to (13) mentioned above (more preferably the variants (9) and (11)) in a binding affinity of less than 40% when its binding affinity to wild type human CD81 is 100%.
The antibody (2) above can be obtained by a publicly known method, for example, by performing PCR with a vector that encodes the base sequence of the variable region of the antibody (1) above as the template to comprehensively introduce mutations to generate a library of phage display mutant antibodies, screening the library with the binding activity for human CD81 or the competitive binding against the antibody (1) above as an index, and performing panning.
The binding property and competitive binding of an antibody can be determined by various binding assays and competitive assays described above. As a result of the assays, when the "equivalent binding property" or "competitive binding" of the tested antibody is confirmed, then its therapeutic effect can be tested by the cell migration experiment described in detail below.

A more preferable example of the antibodies having the binding properties mentioned above is an antibody comprising:
(1) a light chain variable region comprising the above-identified CDRs (a-X) to (c-X) and a heavy chain variable region comprising the above-identified CDRs (d-X) to (f-X); or
(2) the light chain and heavy chain variable region of (1) above, wherein one or more, preferably one to several (e.g., 1, 2, 3, 4 or 5) amino acid residues are substituted and/or deleted and/or added and/or inserted in one or more (e.g., 1, 2, 3, 4, 5 or 6) amino acid sequences selected from the amino acid sequences shown in (a-X) to (f-X), and wherein the antibody has a binding property equivalent to that of any one of the above-mentioned antibodies, or binds to wild type human CD81 competitively with any one of the above-mentioned antibodies. Here, the "equivalent binding property" means the same as above.

More preferably, in the antibody mentioned above, the CDRs (a-X), (b-X) and (c-X) are located in this order from the N-terminus of the light chain. Namely, the CDRs (a-X), (b-X) and (c-X) corresponds to CDR1, CDR2 and CDR3 of the light chain, respectively. Similarly, the CDRs (d-X), (e-X) and (f-X) are located in this order from the N-terminus of the heavy chain. Namely, the CDRs (d-X), (e-X) and (f-X) correspond to CDR1, CDR2 and CDR3 of the heavy chain, respectively.

An even more preferable example of the antibodies having the binding properties mentioned above is an antibody comprising:
(1) a light chain variable region comprising the amino acid sequence shown in (i-X) and a heavy chain variable region comprising the amino acid sequence shown in (j-X); or
(2) the light chain and heavy chain variable region of (1) above, wherein one or more, preferably 1 to 20, more preferably 1 to 10, even more preferably one to several (e.g., 1, 2, 3, 4 or 5) amino acid residues are substituted and/or deleted and/or added and/or inserted in either or both of the amino acid sequences shown in (i-X) and (j-X), and wherein the antibody has a binding property equivalent to that of any one of the above-mentioned antibodies, or binds to wild type human CD81 competitively with any one of the above-mentioned antibodies. Here, the "equivalent binding property" means the same as above.

Another preferable example of the antibodies having the binding properties mentioned above is an antibody that binds to the same or essentially the same epitope of wild type human CD81 as that to which the antibody comprising a light chain variable region comprising the amino acid sequence shown in (i-X) and a heavy chain variable region comprising the amino acid sequence shown in (j-X) binds. A more preferable example is an antibody that binds to the same or essentially the same epitope of wild type human CD81 as that to which the antibody comprising a light chain comprising the amino acid sequence shown in (k-X) and a heavy chain comprising the amino acid sequence shown in (1-X) binds.
Here, "essentially the same epitope" means an epitope that is different from, but sterically overlaps, the epitope recognized by an antibody having the above-mentioned particular light chain (variable region) and heavy chain (variable region) sequences. An antibody recognizing "essentially the same epitope" competes with an antibody having the above-mentioned particular light chain (variable region) and heavy chain (variable region) sequences for binding to wild type human CD81.

The most widely used and rapid methods for determining whether two antibodies bind to identical or sterically overlapping epitopes are competition assays, which can be configured in all number of different formats, using either labeled antigen or labeled antibody. Usually, the antigen is immobilized on a substrate, and the ability of unlabeled antibodies to block the binding of labeled antibodies is measured using radioactive isotopes or enzyme labels.

The antibody of the present invention may also be an antibody comprising at least one CDR selected from among six CDRs belonging to any one of the groups 1 to 24 above, that binds to wild type human CD81 competitively with an antibody comprising all the six CDRs.

Furthermore, the antibody of the present invention may be an antibody that possesses a homology of 80% or more, preferably 90% or more, more preferably 95% or more, to the amino acid sequences of the light chain and heavy chain of an antibody comprising all six CDRs belonging to any one of the groups 1 to 24 above, and binds to wild type human CD81 competitively with the antibody. Amino acid sequence homology as mentioned herein can be calculated using the blastp program of NCBI BLAST (National Center for Biotechnology Information Basic Local Alignment Search Tool) under the following conditions (expectancy=10; gap allowed; matrix=BLOSUM62; filtering=OFF).

### (ii) Another specific embodiment

Another preferable embodiment is an antibody having a binding affinity of less than 40% to each of the human CD81 variants described in (1) to (5), (7), (8), (11) and (12) when its binding affinity to wild type human CD81 is 100%.

A preferable example of the antibodies having the binding properties mentioned above is:
(1) an antibody comprising:
   (a-25) a CDR comprising the amino acid sequence shown in SEQ ID NO:11;
   (b-25) a CDR comprising the amino acid sequence shown in SEQ ID NO:12;
   (c-25) a CDR comprising the amino acid sequence shown in SEQ ID NO:13;
   (d-25) a CDR comprising the amino acid sequence shown in SEQ ID NO:14;
   (e-25) a CDR comprising the amino acid sequence shown in SEQ ID NO:15; and
   (f-25) a CDR comprising the amino acid sequence shown in SEQ ID NO:16, or
(2) an antibody comprising CDRs of (a-25) to (f-25) above, wherein one or more, preferably one to several (e.g., 1, 2, 3, 4 or 5) amino acid residues are substituted and/or deleted and/or added and/or inserted in one or more (e.g., 1, 2, 3, 4, 5 or 6) amino acid sequences selected from the amino acid sequences shown in SEQ ID NO:11 to 16, and wherein the antibody has a binding property equivalent to that of any one of the above-mentioned antibodies, or binds to wild type human CD81 competitively with any one of the above-mentioned antibodies. Here, the "equivalent binding property" means the same as above.
The binding property and competitive binding of an antibody can be determined by various binding assays and competitive assays described above. As a result of the assays, when the "equivalent binding property" or "competitive binding" of the tested antibody is confirmed, then its therapeutic effect can be tested by the cell migration experiment described in detail below.

A more preferable example of the antibodies having the binding properties mentioned above is an antibody comprising:
(1) a light chain variable region comprising the above-identified CDRs (a-25) to (c-25) and a heavy chain variable region comprising the above-identified CDRs (d-25) to (f-25); or
(2) the light chain and heavy chain variable region of (1) above, wherein one or more, preferably one to several (e.g., 1, 2, 3, 4 or 5) amino acid residues are substituted and/or deleted and/or added and/or inserted in one or more (e.g., 1, 2, 3, 4, 5 or 6) amino acid sequences selected from the amino acid sequences shown in SEQ ID NOs:11 to 16, and wherein the antibody has a binding property equivalent to that of any one of the above-mentioned antibodies, or binds to wild type human CD81 competitively with any one of the above-mentioned antibodies. Here, the "equivalent binding property" means the same as above.

More preferably, in the antibody mentioned above, the CDRs (a-25), (b-25) and (c-25) are located in this order from the N-terminus of the light chain. Namely, the CDRs (a-25), (b-25) and (c-25) corresponds to CDR1, CDR2 and CDR3 of the light chain, respectively. Similarly, the CDRs (d-25), (e-25) and (f-25) are located in this order from the N-terminus of the heavy chain. Namely, the CDRs (d-25), (e-25) and (f-25) correspond to CDR1, CDR2 and CDR3 of the heavy chain, respectively.

[0050] An even more preferable example of the antibodies having the binding properties mentioned above is an antibody comprising:
(1) a light chain variable region comprising the amino acid sequence shown in SEQ ID NO:18 and a heavy chain variable region comprising the amino acid sequence shown in SEQ ID NO:20; or
(2) the light chain and heavy chain variable region of (1) above, wherein one or more, preferably 1 to 20, more preferably 1 to 10, even more preferably one to several (e.g., 1, 2, 3, 4 or 5) amino acid residues are substituted and/or deleted and/or added and/or inserted in either or both of the amino acid sequences shown in SEQ ID NOs:18 and 20, and wherein the antibody has a binding property equivalent to that of any one of the above-mentioned antibodies, or binds to wild type human CD81 competitively with any one of the above-mentioned antibodies. Here, the "equivalent binding property" means the same as above.

Another preferable example of the antibodies having the binding properties mentioned above is an antibody that binds to the same or essentially the same epitope of wild type human CD81 as that to which the antibody comprising a light chain variable region comprising the amino acid sequence shown in SEQ ID NO:18 and a heavy chain variable region comprising the amino acid sequence shown in SEQ ID NO:20 binds. A more preferable example is an antibody that binds to the same or essentially the same epitope of wild type human CD81 as that to which the antibody comprising a light chain comprising the amino acid sequence shown in SEQ ID NO:30 and a heavy chain comprising the amino acid sequence shown in SEQ ID NO:32 binds. Here, "essentially the same epitope" mean the same as above.

The antibody of the present invention may also be an antibody that comprises at least one CDR selected from among the six CDRs (a-25) to (f-25) above, and binds to wild type human CD81 competitively with an antibody comprising all the six CDRs.

Furthermore, the antibody of the present invention may be an antibody that possesses a homology of 80% or more, preferably 90% or more, more preferably 95% or more, to the amino acid sequences of the light chain and heavy chain of an antibody comprising all the six CDRs (a-25) to (f-25) above, and binds to wild type human CD81 competitively with the antibody. Here, amino acid homology can be calculated in the same manner as the above.

### (I) Production of antibody

The antibody of the present invention can be any monoclonal antibody, as long as it binds to the particular peptide region of the wild type human CD81 mentioned above. Although the isotype of the antibody is not subject to limitation, it is preferably IgG, IgM or IgA, particularly preferably IgG. Also, the molecule type of the antibody is not subject to limitation, in addition to the entire antibody molecule, the antibody may, for example, be a fragment such as Fab, Fab', or F(ab')₂, a genetically engineered conjugate molecule such as scFv, scFv-Fc, minibody, or diabody, or a derivative thereof modified with a certain molecule, for example, a molecule having a stabilizing action such as polyethylene glycol (PEG), and the like.

Since the antibody of the present invention is used as a pharmaceutical product having humans as the subject of administration thereof, the antibody used in the present invention is an antibody whose risk of showing antigenicity when administered to a human has been reduced; to be specific, the antibody is a (fully) human antibody, a humanized antibody, a non-human (e.g., mouse, rat, rabbit)-human chimeric antibody and the like, particularly preferably a human antibody. A humanized antibody and a chimeric antibody can be prepared by genetic engineering technology according to the method described below. Although a (fully) human antibody can also be produced from human-human (or human-mouse) hybridoma, it is desirable to produce it using a phage display method or a human antibody-producing animal as described below (e.g., mouse), in order to stably supply the antibody in large amounts at low costs.

### (II) Production method of the antibody of the present invention

The antibody of the present invention can be produced by the method described in the following Examples or a known method.

### (i) Preparation of antigen

The antigen used to prepare the antibody of the present invention may be the wild type human CD81 protein or partial peptide thereof, a (synthetic) peptide having one or more kinds of the same antigen determinant as that thereof and the like (hereinafter these are sometimes simply referred to as the antigen of the present invention).

The wild type human CD81 protein or a partial peptide thereof is produced by, for example, (a) preparing the same from a human tissue or cells, by a method known to the public or its modified method (b) chemically synthesizing the same by a publicly known method of peptide synthesis using a peptide synthesizer and the like, (c) culturing a transformant comprising a DNA that encodes wild type human CD81 or a partial peptide thereof, or (d) biochemically synthesizing the same with a nucleic acid that encodes wild type human CD81 or a partial peptide thereof as the template using a cell-free transcription/translation system.

### (iia) Preparation of human antibody using phage display human antibody library

A human antibody can be produced by phage display (Hoogenboom and Winter, J. Mol. Biol., 227:381 (1991); Marks et al., J. Mol. Biol., 222:581 (1991); Cole et al., Monoclonal Antibodies and Cancer Therapy, Alan R. Liss, p. 77 (1985) and Boermer et al., J. Immunol., 147(1):86-95 (1991)).
The method of preparing a phage display human antibody library include, but are not limited to, for example, the methods described below.

Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, besides 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) an E. *coli* having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.

As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the κL chain constant region allocated to downstream of the g3p signal peptide, and a DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an *E*. *coli* having an amber mutation, Fab is presented onto the g3p coat protein, but when it is expressed in the HB2151 strain and the like, which do not have an amber mutation, a soluble Fab antibody is produced. As the scFv-expressing phagemid vector, for example, pHEN1 (J. Mol. Biol., 222: 581-597, 1991) and the like are used.
Meanwhile as examples of the helper phage, M13-K07, VCSM13 and the like can be mentioned.

And as another phage display vector, a vector that is designed as a DNA sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay™ technology of Morphosys Company) and the like, can be mentioned.

As the kind of human antibody library, a naive/non-immunized library, a synthetic library, an immunized library and the like can be mentioned.

The naive/non-immunized library is a library obtained by acquiring the V_{H} and V_{L} genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council (see Annu. Rev. Immunol., 12: 433-455, 1994), the library of Dyax Corp. (see J. Biol. Chem., 1999 (supra); Proc. Natl. Acad. Sci. USA, 14: 7969-7974, 2000) and the like can be mentioned.

A synthetic library is obtained by selecting a functional particular antibody gene in human B cells, and substituting a portion of antigen-binding region in a V gene segment, for example, CDR3 and the like, with DNAs encoding a random amino acid sequence of appropriate length, to construct a library. It is recognized to be excellent in antibody expression efficiency and stability because the library can be constructed with the combination of the V_{H} and V_{L} genes, which produce functional scFv and Fab, since the beginning. Representatively, the HuCAL library of Morphosys AG (see J. Mol. Biol., 296: 57-86, 2000), the library of BioInvent (see Nat. Biotechnol., 18: 852, 2000), the library of Crucell (see Proc. Natl. Acad. Sci. USA, 92: 3938, 1995; J. Immunol. Methods, 272: 219-233, 2003) and the like can be mentioned.

An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described in vitro immunization method, in the same manner as with the above-described naive/non-immunized library, and amplifying the V_{H} and V_{L} genes by RT-PCR, to construct a library. It is possible to obtain the desired antibody even from such libraries of relatively small size because the desired antibody gene is contained in the library already at the beginning.

The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to *E*. *coli* to proliferate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin-(strept) avidin system and the like are preferably used. For washing the unbound phage, a blocking solution such as BSA solution (once or twice), a PBS comprising a surfactant such as Tween (3 to 5 times) and the like can be used. There is also a report mentioning that the use of citrate buffer (pH 5) and the like is preferable for the washing. For elution of the specific phage, an acid (e.g., 0.1 M hydrogen chloride and the like) is normally used; cleavage with a specific protease (e.g., a gene sequence that encodes the trypsin cleavage site can be introduced into the linkage site between the antibody gene and the coat protein gene. In this case, *E. coli* infection and proliferation are possible even if all the coat protein is expressed in the form of a fusion protein because the wild-type coat protein is presented on the surface of the eluted phage), competitive elution with a soluble antigen, or elution by reduction of S-S bond (e.g., in the aforementioned CysDisplay™, the antigen-specific phage can be collected by dissociating the antibody and the coat protein by using a suitable reducing agent after performing panning.) is also possible. When elution has been performed with an acid, the eluate is neutralized with Tris and the like, and the eluted phage is then infected to *E*. *coli,* which is cultured; after which the phage is collected by a conventional method.

After the phage presenting the antigen-specific antibody is concentrated by panning, the phage is infected to *E*. *coli* and the cells are sown onto a plate to perform cell cloning. The phage is again collected from the each clone, and the antigen binding activity is confirmed by the above-described antibody titer assay (e.g., ELISA, RIA and the like) or a measurement utilizing FACS or SPR.

Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an *E. coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using Immobilized Metal Affinity Chromatography (IMAC) method, an anti-c-myc antibody column and the like. When cleavage with a specific protease is utilized in panning, the antibody molecule is separated from the phage surface by an action with the protease, so that the desired antibody can be purified by performing the same purification operation as above mentioned.

The affinity and avidity of the human antibody (e.g., scFv, Fab) thus obtained to the epitope region of wild type human CD81 is confirmed by the binding assays mentioned above using a polypeptide comprising the peptide region.

### (iib) Preparation of human antibody using human antibody-producing animal

Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. Namely, human monoclonal antibodies can be generated by using a human antibody-producing mouse (see Immunol. Today, 17: 391-397, 1996) obtained by crossing a mouse transfected with minigenes of the human Ig H chain and L chain using an ordinary transgenic (Tg) technique with a mouse wherein the endogenous mouse Ig gene has been inactivated using an ordinary KO technique (WO 93/12227, WO 92/03918, WO 94/02602, WO 94/25585, WO 96/34096 and WO 96/33735).

The hybridoma obtained by fusion of the anti-human CD81 antibody-producing cell obtained from the human antibody-producing mouse with a myeloma cell are cultured, and the anti-human CD81 antibody can be recovered from the culture supernatant and purified by conventional methods. The affinity and avidity of the human antibody thus obtained to the epitope region of wild type human CD81 is confirmed by the binding assays mentioned above using a polypeptide comprising the peptide region.

### (iic) Preparation of chimeric antibody

As used herein, "chimeric antibody" means an antibody wherein the sequences of the variable regions of the H chain and L chain (V_{H} and V_{L}) thereof are derived from a non-human animal species, and wherein the sequences of the constant regions (C_{H} and C_{L}) are derived from human. The sequences of the variable regions are preferably derived from, for example, an animal species permitting easy preparation of a hybridoma, such as mouse, rat, rabbit and the like.

As examples of the method of preparing a chimeric antibody, the method described in US Patent No.6,331,415 or a partially modified method thereof and the like can be mentioned.

Host cells are transformed with the chimeric H chain and chimeric L chain expression vector(s) obtained. As the host cells, animal cells, for example, Chinese hamster ovary (CHO) cells, monkey-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter collecting the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or fowl as the host cells, for which a transgenic technique has been established and a know-how of mass propagation as a domestic animal (domestic fowl) has been compiled. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tabacco as the host cells, for which a transgenic technique has been established, and which is cultured in large amounts as a major crop.

### (iii) Humanized antibody

As used herein, "a humanized antibody" means an antibody wherein the sequences of all regions present in the variable region, other than the complementarity determining region (CDR), [i.e., framework region (FR) in constant region and variable region] are derived from a human, and wherein only the sequence of CDR is derived from another mammalian species. The other mammalian species is preferably an animal species, for example, mouse, rat, rabbit and the like, with which production of hybridomas can be easily performed.

As examples of the method of preparing a humanized antibody, the methods described in US Patent Nos. 5,225,539, 5,585,089, 5,693,761 and 5,693,762, EP 239400, WO 92/19759 or partially modified methods therefrom and the like can be mentioned. To be specific, DNAs that encode V_{H} and V_{L} derived from a non-human mammalian species (e.g., mouse) are isolated in the same manner as with the above-described chimeric antibody, after which sequencing is performed by a conventional method using an automated DNA sequencer (e.g., manufactured by Applied Biosystems Company and the like), and the nucleotide sequences obtained or deduced amino acid sequences therefrom are analyzed using a known antibody sequence database [for example, Kabat database (see Kabat et al., "Sequences of Proteins of Immunological Interest", edited by NIH, US Department of Health and Human Services, Public Health Service, 5th edition, 1991) and the like] to determine the CDR and FR of the two chains. A nucleotide sequence wherein the CDR encoding region of a nucleotide sequence that encodes the L chain and H chain of a human antibody having an FR sequence similar to the determined FR sequence is substituted with the determined nucleotide sequence that encodes the CDR of another animal species, is designed, and the nucleotide sequence is divided into fragments of about 20 to 40 bases, and a sequence complementary to the nucleotide sequence is divided into fragments of about 20 to 40 bases so that they alternatively overlap with the aforementioned fragments. It is possible to construct DNAs that encode V_{H} and V_{L} having human-derived FR and a CDR derived from another mammalian species by synthesizing individual fragments using a DNA synthesizer, and hybridizing and ligating them in accordance with conventional methods. In order to transfer a CDR derived from another mammalian species into human-derived V_{H} and V_{L} more quickly and more efficiently, it is preferable to use PCR-based site directed mutagenesis. As examples of such a method, the sequential CDR grafting method described in JP-A-5-227970 and the like can be mentioned.

It should be noted that in preparing a humanized antibody by a method as described above, the antigen binding activity may sometimes decrease, compared with the original non-human antibody, if the amino acid sequence of CDR alone is transplanted to the template human antibody FR. In such cases, it is effective to transplant some FR amino acids around the CDR in combination. The non-human antibody FR amino acid to be transplanted may be an amino acid residue that is important to the maintenance of the steric structure of each CDR; such an amino acid residue can be deduced by a steric structure estimation using a computer.

It is possible to obtain cells or transgenic animal/plant that produces a humanized antibody by ligating the thus-obtained DNAs encoding V_{H} and V_{L} to DNAs encoding human-derived C_{H} and C_{L}, respectively, and introducing the ligated product into suitable host cells.

An alternative method of preparing a humanized antibody without using CDR grafting wherein mouse CDRs are grafted into variable regions of a human antibody is, for example, a method comprising determining which is an amino acid residue that can be substituted in a non-human variable region, on the basis of a conserved structure-function correlation between antibodies. This method can be carried out as described in, for example, EP 0571613 B1, US 5, 766, 886, US 5,770,196, US 5, 821, 123, US 5,869,619 and the like. Provided that the amino acid sequence information on each of the V_{H} and V_{L} of the original non-human antibody, preparation of a humanized antibody using the method can easily be performed by utilizing, for example, the contract antibody preparation service provided by Xoma.

A humanized antibody, like a chimeric antibody, can be modified to scFv, scFv-Fc, minibody, dsFv, Fv and the like by using genetic engineering techniques; and they can be produced in a microorganism such as *E. coli* or yeast by using a suitable promoter.

The affinity and avidity of the humanized antibody thus obtained to the epitope region of wild type human CD81 is confirmed by the binding assays mentioned above using a polypeptide comprising the peptide region.

### (III) Optimization of antibody

The anti-human CD81 antibodies of the invention mentioned above may be readily prepared to include various changes, substitutions, insertions, and deletions. For example, to maximize the expression level of an antibody, the nucleotide sequences of the antibody gene may be optimized so as to match the codon usage frequency of the cell used for antibody expression. Additional modifications to enhance antibody stability include modification of IgG4 to replace the serine at residue 228 in the hinge region with proline as described below. As other modifications, substitutions as required to optimize efficiency in conjugating the antibody with a drug can be mentioned. For example, an antibody may be modified at its carboxyl terminus to include amino acids for drug attachment, for example, one or more cysteine residues may be added. The constant regions may be modified to introduce sites for binding of carbohydrates or other moieties.
Mutants of anti-CD81 antibodies of the invention may be produced using standard recombinant techniques, including site-directed mutagenesis, or recombination cloning.

A particularly preferred type of substitutional variant involves substituting one or more hypervariable region residues of a parent antibody (e.g., a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable regions are mutated to generate all possible amino substitutions at each site. The antibody variants thus generated are displayed as fused proteins with a coat protein g3p of filamentous phage M13 on phages. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Once such variants are generated, the panel of variants is subjected to screening as described herein and antibodies with superior properties in one or more relevant assays may be selected for further development. Nucleic acid molecules encoding amino acid sequence variants of the anti-human CD81 antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-human CD81 antibody.
Preferred affinity matured antibodies have an affinity which is five times, more preferably 10 times, even more preferably 20 or 30 times greater than the starting antibody from which the matured antibody is prepared.

### IgG4 mutation

When an IgG4 antibody is administered to human or some animals, a phenomenon that the serum concentration of IgG4 antibody is reduced due to swapping with an endogenous IgG4 has been observed. It has been known that this IgG4 exchange is inhibited by the point mutation of Ser at residue 228 with Pro (Mol. Immunol. 30, 105, 1993, Protein Sci. 6, 407, 1997, Mol Immunol. 38, 1, 2001, Nat Biotechnol. 27, 767, 2009).

### (IV) Confirmation of therapeutic effect of antibody

The antibody of the present invention has a suppressive effect on T lymphocyte migration. This action is detected using a widely known evaluation system for lymphocyte function. For example, the effect on T lymphocyte migration is evaluated by bringing human peripheral blood mononuclear cells (PBMCs) cultured in the presence of cytokines (e.g., PHA, IL-2, TNF-α, IL-22, IL-7 and VIP) into contact with a test antibody, stimulating the migration of PBMCs (e.g., by adding a chemokine, stromal cell-derived factor 1 (SDF-1; also known as CXCL12), and determining inhibition of the migration (see, for example, Blood 2009 August 13; 114 (7): 1366-1373). To be specific, a cell migration experiment described below can be used.
As the lymphocytes, established cell lines such as Jurkat cells (Clone E6-1, ATCC Number TIB-152), and cells derived from human peripheral blood can be used. Human peripheral blood-derived cells are commercially available (KAC Co., Ltd. and the like). Alternatively, human peripheral blood-derived cells can also be prepared by isolating a mononuclear cell fraction containing monocytes and lymphocytes from a peripheral blood of a healthy human using Ficoll/Paque density gradient centrifugation according to The Journal of Immunology, 147, 2251 (1991).

### Cell migration experiment

The ability of an antibody or a functional fragment thereof to inhibit its antigen's function relating to cell migration can be evaluated using a cell migration experiment. In general, a cell migration experiment is performed by separating appropriate cells such as leukocytes (e.g., lymphocytes, eosinophils, basophils) and a chemotactic factor by a barrier (e.g., endothelium, filter), and monitoring the migration of the cells toward or across the barrier. For example, the suppressive effect on cell migration. of a test antibody can be examined by adding a culture medium containing a chemotactic factor such as SDF-1 to a well of 96-well migration plate (the first chamber), putting a transwell having the bottom surface consisting of a cell-permeable microporous membrane (the second chamber) onto the well, adding the test antibody and the cells, measuring the migration of the cells from the second chamber to the first chamber and comparing the extent of migration with that in the absence of antibody or in the presence of a non-specific antibody (Immunol. Invest. 17: 625-677 (1988)).

### (V) Production of recombinant antibody

Any production system can be used for the production of the antibody used in the present invention. The production systems for producing antibodies include *in vitro* and in *vivo* production systems. *In vitro* production systems include production systems using eukaryotic cells and those using prokaryotic cells.
Antibody genes constructed as described above may be expressed and obtained in a known method. In the case of mammalian cells, expression may be accomplished using a DNA in which a commonly used useful promoter, the antibody gene to be expressed, and the poly A signal at 3*'* downstream thereof have been functionally linked or a vector containing said DNA. Examples of the promoter/enhancer include human cytomegalovirus immediate early promoter/enhancer.

Additionally, as the promoter/enhancer which can be used for expression of antibody for use in the present invention, there are viral promoters/enhancers such as retrovirus, polyoma virus, adenovirus, and simian virus 40 (SV40), and promoters/enhancers derived from mammalian cells such as human elongation factor 1α. (hEF1α).

For example, expression may be readily accomplished by the method of Mulligan et al. (Mulligan, R.C. et al., Nature (1979) 277, 108-114) when SV40 promoter/enhancer is used, or by the method of Mizushima et al. (Mizushima, S. and Nagata, S. Nucleic Acids Res. (1990) 18, 5322) when hEF1α. promoter/enhancer is used.

In the case of E. coli, expression may be conducted by functionally linking a commonly used useful promoter, a signal sequence for antibody secretion, and the antibody gene to be expressed, followed by expression thereof. As the promoter, for example, there can be mentioned lacZ promoter and araB promoter. The method of Ward et al. (Ward, E.S. et al., Nature (1989) 341, 544-546; Ward, E.S.. et al., FASEB J. (1992) 6, 2422-2427) may be used when lacZ promoter is used, and the method of Better et al. (Better, M. et al., Science (1988) 240, 1041-1043) may be used when araB promoter is used.

As the signal sequence for antibody secretion, when produced in the periplasm of E. coli, the pelB signal sequence (Lei, S. P. et al., J. Bacteriol. (1987) 169, 4379-4383) can be used. After separating the antibody produced in the periplasm, the structure of the antibody is appropriately refolded before use (see, for example, WO 96/30394).

As the origin of replication, there can be used those derived from SV40, polyoma virus, adenovirus, bovine papilloma virus (BPV) and the like. Furthermore, for the amplification of the gene copy number in the host cell system, expression vectors can include as selectable markers the aminoglycoside transferase (APH) gene, the thymidine kinase (TK) gene, E. coli xanthine guaninephosphoribosyl transferase (Ecogpt) gene, the dihydrofolate reductase (dhfr) gene and the like.

When the eukaryotic cells are used, there are the production systems which employ animal cells, plant cells, and fungal cells. Known animal cells include (1) mammalian cells such as CHO cells, COS cells, myeloma cells, baby hamster kidney (BHK) cells, HeLa cells, and Vero cells, (2) amphibian cells such as Xenopusoocytes, or (3) insect cells such as sf9, sf21, and Tn5. Known plant cells include, for example, those derived from Nicotiana tabacum, which is subjected to callus culture. Known fungal cells include yeasts such as the genus Saccharomyces, more specifically Saccharomyces cerevisiae, or filamentous fungi such as the genus Aspergillus, more specifically Aspergillus niger.

When the prokaryotic cells are used, there are the production systems which employ bacterial cells. Known bacterial cells include Escherichia coli (E. coli), and Bacillus subtilis.

By introducing via transformation the gene of the desired antibody into these cells and culturing the transformed cells in vitro, the antibody can be obtained. Culturing is conducted in the known methods. For example, as the culture medium DMEM, MEM, RPMI1640, and IMDM can be used, and serum supplements such as fetal calf serum (FCS) may be used in combination. In addition, antibodies may be produced in vivo by implanting cells into which the antibody gene has been introduced into the abdominal cavity of an animal and the like.

As *in vivo* production systems, there can be mentioned those which employ animals and those which employ plants. When animals are used, there are the production systems which employ mammals and insects.

As mammals, goats, pigs, sheep, mice, and cattle can be used (Vicki Glaser, SPECTRUM Biotechnology Applications, 1993). Also, as insects, silkworms can be used.
When plants are used, tabacco, for example, can be used.

Antibody genes are introduced into these animals or plants, and the antibodies are produced in such animals or plants, and recovered. For example, an antibody gene is inserted into the middle of the gene encoding protein which is inherently produced in the milk such as goat β-casein to prepare fusion genes. DNA fragments containing the fusion gene into which the antibody gene has been inserted are injected into a goat embryo, and the embryo is introduced into a female goat. The desired antibody is obtained from the milk produced by the transgenic goat born to the goat who received the embryo or off springs thereof. In order to increase the amount of milk containing the desired antibody produced by the transgenic goat, hormones may be given to the transgenic goat as appropriate. (Ebert, K. M. et al., Bio/Technology (1994) 12, 699-702).

When silkworms are used, baculovirus into which the desired antibody gene has been inserted is infected to the silkworm, and the desired antibody can be obtained from the body fluid of the silkworm (Maeda, S. et al., Nature (1985) 315, 592-594). Moreover, when tabacco is used, the desired antibody gene is inserted into an expression vector for plants, for example pMON 530, and then the vector is introduced into a bacterium such as Agrobacterium tumefaciens. The bacterium is then infected to tabacco such as Nicotiana tabacum to obtain the desired antibody from the leaves of the tabacco (Julian, K.-C. Ma et al., Eur. J. Immunol. (1994) 24, 131-138).

When antibody is produced in *in vitro* or *in vivo* production systems, as described above, DNA encoding the heavy chain (H chain) or the light chain (L chain) of antibody may be separately integrated into an expression vector and the hosts are transformed simultaneously, or DNA encoding the H chain and the L chain may be integrated into a single expression vector and the host is transformed therewith (see International Patent Application WO 94-11523).

Antibodies for use in the present invention may be antibody fragments or modified versions thereof as long as they are preferably used. For example, as fragments of antibody, there may be mentioned Fab, F(ab')2, Fv or single-chain Fv (scFv) in which Fv's of H chain and L chain were ligated via a suitable linker. Specifically antibodies are treated with an enzyme, for example, papain or pepsin, to produce antibody fragments, or genes encoding these antibody fragments are constructed, and then introduced into an expression vector, which is expressed in a suitable host cell (see, for example, Co, M.S. et al., J. Immunol. (1994) 152, 2968-2976; Better, M. & Horwitz, A.H., Methods in Enzymology (1989) 178, 476-496, Plueckthun, A. & Skerra, A., Methods in Enzymology (1989) 178, 476-496, Lamoyi, E., Methods in Enzymology (1986) 121, 652-663; Rousseaux, J. et al., Methods in Enzymology (1986) 121, 663-669; Bird, R.E. et al., TIBTECH (1991) 9, 132-137).

scFv can be obtained by ligating the V region of H chain and the V region of L chain of antibody. In the scFv, the V region of H chain and the V region of L chain are preferably ligated via a linker, preferably a peptide linker (Huston, J. S. et al., Proc. Natl. Acad. Sci. U.S.A. (1988) 85, 5879-5883). The V region of H chain and the V region of L chain in the scFv may be derived from any of the above-mentioned antibodies. As the peptide linker for ligating the V regions, any single-chain peptide comprising, for example, 12-19 amino acid residues may be used.

DNA encoding scFv can be obtained using DNA encoding the H chain or the H chain V region of the above antibody and DNA encoding the L chain or the L chain V region of the above antibody as the template by amplifying the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNA encoding scFv are constructed, an expression vector containing them and a host transformed with said expression vector can be obtained by the conventional methods, and scfv can be obtained using the resultant host by the conventional methods.

These antibody fragments can be produced by obtaining the gene thereof in a similar manner to that mentioned above and by allowing it to be expressed in a host. "Antibody" as used in the claim of the present application encompasses these antibody fragments.

As modified antibodies, antibodies associated with various molecules such as polyethylene glycol (PEG) can be used. "Antibody" as used in the claim of the present application encompasses these modified antibodies. These modified antibodies can be obtained by chemically modifying the antibodies thus obtained. These methods have already been established in the art.

Antibodies produced and expressed as described above can be separated from the inside or outside of the host cell and then may be purified to homogeneity. Separation and purification of the antibody for use in the present invention may be accomplished by affinity chromatography. As the column used for such affinity chromatography, there can be mentioned Protein A column and Protein G column. Examples of the carriers used in the Protein A column are Hyper D, POROS, Sepharose F. F. and the like. Alternatively, methods for separation and purification conventionally used for proteins can be used without any limitation. Separation and purification of the antibody for use in the present invention may be accomplished by combining, as appropriate, chromatography other than the above-mentioned affinity chromatography, filtration, ultrafiltration, salting-out, dialysis and the like. Chromatography includes, for example, ion exchange chromatography, hydrophobic chromatography, gel-filtration and the like. These chromatographies can be applied into HPLC (High Performance Liquid Chromatography). Alternatively, reverse-phase HPLC can be used.

The concentration of antibody obtained above can be determined by the measurement of absorbance or by the enzyme-linked immunosorbent assay (ELISA) and the like. Thus, when absorbance measurement is employed, the antibody for use in the present invention or a sample containing the antibody is appropriately diluted with PBS(-) and then the absorbance is measured at 280 nm, followed by calculation using the absorption coefficient of 1.35 OD at 1 mg/ml. When the ELISA method is used, measurement is conducted as follows. Thus, 100 µl of goat anti-human IgG (manufactured by TAGO) diluted to 1 µg/ml in 0.1 M bicarbonate buffer, pH 9.6, is added to a 96-well plate (manufactured by Nunc), and is incubated overnight at 4°C to immobilize the antibody.

After blocking, 100 µl each of appropriately diluted antibody for use in the present invention or a sample containing the antibody, or 100 µl of human IgG (manufactured by CAPPEL) as the standard is added, and incubated at room temperature for 1 hour. After washing, 100 µl of 5000-fold diluted alkaline phosphatase-labeled anti-human IgG antibody (manufactured by BIO SOURCE) is added, and incubated at room temperature for 1 hour. After washing, the substrate solution is added and incubated, followed by the measurement of absorbance at 405 nm using the MICROPLATE READER Model 3550 (manufactured by Bio-Rad) to calculate the concentration of the desired antibody.

### (VI) Pharmaceutical composition containing the antibody of the present invention

The invention also provides an agent for the prophylaxis and/or treatment of inflammatory bowel diseases (IBD), multiple sclerosis, psoriasis, or hematological cancers. The term "treatment" includes not only complete cure but also amelioration of a symptom. It is known that CD81 is overexpressed in an IBD patient and an anti-CD81 antibody is useful for preventing, ameliorating or treating IBD. Herein, inflammatory bowel diseases (IBD)" means diseases including ulcerative colitis and Crohn's disease.
Since the present inventors obtained new findings that the anti-CD81 antibodies suppressed T cell migration, the antibody of the present invention is also useful for preventing, improving or treating a disease associated with T cell migration such as multiple sclerosis and psoriasis.

Since the antibody of the present invention does not enhance the production of cytokines such as interleukin-2 from T cells, the pharmaceutical composition containing the antibody does not cause adverse side effects due to cytokine overproduction. The effect on the cytokine production in lymphocytes can be determined by a known method, for example, the quantification of cytokines in culture supernatants of lymphocytes cultured under various conditions using ELISA or intracellular cytokine staining of the lymphocytes after treatment, as described in Nature Immunology, VOLUME 8 NUMBER 9 SEPTEMBER 2007, 942.

The present inventors clarified a cytotoxic effect of the antibody of the present invention on cancer cells derived from patients with hematological cancer, and found that the antibody of the present invention is useful as a prophylactic, ameliorating or therapeutic agent for hematological cancers. According to the WHO Classification, hematological cancers are classified into leukemias, malignant lymphoma, multiple myeloma, and myelodysplastic syndrome. Leukemias are further classified into acute myelogenous leukemia, acute lymphatic leukemia, chronic myelogenous leukemia, and chronic lymphatic leukemia. Malignant lymphomas are classified into Hodgkin's lymphoma and non-Hodgkin's lymphoma.

Acute myelogenous leukemia is the most prevalent type of adult leukemia. Although acute lymphatic leukemia is relatively prevalent in children, it occurs at a certain incidence in adults as well. Chronic myelogenous leukemia is a disease for which therapeutic outcomes have improved dramatically in recent years. Chronic lymphatic leukemia is a type of leukemia relatively prevalent in Europe and the US, but not less prevalent in Japanese. Of malignant lymphomas, non-Hodgkin's lymphomas include adult T cell lymphoma, lymphoblastic lymphoma, diffuse large-cell lymphoma, Burkitt's lymphoma, follicular lymphoma, MALT lymphoma, peripheral T cell lymphoma, mantle cell lymphoma and the like. MALT lymphoma and follicular lymphoma are tumors of low malignancy that advances so slowly that no major advance occurs during many years. In contrast, Burkitt's lymphoma, lymphoblastic lymphoma and adult T cell lymphoma are tumors of extremely high malignancy that aggravate week by week. Other types positioned therebetween are peripheral T cell lymphoma, diffuse large-cell lymphoma, and mantle cell lymphoma. These tumors advance month by month. Multiple myeloma is a type of tumor in the stage of plasma cells resulting from maturation of B lymphocytes to produce immunoglobulins. Myelodysplastic syndrome generically refers to a class of diseases characterized by morphological and functional abnormalities of bone marrow stem cells.

Available treatments for hematological cancers include chemotherapies, radiotherapies, molecule-targeting treatments, and high-dose chemotherapies in combination with hematopoietic stem cell transplantation. While pediatric acute lymphatic leukemia is treatable with a long survival rate of 80%, adult acute lymphatic leukemia has a low long survival rate of 15-35%, although complete remission is achieved in 60-80% of the patients. The therapeutic outcomes for chronic lymphatic leukemia have been improving since the advent of Gleevec (imatinib mesylate). Improved therapeutic outcomes have been noted in Hodgkin's lymphoma and non-Hodgkin's lymphoma of low or moderate malignancy. Meanwhile, in adult T cell lymphoma, various therapies fail to produce improved therapeutic outcomes, the median survival time being about 1 year (IGAKU NO AYUMI, Vol.212, No.5, pp.461-466, 2005). Lymphoblastic lymphoma is a set of diseases classified as non-Hodgkin's lymphoma of high malignancy. For Burkitt's lymphoma, the prognosis has been improved by short-time high-dose chemotherapy; the 2-year survival rate is currently about 70% or more, demonstrating improved therapeutic outcomes (Magrath, I. et al., J. Clin. Oncol., 14:925-943, 1996; Mead, G.M. et al., Ann. Oncol., 13:1264-1274, 2002); however, the 3-year survival rate remains low at 49%, so that a further improvement in the therapeutic outcomes is needed (Thomas D.A. et al., J. Clin. Oncol., 17:2461-2470, 1999). In diffuse large-cell lymphoma, a combination therapy with an anti-CD20 antibody (rituximab) and CHOP therapy (cyclophosphamide, doxorubicin, vincristine, prednisolone) is becoming a standard treatment with improved therapeutic outcomes for young low-risk patients in the progression stage. However, for young high-risk patients in the progression stage, no therapy surpassing CHOP therapy is available. Regarding the treatment of multiple myeloma, anticancer agents are somewhat effective, but this disease is highly malignant so that the treatment is not as effective as for leukemia and lymphoma. For myelodysplastic syndrome, the 5-to 10-year survival rate is about 30-40%. Hematological cancers of high malignancy for which further improvements in the therapeutic efficacy are expected include acute myelogenous leukemia, acute lymphatic leukemia, lymphoblastic lymphoma, diffuse large-cell lymphoma, Burkitt's lymphoma, mantle cell lymphoma, peripheral T cell lymphoma, adult T cell lymphoma, multiple myeloma, myelodysplastic syndrome and the like.

As stated above, no therapeutic methods or drugs are available with satisfactory therapeutic efficacy for hematological cancers of moderate to high malignancy; there is a demand for a novel therapeutic method and drug. It was found that anti-CD81 antibodies, whose potential as therapeutic drugs for hematological cancers has been unclear so far, have cytotoxic effects on some types of hematological cancers for which the therapeutic efficacy is lacked, i.e., Jurkat cells (a cancer cell line derived from a patient with acute lymphatic leukemia) and Ramos cells (a cancer cell line derived from a patient with Burkitt's lymphoma), based on their complement-dependent cytotoxicity. The finding that the anti-CD81 antibody exhibits a cytotoxic effect on cancer cells was not known so far.

Accordingly, the present invention also provides a prophylactic, ameliorating or therapeutic agent for hematological cancers with the antibody of the present invention as an active ingredient, preferably a prophylactic, ameliorating or therapeutic agent for hematological cancers of high malignancy.

The present invention is applicable not only to hematological cancers, but also to any type of cancer cell that expresses CD81. Therefore, drugs with the antibody of the present invention as an active ingredient are effective as a prophylactic, ameliorating or therapeutic agent for cancers caused by CD81-expressing cancer cells.

The agent containing the anti-CD81 antibody as an active ingredient is itself administered as an agent formulated by a known manufacturing pharmaceutical method. For example, it can be used in the form of an injection solution of a sterile solution or suspension with water or other pharmaceutically acceptable liquids. Further, it is considered to be formulated by being properly mixed with pharmacologically acceptable carriers or media, such as sterile water, a physiological saline solution, an emulsifying agent, a suspending agent, a surfactant, a stabilizer, a vehicle and a preservative in the unit dosage form required for formulation, which is generally approved. The amount of the active ingredient in these pharmaceutical preparations is adjusted such that an appropriate volume in the prescribed range is obtained.

The sterile composition for injection can be formed according to ordinary formulation using a vehicle such as distilled water for injection. Examples of the aqueous solution for injection include a physiological saline solution and isotonic solutions containing glucose and other auxiliaries such as D-sorbitol, D-mannose, D-mannitol and sodium chloride. An appropriate solubilizer, for example, an alcohol such as ethanol, polyalcohol such as propylene glycol or polyethylene glycol, a nonionic surfactant such as polysorbate 80™ or HCO-50 may be used in combination.

Examples of oil include sesame oil and soybean oil, and benzyl benzoate or benzyl alcohol may be used in combination as a solubilizer. A buffering agent such as a phosphate buffer or a sodium acetate buffer, a soothing agent such as procaine hydrochloride, a stabilizer such as benzyl alcohol, phenol and an antioxidant may be incorporated. The thus-formed injection solution is usually filled in an appropriate ampule.

Regarding the agent containing the anti-CD81 antibody as an active ingredient, both of the oral administration and the parenteral administration are possible. The parenteral administration is preferable. Specific examples thereof include an injection solution dosage form, a transnasal dosage form, a transpulmonary dosage form, a percutaneous dosage form and the like. Regarding examples of the injection solution dosage form, systemic or local administration can be conducted by intravenous injection, intramuscular injection, intraperitoneal injection, subcutaneous injection or the like.

The dose can properly be selected depending on the age and condition of patients. For example, the dose can be selected in the range of from 0.0001 mg to 1,000 mg per kilogram of the body weight for one administration. Alternatively, the dose can be selected in the range of from 0.001 to 100,000 mg/body for a patient. However, the therapeutic agent of the invention is not limited by these doses.

The present invention is explained in more detail in the following by referring to Examples, which are not to be construed as limitative.

### Examples

### Example 1 Preparation of human CD81 antibody

### (1) Selection of human anti-human CD81 antibody fragment

A complete human antibody library n-CoDeR (Nature Biotechnol. 18: 852-856, 2000; WO98/32845) was screened for specific binding with human CD81, using human CD81 protein and human CD9 protein having a homology with CD81, or human CD81-expressing and -non-expressing cells to obtain human antibody fragments (scFv) specifically binding with human CD81. First, phages bound to biotinylated CD81 protein in the presence of competitive CD9 protein were recovered using streptoavidin-labeled magnetic beads. Unbound phages were washed out and the bound phages were eluted by trypsin treatment. Panning was carried out three times, and the selected phage library was expressed as an antibody fragment in *E*. *coli.* Antibody fragments that bind to CD81-expessing cells but do not bind to CD81-non-expressing cells were screened out using FMAT (Fluorometric Microvolume Assay Techonology). FMAT is performed by adding antibody fragments to the cells immobilized on the wells of 384-well culture plate, and detecting its binding using a fluorescent-labeled anti-scFv antibody. As a result of the screening, two human CD81-specific human antibody fragments were selected. The nucleotide sequences (scFv-002-A07: SEQ ID NO:33, scFv-005-C01: SEQ ID NO:35) and the deduced amino acid sequences (scFv-002-A07: SEQ ID NO:34, scFv-005-C01: SEQ ID NO:36) of these fragments were determined by sequencing.

### (2) Production of anti-human CD81 antibody

Using the sequence information of the fragments obtained (1) above, anti-human CD81 antibody genes (heavy chain variable region (V_{H}): SEQ ID NO:9 (002-A07); SEQ ID NO:19 (005-C01) and light chain variable region (V_{L}): SEQ ID NO:7 (002-A07); SEQ ID NO:17 (005-C01)) were prepared by a known method (Nat Biotechnol., 18, 852-856, 2000), amplified by PCR using a set of primers having BsmI and NheI recognition sites. The amplified fragments were digested with BsmI and NheI and subcloned into pCEP4 (Invitrogen)-derived vectors. In the heavy chain expression vector, a genomic region of mutant human γ4 chain wherein the 228th Ser is substituted with Pro (S228P) is inserted, and in the light chain expression vector, a genomic region of human λ chain is inserted. Thus constructed heavy and light chain expression vectors were co-transfected into HEK293-EBNA cells (ATCC-CRL-10852) using FuGene6 (Roche) according to the manufacture's protocol and the cells were cultured. After 6 days culture, the IgG4 antibodies were purified from the culture supernatants. Briefly, the culture solution was centrifuged and the supernatant was subjected to protein A chromatography. The purified material was dialyzed to 10 mM NaP buffer (0.15 M NaCl, pH 6.5). The 002-A07 IgG4 antibody thus obtained has the heavy chain consisting of the amino acid sequence shown in SEQ ID NO:28 and the light chain consisting of the amino acid sequence shown in SEQ ID NO:26. The 005-C01 IgG4 antibody thus obtained has the heavy chain consisting of the amino acid sequence shown in SEQ ID NO:32 and the light chain consisting of the amino acid sequence shown in SEQ ID NO:30.

### Experimental Example 1 Binding activity of anti-CD81 antibody on CD81-expressing cells

### (1) Cell preparation

Based on known information (JOURNAL OF VIROLOGY., 79, 4316-4328, 2005), Jurkat E6.1 cells and human PBMC (Peripheral Blood Mononuclear cells) were used as human CD81-expressing cells to confirm binding of the antibodies of the present invention obtained in Example 1 to the human CD81-expressing cells. Jurkat E6.1 cells were purchased from European Collection of Cell Cultures (ECACC) (Cat No.88042803). Human PBMC were purchased from KAC Co., Ltd. (KAC). These cells were collected by centrifugation (4,000 rpm, 3 min, 4°C) and suspended in FACS staining buffer (phosphate buffer, 0.09% sodium azide, 1% bovine serum albumin (BSA: Hyclone Laboratories)). The number of viable cells was counted using trypan blue and 10⁶ cells/100 µl were added in an eppendorf tube.

### (2) Cell staining

To the cells prepared in (1) above was added the anti-human CD81 antibody (002-A07 or 005-C01) prepared in Example 1 or a human IgG4 (Acris) as a control (0.1 µg), and the cells were incubated for 20 min at 4°C. After washing the cells with FACS staining buffer, PE (phycoerythrin)-labeled anti-human IgG4 antibody (Beckman coulter; 0.05 µg) was added to the cells and incubated for 15 min at 4°C. After washing with FACS staining buffer twice, the cells were centrifuged (4,000 rpm, 3 min, 4°C), the supernatant was removed and the precipitated cells were fixed with BD Cytefix/Cyteperm buffer (BD Biosciences). After washing, buffer was changed to PBS (NACALAI TESQUE, INC.). The binding ratio (%) of the anti-human CD81 antibody to the cells was analyzed by flow cytometry using FACS Calibur (BD Biosciences) and calculated as the percentage of cells having a PE-derived fluorescent intensity greater than that observed when the control IgG4 is used. The results are shown in Table 1. 002-A07 and 005-C01 strongly bound to Jurkat E6.1 cell and human PBMC.

**Table 1 Binding of anti-human CD81 antibodies to Jurkat and PBMC.**

| | PBMC | Jurkat |
|---|---|---|
| | Binding ratio to cells (%) | |
| control IgG4 | 1 | 1 |
| 002-A07 | 99 | 100 |
| 005-C01 | 98 | 100 |

The data presented are representative of three individual experiments.

### Experimental Example 2 Suppressive effect of anti-human CD81 antibody on chemotaxis of Jurakat cells

### (1) Subculture of Jurkat cells

Jurkat E6.1 cells (purchased from ECACC: Cat No. 88042803) were maintained in RPMI1640 medium (GIBCO) supplemented with 10% fetal calf serum (FCS: Hyclone Laboratories) in humidified incubators in 5% CO₂ at 37°C. Cell density was kept between 2x10⁵ and 1x10⁶ cells/ml by diluting with the medium.

### (2) Antibody treatment

Jurkat E6.1 cells were suspended at 4x10⁶ cells/ml in chemotaxis medium (RPMI1640 (GIBCO) containing 0.5% BSA (SIGMA), 50 mM HEPES (GIBCO)). The anti-human CD81 antibody (002-A07 or 005-C01) or control human IgG4 (Acris Antibodies GmbH) was added to the cells at the final concentrations described in Table 2. The cells were incubated for 2 hours in humidified incubators in 5% CO₂ at 37°C.

### (3) chemotaxis assay

Chemotaxis was examined using a 96-well chemotaxis chamber (Corning Inc, 5 µm pore size). The lower wells were filled with 235 µl of chemotaxis medium in the presence of 10ng/ml SDF-1 (PEPROTECH). 75 µl of preincubated Jurkat cells with or without anti-human CD81 antibody were loaded onto the upper wells and incubated for 2 hours in humidified incubators in 5% CO₂ at 37°C. After incubation, 50 µl of lower well cell suspension was transferred to 96 well black plate (Corning Inc) and added 50 µl of ATP lite (Perkin Elmer). The number of the migrated cells was calculated by measuring luminescence intensity using Envision 2102 multilabelreader (Perkin Elmer).

### (4) Analysis of chemotaxis assay

Percent Chemotaxis was calculated as the percentage of the number of the migrated Jurkat cells when the anti-human CD81 antibody was added, to the number of the migrated Jurkat cells when control IgG4 was added. As a result, both anti-human CD81 antibodies inhibited chemotaxis of Jurkat cells (Table 2). Since the suppressing effect of 002-A07 and 005-C01 on T cell migration of Jurkat cells was observed, it was confirmed that the anti-CD81 antibodies (002-A07 and 005-C01) can be a therapeutic agent of IBD.

**Table 2 Suppressive effect of 002-A07 and 005-C01 on chemotaxis of Jurkat cells**

| | Jurkat cell chemotaxis (%) | | | | |
|---|---|---|---|---|---|
| antibody conc. (µg/ml) | 1 | 0.5 | 0.2 | 0.04 | 0.005 |
| control IgG4 | 100 | 100 | 100 | 100 | 100 |
| 002-A07 | 18 | 9 | 20 | 51 | 95 |
| 005-A01 | 20 | 42 | 44 | 82 | 102 |

The data presented are average value of three individual experiments.

### Experimental Example 3 Suppressive effect of anti-human CD81 antibody on chemotaxis of human PBMC (peripheral blood mononuclear cells)

Suppressive effect of anti-human CD81 antibodies on chemotaxis of human PBMC was examined according to the method described in Biologicals, 2007 Oct;35(4):227-33, Epub 2007 Aug 28.

### (1) Cell preparation

Human PBMC was purchased from KAC. According to the attached information from KAC, 60% of the human PBMC used was T cells (CD3-positive cells). The human PBMC were maintained in RPMI1640 medium (GIBCO) supplemented with 10% fetal calf serum (FCS: Hyclone Laboratories) in humidified incubators in 5% CO₂ at 37°C. The cells (1x10⁶ cells/ml) were incubated with 5 µg/ml phytohemagglutinin (PHA, Wako) and 50 ng/ml human recombinant IL-2 (R&D system) for 4 days in humidified incubators in 5% CO₂ at 37°C.

### (2) Antibody treatment

Human PBMC were suspended at 4x10⁶ cells/ml in chemotaxis medium (RPMI1640 (GIBCO) containing 0.5% BSA (SIGMA), 50 mM HEPES (GIBCO)). The anti-human CD81 antibody (002-A07 or 005-C01) or control human IgG4 (Acris Antibodies GmbH) was added to the cells at the concentrations described in Table 3. The cells were incubated for 2 hours in humidified incubators in 5% CO₂ at 37°C.

### (3) Chemotaxis assay

Chemotaxis was examined using a 96-well chemotaxis chamber (corning coster, 5 µm pore size). The lower wells were filled with 235 µl of chemotaxis medium in the presence of 50 ng/ml SDF-1. 75 µl of preincubated human PBMC with or without anti-human CD81 antibody were loaded onto the upper wells and incubated for 2 hours in humidified incubators in 5% CO₂ at 37°C. After incubation, 50 µl of lower well cell suspension was transferred to 96 well black plate (Corning Inc) and added 50 µl of ATP lite (Perkin Elmer). The number of migrated cells was calculated by measuring luminescence intensity using Envision 2102 multilabelreader (Perkin Elmer).

### (4) Analysis of chemotaxis assay

Percent Chemotaxis was calculated as the percentage of the number of the migrated human PBMC when the anti-human CD81 antibody was added, to the number of the migrated human PBMC when control IgG4 was added. As a result, both anti-human CD81 antibodies inhibited chemotaxis of human PBMC (Table 3). Since the suppressing effect of 002-A07 and 005-C01 on T cell migration of human PBMC containing primary human T cells was observed, it was confirmed that the anti-CD81 antibodies (002-A07 and 005-C01) can be a therapeutic agent of IBD.

**Table 3 Suppressive effect of 002-A07 and 005-C01 on chemotaxis of human PBMC**

| | human PBMC chemotaxis (%) | | | |
|---|---|---|---|---|
| antibody conc. (µg/ml) | 5 | 1 | 0.2 | 0.04 |
| control IgG4 | 100 | 100 | 100 | 100 |
| 002-A07 | 0 | 3 | 17 | 59 |
| 005-C01 | 27 | 58 | 63 | 87 |

### Experimental Example 4 Effects of anti-human CD81 antibody on cytokine production in and cell proliferation of human PBMC

The cytokine production in human PBMC was determined according to Life Sci. 2001 Nov 21;70(1):81-95.

### (1) Cell culture and stimulation

Human PBMC was purchased from KAC. According to the attached information from KAC, 60% of the human PBMC used was T cells (CD3-positive cells). The human PBMC were maintained in RPMI1640 medium (GIBCO) supplemented with 10% fetal calf serum (FCS: Hyclone Laboratories) in humidified incubators in 5% CO₂ at 37°C. To 96-well plate (IWAKI) was added the anti-human CD81 antibody (002-A07 or 005-C01) or control IgG4 (Acris Antibodies GmbH) at the concentrations described in Table 4, and the human PBMC (1x10⁵ cells/ml) were added to each well (100 µl/well). To the cells were added 1000 ng/well anti CD3 antibody (Clone:OKT3, Bio Legend) and 1000 ng/well anti-CD28 antibody (BD Biosciences) (50 µl/well each) and incubated for 48 hours in humidified incubators in 5% CO₂ at 37°C. After incubation, the IL-2 level of supernatants was measured by ELISA assay.

### (2) IL-2 ELISA assay

IL-2 concentration produced from human PBMC to which antibody was added was measured by human IL-2 ELISA kit (R&D system). ELISA assay was conducted according to the manufacture's protocol. Percent IL-2 production was calculated as the percentage of the IL-2 level produced from human PBMC when the anti-human CD81 antibody was added, to the IL-2 level produced from human PBMC when control IgG4 was added. The results are shown in Table 4.

### (3) Alamar blue assay

Cell proliferation was measured by Alamar blue (BIOSOURCE). This assay was conducted according to the manufacture's protocol. Percent cell proliferation was calculated as the percentage of the cell proliferation of human PBMC when the anti-human CD81 antibody was added, to the cell proliferation of human PBMC when control IgG4 was added. The results are shown in Table 5.

**Table 4 Effect of 002-A07 and 005-C01 on IL-2 production in human PBMC**

| | human PBMC IL-2 production (%) | | | |
|---|---|---|---|---|
| antibody conc. (µg/ml) | 5 | 1 | 0.2 | 0.04 |
| control IgG4 | 100 | 100 | 100 | 100 |
| 002-A07 | 123 | 108 | 110 | 98 |
| 005-C01 | 110 | 100 | 101 | 104 |

**Table 5 Effect of 002-A07 and 005-C01 on cell proliferation of human PBMC**

| | human PBMC cell proliferation (%) | | | |
|---|---|---|---|---|
| antibody conc. (µg/ml) | 5 | 1 | 0.2 | 0.04 |
| control IgG4 | 100 | 100 | 100 | 100 |
| 002-A07 | 107 | 104 | 105 | 105 |
| 005-C01 | 114 | 102 | 103 | 104 |

These results demonstrate that both anti-human CD81 antibodies do not stimulate cytokine production in or cell proliferation of human PBMC. Thus, 002-A07 and 005-C01 do not affect cytokine production or cell proliferation that is an index for T cell activation. Accordingly, it was confirmed that the antibodies have no concern for side effect such as cytokine storm due to cytokine overproduction or immunosuppression due to the suppression of T cell function.

### Experimental Example 5 Suppressive effect of anti-human CD81 antibody on chemotaxis of PBMC from IBD patients

### (1) Cell preparation

IBD patient PBMC (purchased from Tissue solution and the like) are maintained in RPMI1640 medium (GIBCO) supplemented with 10% fetal calf serum (FCS: Hyclone Laboratories) in humidified incubators in 5% CO₂ at 37°C. The cells are incubated with or without phytohemagglutinin (PHA), human recombinant IL-2, human TNFα, vasoactive intestinal peptide (VIP), IL-22 and IL-7 for 4 days in humidified incubators in 5% CO₂ at 37°C.

### (2) Antibody treatment

The IBD patient's PBMC are suspended in chemotaxis medium (RPMI1640 (GIBCO) containing 0.5% BSA (SIGMA), 50 mM HEPES (GIBCO)). The anti-human CD81 antibody (002-A07 or 005-C01) or control human IgG4 (Acris Antibodies GmbH) is added to the cells at various concentrations. The cells were incubated for 2 hours in humidified incubators in 5% CO₂ at 37°C.

### (3) Chemotaxis assay

Chemotaxis is examined using a 96-well chemotaxis chamber (corning coster, 5 µm pore size). The lower wells are filled with 235 µl of chemotaxis medium in the presence of SDF-1. 75 µl of preincubated IBD patient PBMC with or without anti-human CD81 antibody are loaded onto the upper wells and incubated for 2 hours in humidified incubators in 5% CO₂ at 37°C. After incubation, 50 µl of lower well cell suspension is transferred to 96 well black plate (Corning Inc) and added 50 µl of ATP lite (Perkin Elmer). The number of the migrated cells is calculated by measuring luminescence intensity using Envision 2102 multilabelreader (Perkin Elmer).

### (4) Analysis of Chemotaxis assay

Percent chemotaxis is calculated as the percentage of the number of the migrated IBD patient PBMC when the anti-human CD81 antibody is added, to the number of the migrated IBD patient PBMC when control IgG4 is added.

### Experimental Example 6 Effect of anti-human CD81 antibody on cytokine production in IBD patient PBMC

### (1) Cell culture and stimulation

IBD patient's PBMC (purchased from Tissue solution and the like) are maintained in RPMI1640 medium (GIBCO) supplemented with 10% fetal calf serum (FCS: Hyclone Laboratories) in humidified incubators in 5% CO₂ at 37°C. To 96-well plate (IWAKI) is added the anti-human CD81 antibody (002-A07 or 005-C01) or control IgG4 (Acris Antibodies GmbH) at various concentrations, and the IBD patient PBMC are added to each well. To the cells are added 1000 ng/well anti CD3 antibody (Clone:OKT3, Bio Legend) and 1000 ng/well anti-CD28 antibody (BD Biosciences) (50 µl/well each) and incubated for 48 hours in humidified incubators in 5% CO₂ at 37°C. After incubation, the IL-2 level of supernatants is measured by ELISA assay.

### (2) IL-2 ELISA assay

IL-2 concentration produced from IBD patient's PBMC to which antibody is added is measured by human IL-2 ELISA kit (R&D system). ELISA assay is conducted according to the manufacture's protocol. Percent IL-2 production is calculated as the percentage of the IL-2 level produced from human PBMC when the anti-human CD81 antibody is added, to the IL-2 level produced from human PBMC when control IgG4 is added.

### Experimental Example 7 Epitope mapping of anti-human GD81 antibody using human and chicken CD81 chimeras

To identify the epitopes to which each of the anti-human CD81 antibodies (002-A07 and 005-C01) bind, ELISA assays were performed using human-chicken CD81 chimera constructs listed in Table 6. Each construct was transiently expressed in CHO cells and its cell membrane fraction was solubilized with detergents and immobilized onto a plate. The results are summarized in Table 7.

### (1) Expression of chimera CD81 proteins

Using human CD81 gene (SEQ ID NO:21) and chicken CD81 gene (SEQ ID NO:23), 10 types of chimera CD81 genes depicted in Table 6 were constructed. Each chimera gene was subcloned into pcDNA-DEST40 vector so as to express as a fusion protein having V5 and 6×His tags at the C-terminal. Each chimera construct was transiently expressed in CHO cells with Trans-IT LT-1 (TAKARA BIO, Code MIR2304) according to the manufacture's protocol, and 48 hours after, the membrane fraction was prepared by following procedure. To the CD81 chimera-expressing CHO cells was added HBS buffer (20 mM Hepes (Invitrogen), 150 mM NaCl (NACALAI TESQUE, INC.)) containing 1% (w/v) n-Octylglucoside (NACALAI TESQUE, INC.), and the cells were incubated for 5 min at 4°C. Then, the cell suspension was centrifuged for 20 min at 10000 g and the supernatant was collected. The protein content in the supernatant was determined with BCA Protein Assay Kit (Thermo Scientific Pierce, code:23225).

### (2) ELISA assay

As control antibodies, human IgG4 antibody (Acris Antibodies GmbH) and mouse IgG antibody were used. Human-chicken chimera protein was captured via His tag introduced into the C-terminal by adding the membrane fraction (5 µg/100µL/well) prepared (1) above to Ni NTA His Sorb plate (QIAGEN) in the presence of 1% (w/v) n-Octylglucoside and incubating for 3 hours at 4°C. After washing with TBST (TBS, 0.05% (v/v) Tween 20) three times, an anti-human CD81 antibody or a control antibody (50 µL/well) was added and incubated for 1 hour at room temperature (RT). After washing, 2000 fold-diluted HRP-labeled anti-human IgG4 antibody (Mouse anti-human IgG4 HRP clone:HP6023 Beckman Coulter) or 1000 fold-diluted HRP-labeled anti-mouse IgG antibody (Invitrogen) (50 µL/well) were added. The plate was incubated for 1 hour at RT and washed with TBST three times. And then, peroxidase activity was determined by TMB One solution (Promega; Code 53025). The reaction was stopped with 2 M H₂SO₄ (50 µL) and absorbance at 450 nm was measured.

### (3) Interpretation of results

Human CD81 have two extracellular domains. 002-A07 and 005-C01 bound to hCD81, c80h, h175c and h190c, but did not bind to cCD81, c138h, c156h, c175h, c190h, h80c, h138c and h156c. These results indicate that the epitopes recognized by 002-A07 and 005-C01 antibodies binding to human CD81 are present between the 80th amino acid residue and 175th amino acid residue of human CD81 polypeptide.

**Table 6 human-chicken CD81 chimeras**

| | human CD81 region | chicken CD81 region |
|---|---|---|
| hCD81 (SEQ ID NO:22) | 1-236 | - |
| cCD81 (SEQ ID NO:24) | - | 1-237 |
| h80c | 1-80 | 81-237 |
| h138c | 1-138 | 139-237 |
| h156c | 1-156 | 158-237 |
| h175c | 1-175 | 177-237 |
| h190c | 1-190 | 192-237 |
| c80h | 81-236 | 1-80 |
| c138h | 139-236 | 1-138 |
| c156h | 157-236 | 1-157 |
| c175h | 176-236 | 1-176 |
| c190h | 191-236 | 1-191 |

Each number shows the position of amino acid residues.

**Table 7 Epitope mapping using human-chicken CD81 chimeras**

| CD81 antigen construct | | hCD81 | cCD81 | c80h | c138h | c156h | c175h | c190h | h80c | h138c | h156c | h175c | h190c |
|---|---|---|---|---|---|---|---|---|---|---|---|---|---|
| Antibody | 002-A07 | ○ | × | ○ | × | × | × | × | × | × | × | ○ | ○ |
| | 005-C01 | ○ | × | ○ | × | × | × | × | × | × | × | ○ | ○ |

| | | | | | | | | | | | | | |
|---|---|---|---|---|---|---|---|---|---|---|---|---|---|
| ○: Absorbance at 450 nm is 100-10% of that in the case CD81 is hCD81 ×: Absorbance at 450 nm is less than 10% of that in the case CD81 is hCD81 | | | | | | | | | | | | | |

### Experimental Example 8 Epitope mapping of anti-human CD81 antibody using alanine-scanning

As shown in Experimental Example 7, the epitope region of human CD81 for 002-A07 and 005-C01 antibodies was determined. In this Example, in order to assess the contribution of individual amino acid residues in the epitope region to binding with the anti-human CD81 antibodies and identify detailed epitope residues, alanine-scanning mutagenesis (Cunningham & Wells, Science 244: 1081-1085 (1989)) was carried out in the sequence of the epitope region determined in Experimental Example 7. Human CD81 mutants were prepared by site-directed mutagenesis (this work was out sourced to TAKARA BIO) and subcloned into pcDNA-DEST40 vector. Subsequently, these mutants were expressed in CHO cells and the binding of 002-A07 and 005-C01 antibodies with individual mutants was assayed in the same manner as in Experimental Example 7. Relative binding affinity was calculated as the percentage of binding affinity of 002-A07 or 005-C01 antibody with each mutant to binding affinity of 002-A07 or 005-C01 antibody with wild type human CD81, and the mutants were classified into 3 groups based on the relative binding affinity. The expression levels of the individual mutants were corrected by the expression levels of V5 tag which was inserted into the C-terminal of the mutants. The results are summarized in Table 8.
As shown in Experimental Example 7, the anti-human CD81 antibodies 002-A07 and 005-C01 recognize the same epitope region that is located between the 80th and 175th amino acid residues of human CD81. The results of alanine scanning indicate that human CD81 has 13 amino acid residues (V135, D137, A143, H151, G158, T163, A164, L165, S168, V169, L170, N172 and L174) critical for binding with 002-A07 and 17 amino acid residues (Y127, A130, L131, V135, V136, D137, N142, A143, L154, G158, T163, L165, S168, V169, L170, K171 and L174) critical for binding with 005-C01 within the epitope region, respectively.

**Table 8 Epitope mapping of anti-human CD81 antibodies by alanine scanning**

| Amino acid residue | | | 002-A07 | 005-C01 |
|---|---|---|---|---|
| No. | Wild type (codon) | Substitutions (codon) | | |
| 127 | Y (TAT) | F (TTC) | ○ | Δ |
| 130 | A (GCC) | T (ACC) | ○ | Δ |
| 131 | L (CTA) | A (GCC) | ○ | × |
| 135 | V (GTC) | A (GCC) | × | × |
| 136 | V (GTC) | A (GCC) | ○ | Δ |
| 137 | D (GAT) | A (GCC) | × | × |
| 142 | N (AAC) | A (GCC) | ○ | Δ |
| 143 | A (GCC) | T (ACC) | Δ | × |
| 151 | H (CAC) | A (GCC) | Δ | ○ |
| 154 | L (CTT) | A (GCC) | ○ | × |
| 158 | G (GGC) | S (AGC) | × | × |
| 163 | T (ACT) | A (GCC) | Δ | Δ |
| 164 | A (GCT) | T (ACC) | × | ○ |
| 165 | L (TTG) | A (GCC) | × | × |
| 168 | S (TCA) | A (GCC) | Δ | × |
| 169 | V (GTG) | A (GCC) | × | × |
| 170 | L (CTC) | A (GCC) | Δ | × |
| 171 | K (AAG) | A (GCC) | ○ | Δ |
| 172 | N (AAC) | A (GCC) | Δ | ○ |
| 174 | L (TTG) | A (GCC) | Δ | × |

| | | | | |
|---|---|---|---|---|
| Each symbol shows; ○: relative binding affinity is equal or slightly weak compared to wild type human CD81 (relative binding affinity is ranged 40-100%). Δ: relative binding affinity is significantly weak compared to wild type human CD81 (relative binding affinity is ranged 20-40%). ×: relative binding affinity is quite weak compared to wild type human CD81 (relative binding affinity is ranged under 20 %). (Since the 81st to 112nd amino acid residues are transmembrane or intracellular domains, alanine mutants were not prepared.) | | | | |

### Experimental Example 9 Epitope mapping of the anti-human CD81 antibody using homolog-scanning

Since alanine-scanning may cause unwanted conformational change and physicochemical disruptions (Cunningham and Wells, Science 244: 1081-1085 (1989)), we conducted homolog-scanning which is other type of systematic scanning to further increase the resolution of energetic profiling of functional epitopes. The homolog-scanning mutagenesis is designed to minimize the possibility of structural disruption upon side chain substitution by introducing a substitutional group so as to maintain the structure and function of protein (Protein Science (2005), 14:2405-2413).
As shown in Experimental Example 8, human CD81 has 13 and 17 amino acid residues critical for binding with 002-A07 and 005-C01, respectively, within the epitope region. Homolog mutants were prepared in these residues according to the rule described by Protein Science (2005), 14:2405-2413). These homolog mutants were expressed in CHO cells and the binding of the anti-human CD81 antibodies with the individual mutants was assayed in the same manner as Experimental Example 8.
The results are summarized in Table 9. The homolog-scanning revealed that human CD81 has 9 amino acid residues critical for binding with 002-A07 and 9 amino acid residues critical for binding with 005-C01, respectively, within the epitope region. The 151st, 164th, 168th and 172nd residues are specifically critical for binding with 002-A07. On the other hand, the 127th, 130th, 143rd and 154th residues are specifically critical for binding with 005-C01.

**Table 9 Epitope mapping of anti-human CD81 antibodies by homolog-scanning**

| **002-A07** | | | | |
|---|---|---|---|---|
| Amino acid residue | | | Homo | Ala |
| No. | Wild type (codon) | Substitutions (codon) | | |
| 135 | V (GTC) | L (CTG) | × | × |
| 137 | D (GAT) | E (GAG) | × | × |
| 143 | A (GCC) | V (GTG) | ○ | Δ |
| 151 | H (CAC) | R (CGC) | Δ | Δ |
| 158 | G (GGC) | A (GCC) | × | × |
| 163 | T (ACT) | S (TCT) | ○ | Δ |
| 164 | A (GCT) | V (GTG) | × | × |
| 165 | L (TTG) | I (ATC) | ○ | × |
| 168 | S (TCA) | T (ACA) | × | Δ |
| 169 | V (GTG) | L (CTG) | × | × |
| 170 | L (CTC) | I (ATC) | Δ | Δ |
| 172 | N (AAC) | Q (CAG) | Δ | Δ |
| 174 | L (TTG) | I (ATC) | ○ | Δ |
| | | | | |

| **005-C01** | | | | |
|---|---|---|---|---|
| Amino acid residue | | | Homo | Ala |
| No. | Wild type (codon) | Substitutions (codon) | | |
| 127 | Y (TAT) | W (TGG) | × | Δ |
| 130 | A (GCC) | V (GTG) | × | Δ |
| 131 | L (CTA) | I (ATC) | ○ | × |
| 135 | V (GTC) | L (CTG) | × | × |
| 136 | V (GTC) | L (CTG) | ○ | Δ |
| 137 | D (GAT) | E (GAG) | × | × |
| 142 | N (AAC) | Q (CAG) | ○ | Δ |
| 143 | A (GCC) | V (GTG) | Δ | × |
| 154 | L (CTT) | I (ATC) | Δ | × |
| 158 | G (GGC) | A (GCC) | × | × |
| 163 | T (ACT) | S (TCT) | ○ | Δ |
| 165 | L (TTG) | I (ATC) | ○ | × |
| 168 | S (TCA) | T (ACA) | ○ | × |
| 169 | V (GTG) | L (CTG) | × | × |
| 170 | L (CTC) | I (ATC) | Δ | × |
| 171 | K (AAG) | R (AGG) | ○ | Δ |
| 174 | L (TTG) | I (ATC) | ○ | × |

| | | | | |
|---|---|---|---|---|
| Each symbol shows; ○: relative binding affinity is equal or slightly weak compared to wild type human CD81 (relative binding affinity is ranged 40-100%). A: relative binding affinity is significantly weak compared to wild type human CD81 (relative binding affinity is ranged 20-40%). ×: relative binding affinity is quite weak compared to wild type human CD81 (relative binding affinity is ranged under 20%). Homo: homolog mutants; Ala: alanine mutants | | | | |

### Example 2: Generating 002-A07 mutant antibodies

### Construction of glycosylation site mutants

The VL region from n-CoDeR (Registered Trade Mark) clone 002-A07 was modified to eliminate the potential N-glycosylation site (amino acid sequence: NLS) located in CDR3. Six VL variants were designed and purchased from Geneart AG (Regensburg Germany): N113S, N113G, N113Q, S115A, S115G and S115N. These were inserted into an expression vector containing the λ constant region as described in Example 1. The six variant light chains were transfected, together with the 002-A07 γ4 S228P heavy chain, into HEK293-EBNA cells. Antibodies were expressed and purified as described in Example 1.

### Isolation of clones from affinity maturation

### Library construction

A mutagenized library was created for n-CoDeR (Registered Trade Mark) clone 002-A07. Plasmid DNA was used as template in an error-prone PCR protocol (Saviranta et *al.* 1998) where the mutations are introduced randomly over the entire antibody variable regions. The resulting fragments were ligated into a phagemid vector and electroporated into *E*. *coli* HB101F' (constructed by conjugation of the F' plasmid into strain HB101 (Invitrogen)) for the construction of a Fab library. The library was stored as bacterial glycerol stocks at -80°C. (Saviranta P, Pajunen M, Jauria P, Karp M, Pettersson K, Mäntsälä P and Lövgren T (1998). "Engineering the steroid-specificity of an anti-17β-estradiol Fab by random mutagenesis and competitive phage panning". Prot Eng 11(2) 143-152.)

### Phage display panning and screening of individual soluble Fab

Phages with Fab display were expressed from the *E*. *coli* library using helper phage R408 (Stratagene) and purified from the culture supernatant using PEG precipitation.

CD81-specific clones with improved affinity were isolated by phage display technology. Two parallel panning strategies (A and B), each consisting of two consecutive pannings, were used to isolate clones with improved affinity. In strategy A purified recombinant human CD81 protein coated on polystyrene beads was used as target. In strategy B Jurkat cells with endogenous expression of human CD81 were used as target. Unbound phages were removed by washing. Target binding Fab-phages were eluted using trypsin and amplified in *E. coli* HB101F'.

Phagemid DNA was isolated from the amplified pool of clones after panning 2. Gene III was excised by restriction enzyme digestion followed by re-ligation resulting in a Fab expressing plasmid pool. Plasmids were transformed into *E*. *coli* TOP10 (Invitrogen) and transformants expressing individual soluble Fab selected on antibiotic-containing agar plates. Individual bacterial colonies were transferred from agar plates to microtiter plates for expression of soluble Fab with C-terminal His-tag.

Primary screening was performed using an ELISA set-up with sequential addition of the following reagents: 1) coating of monoclonal anti-His antibody; 2) His-tagged Fab from affinity maturation; 3) FLAG-tagged recombinant human CD81 protein; 4) AP-conjugated anti-FLAG antibody; 5) Luminescence substrate. Clones with the highest activity in the primary screening were cherry picked to new microtiter plates and re-expressed. A secondary screening was performed with the same ELISA set-up as described for the primary screening. Clones with improved binding compared to the parent clone 002-A07 in Fab format were analyzed by DNA sequencing. Unique Fab clones were purified from *E. coli* periplasm using Ni-NTA chromatography. Affinity ranking of purified Fab clones was performed using flow cytometry with Jurkat cells and Biacore with immobilized recombinant human CD81 protein.

### Production of IgG4-S228P

Clones with improved affinity compared to the parent clone were converted to IgG4-S228P format, expressed and purified as described in Example 1.

The sequences of the 002-A07 mutant antibodies obtained, determined by DNA sequencing, are shown in the sequence listing according to the correspondence in the table 10 below.

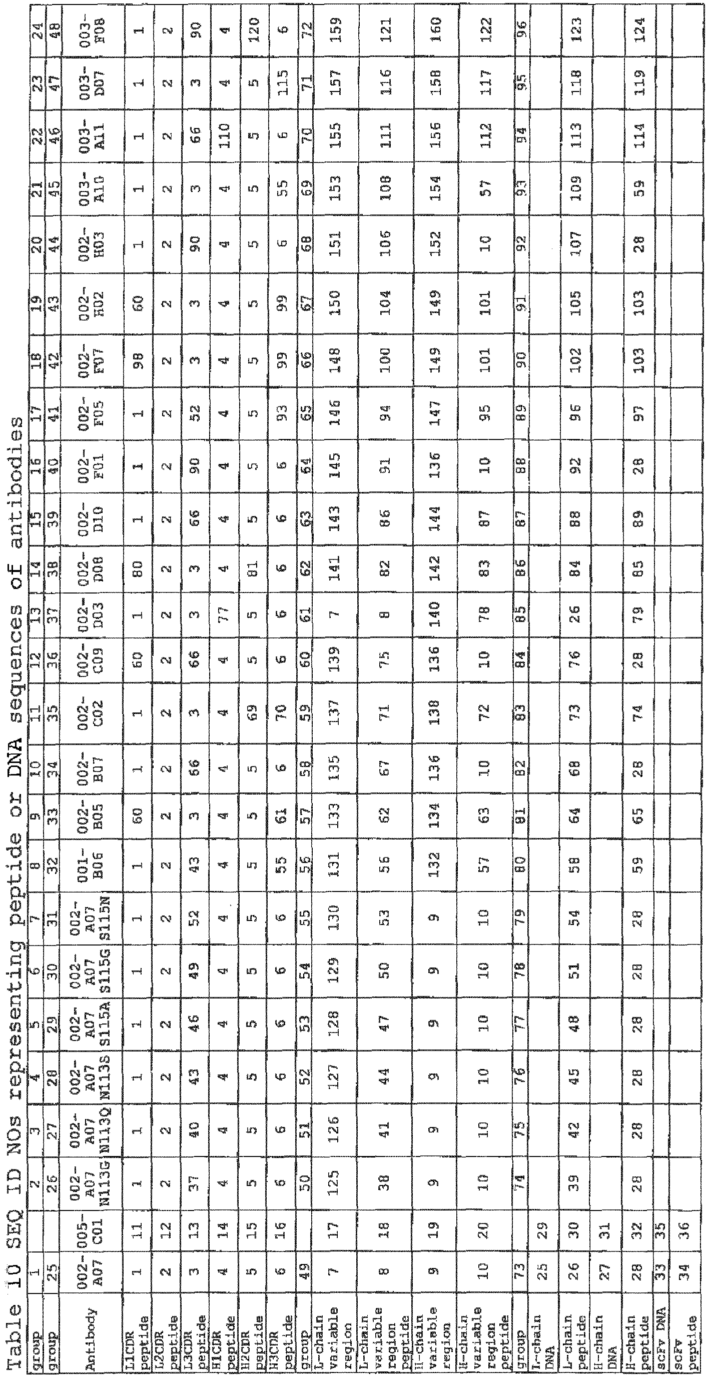

### Experimental Example 10: Suppressive effects of 002-A07 mutant antibodies on chemotaxis of Jurkat cells

This experiment was performed in the same manner as Experimental Example 2. As a result, all 002-A07 mutant antibodies exhibited suppressive effects on chemotaxis of Jurkat cells (a human T cell line) (Table 11).

**Table 11 Suppressive effects of 002-A07 mutant antibodies on chemotaxis of Jurkat cells**

| antibody | 10 µg/mL | 1 µg/mL | 0.1 µg/mL | 0.01 µg/mL | 0.001 µg/mL |
|---|---|---|---|---|---|
| control IgG4 | 100 | 100 | 100 | 100 | 100 |
| 002-A07 | 3 | -2 | 21 | 93 | 90 |
| 001-B06 | 3 | 3 | 35 | 91 | 102 |
| 002-B05 | -10 | -1 | 10 | 78 | 84 |
| 002-B07 | -9 | 4 | 8 | 87 | 92 |
| 002-C02 | -4 | -5 | 31 | 104 | 106 |
| 002-C09 | 11 | -1 | 15 | 82 | 101 |
| 002-D03 | 16 | 14 | 25 | 91 | 105 |
| 002-D08 | 14 | 19 | 67 | 105 | 101 |
| 002-D10 | 10 | 6 | 34 | 87 | 97 |
| 002-F01 | 9 | 4 | 35 | 87 | 89 |
| 002-F05 | 7 | 5 | 27 | 79 | 102 |
| 002-F07 | 15 | 13 | 38 | 88 | 101 |
| 002-H02 | -2 | -10 | -1 | 85 | 87 |
| 002-H03 | -3 | 0 | 13 | 89 | 106 |
| 003-A10 | 3 | 3 | 15 | 93 | 104 |
| 003-A11 | 2 | 1 | 16 | 86 | 91 |
| 003-D07 | -9 | -5 | 42 | 79 | 87 |
| 003-F08 | -4 | -3 | 21 | 79 | 80 |
| 002-A07 N113G | 7 | 13 | 38 | 101 | 99 |
| 002-A07 N113Q | 12 | 13 | 37 | 99 | 104 |
| 002-A07 N113S | 12 | 13 | 40 | 101 | 99 |
| 002-A07 S115A | -9 | -4 | 32 | 78 | 82 |
| 002-A07 S115G | -3 | -9 | 13 | 74 | 84 |
| 002-A07 S115N | -9 | -11 | -4 | 69 | 79 |

### Experimental Example 11: Effects of 002-A07 mutant antibodies on cytokine production by and cell proliferation of human PBMC

This experiment was performed in the same manner as Experimental Example 4. 5A6, a commercially available mouse anti-human CD81 antibody (Santa Cruz Co.) that enhances IL-2 production while suppressing T cell migration, was used for control. As a result, none of the 002-A07 mutant antibodies enhanced IL-2 production by human PBMCs (Table 12), nor did they have any noticeable effect on the cell proliferation (Table 13).

**Table 12 Effects of 002-A07 mutant antibodies on IL-2 production by human PBMC**

| antibody | 10 µg/mL | 1 µg/mL |
|---|---|---|
| mouse IgG | 100 | 100 |
| 5A6 | 320 | 179 |
| human IgG4 | 100 | 100 |
| 002-A07 | 109 | 111 |
| 001-B06 | 174 | 130 |
| 002-B05 | 59 | 109 |
| 002-B07 | 67 | 100 |
| 002-C02 | 57 | 90 |
| 002-C09 | 77 | 95 |
| 002-D03 | 49 | 127 |
| 002-D08 | 101 | 140 |
| 002-D10 | 96 | 169 |
| 002-F01 | 101 | 188 |
| 002-F05 | 92 | 187 |
| 002-F07 | 78 | 224 |
| 002-H02 | 61 | 156 |
| 002-H03 | 82 | 124 |
| 003-A10 | 109 | 114 |
| 003-A11 | 84 | 128 |
| 003-D07 | 78 | 89 |
| 003-F08 | 98 | 108 |
| 002-A07 N113G | 117 | 114 |
| 002-A07 N113Q | 101 | 92 |
| 002-A07 N113S | 80 | 92 |
| 002-A07 S115A | 76 | 96 |
| 002-A07 S115G | 115 | 91 |
| 002-A07 S115N | 120 | 118 |

**Table 13 Effects of 002-A07 mutant antibodies on cell proliferation of human PBMC**

| antibody | 10 µg/mL | 1 µg/mL |
|---|---|---|
| mouse IgG | 100 | 100 |
| 5A6 | 91 | 95 |
| human IgG4 | 100 | 100 |
| 002-A07 | 102 | 101 |
| 001-B06 | 125 | 102 |
| 002-B05 | 121 | 103 |
| 002-B07 | 111 | 106 |
| 002-C02 | 113 | 109 |
| 002-C09 | 136 | 105 |
| 002-D03 | 114 | 107 |
| 002-D08 | 107 | 97 |
| 002-D10 | 112 | 97 |
| 002-F01 | 109 | 100 |
| 002-F05 | 106 | 103 |
| 002-F07 | 108 | 97 |
| 002-H02 | 105 | 96 |
| 002-H03 | 118 | 103 |
| 003-A10 | 112 | 110 |
| 003-A11 | 118 | 110 |
| 003-D07 | 121 | 97 |
| 003-F08 | 120 | 110 |
| 002-A07 N113G | 117 | 103 |
| 002-A07 N113Q | 123 | 103 |
| 002-A07 N113S | 119 | 108 |
| 002-A07 S115A | 126 | 108 |
| 002-A07 S115G | 145 | 97 |
| 002-A07 S115N | 121 | 87 |

### Experimental Example 12: Epitope mapping of 002-A07 mutant antibodies using alanine scanning

This experiment was performed in the same manner as Experimental Example 8. Since the 1st to 43rd, 63rd to 112th, 202nd and subsequent residues are transmembrane or intracellular domains (Levy, S. et al., Annu. Rev. Immunol. (1998) 16, 89-109), no alanine mutant was generated. Since the 156th, 157th, 175th, and 190th residues are cysteine residues, no alanine mutant was generated. The relative binding affinity for alanine mutants not listed in Table 14 was equal or slightly weak in all 002-A07 mutant antibodies compared to wild type human CD81 (relative binding affinity is ranged 40-100%).

**Table 14 Epitope mapping of 002-A07 mutant antibodies by alanine scanning**

| substitution | Y127F | V135A | D137A | A143T | H151A | G158S | T163A | A164T | L165A | S168A | V169A | L170A | K171A | N172A | L174A | I194A |
|---|---|---|---|---|---|---|---|---|---|---|---|---|---|---|---|---|
| 002-A07 | ○ | × | × | Δ | × | × | × | × | × | Δ | × | Δ | Δ | Δ | Δ | × |
| 002-A07 N113G | ○ | × | × | ○ | × | × | × | × | × | Δ | × | × | ○ | ○ | ○ | × |
| 002-A07 N113Q | ○ | × | × | ○ | × | × | × | × | × | Δ | × | Δ | ○ | ○ | ○ | × |
| 002-A07 N113S | ○ | × | × | ○ | × | × | × | × | × | Δ | × | × | ○ | ○ | ○ | × |
| 002-A07 S115A | Δ | × | × | Δ | × | × | × | × | × | A | × | × | ○ | ○ | ○ | × |
| 002-A07 S115G | ○ | × | × | ○ | Δ | × | × | × | × | Δ | × | Δ | ○ | Δ | ○ | × |
| 002-A07 S115N | ○ | × | Δ | ○ | ○ | Δ | ○ | × | × | Δ | × | ○ | ○ | ○ | ○ | Δ |
| 001-B06 | ○ | Δ | ○ | ○ | ○ | ○ | ○ | Δ | Δ | ○ | × | ○ | ○ | ○ | ○ | Δ |
| 002-B05 | ○ | × | × | ○ | Δ | × | Δ | × | × | ○ | × | Δ | ○ | ○ | ○ | Δ |
| 002-B07 | ○ | × | Δ | ○ | ○ | Δ | ○ | × | × | ○ | × | ○ | ○ | ○ | ○ | Δ |
| 002-C02 | ○ | × | × | ○ | ○ | Δ | ○ | × | × | ○ | × | ○ | ○ | ○ | ○ | Δ |
| 002-C09 | ○ | × | Δ | ○ | ○ | Δ | ○ | × | × | ○ | × | ○ | ○ | ○ | ○ | Δ |
| 002-D03 | ○ | × | × | ○ | ○ | Δ | ○ | × | × | ○ | × | ○ | ○ | ○ | ○ | Δ |
| 002-D08 | ○ | × | Δ | ○ | ○ | Δ | ○ | × | × | ○ | × | Δ | ○ | ○ | ○ | ○ |
| 002-D10 | ○ | × | ○ | ○ | ○ | ○ | ○ | × | × | ○ | × | ○ | ○ | ○ | ○ | Δ |
| 002-F01 | ○ | × | Δ | ○ | ○ | Δ | ○ | × | × | Δ | × | Δ | ○ | ○ | ○ | x |
| 002-F05 | ○ | × | Δ | ○ | ○ | Δ | ○ | × | × | × | × | ○ | ○ | ○ | ○ | × |
| 002-F07 | ○ | × | Δ | ○ | ○ | Δ | ○ | × | × | ○ | × | ○ | ○ | Δ | Δ | × |
| 002-H02 | ○ | × | Δ | ○ | ○ | ○ | ○ | × | × | ○ | × | ○ | ○ | ○ | ○ | Δ |
| 002-H03 | ○ | × | ○ | ○ | ○ | ○ | ○ | × | × | ○ | × | ○ | ○ | ○ | ○ | Δ |
| 003-A10 | ○ | Δ | ○ | ○ | ○ | ○ | ○ | Δ | Δ | ○ | × | ○ | Δ | × | Δ | Δ |
| 003-A11 | ○ | × | Δ | ○ | ○ | Δ | ○ | × | × | ○ | × | ○ | ○ | Δ | Δ | × |
| 003-D07 | ○ | × | × | ○ | Δ | × | Δ | × | × | ○ | × | Δ | ○ | × | Δ | × |
| 003-F08 | ○ | × | × | ○ | Δ | × | Δ | × | × | ○ | × | Δ | ○ | Δ | Δ | Δ |

| | | | | | | | | | | | | | | | | |
|---|---|---|---|---|---|---|---|---|---|---|---|---|---|---|---|---|
| Each symbol shows; ○: relative binding affinity is equal or slightly weak compared to wild type human CD81 (relative binding affinity is ranged 40- 100%). Δ: relative binding affinity is significantly weak compared to wild type human CD81 (relative binding affinity is ranged 20 - 40%). x: relative binding affinity is quite weak compared to wild type human CD81 (relative binding affinity is ranged under 20%). | | | | | | | | | | | | | | | | |

### Experimental Example 13: Epitope mapping of 002-A07 mutant antibodies using homolog-scanning

Amino acid residues thought to be important to the binding of 002-A07 mutant antibodies, judging from the results of alanine scanning, were subjected to homolog-scanning in the same manner as Experimental Example 9. As a result, the amino acid residues important to the binding shown below were identified respectively for the 002-A07 mutant antibodies. It was found that all 002-A07 mutant antibodies bind to the same epitope as with 002-A07.

| | |
|---|---|
| 002-A07 N113G : | V135, D137, H151, G158, A164, S168, V169, L170 |
| 002-A07 N113Q : | V135, D137, H151, G158, A164, S168, V169, L170 |
| 002-A07 N113S : | V135, D137, H151, G158, A164, S168, V169, L170 |
| 002-A07 S115A: | Y127, V135, D137, A143, H151, G158, A164, S168, V 169, L170 |
| 002-A07 S115G: | V135, D137, H151, G158, A164, S168, V169, L170, N 172 |
| 002-A07 S115N: | V135, D137, A164, S168, V169 |
| 001-B06 : | A164, V169 |
| 002-B05 : | V135, D137, H151, G158, A164, V169, L170 |
| 002-B07 : | V135, A164, V169 |
| 002-C02 : | V135, D137, A164, V169 |
| 002-C09 : | A164, V169 |
| 002-D03 : | V135, D137, A164, V169 |
| 002-D08 : | A164, V169 |
| 002-D10 : | A164, V169 |
| 002-F01 : | V135, D137, G158, A164, S168, V169 |
| 002-F05 : | V135, D137, A164, S168, V169 |
| 002-F07 : | V135, D137, A164, V169 |
| 002-H02 : | A164, V169 |
| 002-H03 : | A164, V169 |
| 003-A10 : | A164, V169 |
| 003-A11 : | A164, V169 |
| 003-D07 : | V135, D137, H151, G158, A164, V169 |
| 003-F08 : | V135, D137, H151, G158, A164, V169, L170 |

**Table 15 Epitope mapping of 002-A07 mutant antibodies by homolog-scanning**

| **substitution** | **Y127W** | **V135L** | **D137G** | **A143V** | **H151R** | **G158A** | **T163S** | **A164V** | **L165I** | **S168T** | **V169L** | **L170I** | **K171R** | **N172Q** | **L174I** | **I194L** |
|---|---|---|---|---|---|---|---|---|---|---|---|---|---|---|---|---|
| **002-A07** | - | × | × | ○ | × | × | ○ | × | ○ | × | × | Δ | ○ | Δ | ○ | ○ |
| **002-A07 N113G** | - | × | × | - | × | × | ○ | × | ○ | × | × | Δ | - | - | - | ○ |
| **002-A07 N113Q** | - | × | × | - | × | × | ○ | × | ○ | × | × | Δ | - | - | - | ○ |
| **002-A07 N113S** | - | × | × | - | × | × | ○ | × | ○ | × | × | Δ | - | - | - | ○ |
| **002-A07 S115A** | × | × | × | Δ | × | × | ○ | × | ○ | × | ○ | Δ | - | - | - | ○ |
| **002-A07 S115G** | - | × | × | - | × | × | ○ | × | ○ | × | × | Δ | - | ○ | - | ○ |
| **002-A07 S115N** | - | Δ | Δ | - | - | ○ | - | × | ○ | × | × | - | - | - | - | ○ |
| **001-B06** | - | ○ | - | - | - | - | - | Δ | ○ | - | Δ | - | - | - | - | ○ |
| **002-B05** | - | × | Δ | - | × | A | ○ | × | ○ | - | × | Δ | - | - | - | ○ |
| **002-B07** | - | Δ | ○ | - | - | ○ | - | × | ○ | - | × | - | - | - | - | ○ |
| **002-C02** | - | Δ | × | - | - | ○ | - | × | ○ | - | × | - | - | - | - | ○ |
| **002-C08** | - | ○ | ○ | - | - | ○ | - | × | ○ | - | × | - | - | - | - | ○ |
| **002-D03** | - | Δ | Δ | - | - | ○ | - | × | ○ | - | × | - | - | - | - | ○ |
| **002-D08** | - | ○ | ○ | - | - | ○ | - | × | ○ | - | Δ | ○ | - | - | - | - |
| **002-D10** | - | ○ | - | - | - | - | - | × | ○ | - | × | - | - | - | - | ○ |
| **002-F01** | - | Δ | Δ | - | - | Δ | - | × | ○ | × | × | ○ | - | - | - | ○ |
| **002-F05** | - | Δ | Δ | - | - | ○ | - | × | ○ | × | × | - | - | - | - | ○ |
| **002-F07** | - | Δ | Δ | - | - | ○ | - | × | ○ | - | × | - | - | ○ | ○ | ○ |
| **002-H02** | - | ○ | ○ | - | - | - | - | × | ○ | - | × | - | - | - | - | ○ |
| **002-H03** | - | ○ | - | - | - | - | - | × | ○ | - | Δ | - | - | - | - | ○ |
| **002-A10** | - | ○ | - | - | - | - | - | Δ | ○ | - | Δ | - | ○ | ○ | ○ | ○ |
| **003-A11** | - | ○ | ○ | - | - | ○ | - | × | ○ | - | × | - | - | ○ | ○ | ○ |
| **003-D07** | - | × | Δ | - | × | Δ | ○ | × | ○ | - | × | ○ | - | ○ | ○ | ○ |
| **003-F08** | - | × | × | - | × | Δ | ○ | × | ○ | - | × | Δ | - | ○ | ○ | ○ |

| | | | | | | | | | | | | | | | | |
|---|---|---|---|---|---|---|---|---|---|---|---|---|---|---|---|---|
| Each symbol shows; ○: relative binding affinity is equal or slightly weak compared to wild type human CD81 (relative binding affinity is ranged 40- 100%). Δ: relative binding affinity is significantly weak compared to wild type human CD81 (relative binding affinity is ranged 20 - 40%). ×: relative binding affinity is quite weak compared to wild type human CD81 (relative binding affinity is ranged under 20%). -: Not tested (since the mutant was judged to be unimportant to the binding based on the results of alanine scanning). | | | | | | | | | | | | | | | | |

### Example 3: Generating human IgG1-type anti-CD81 antibody

An EcoRI site and an XhoI site were added to each ends of the DNA fragment encodes L-chain peptide sequence of 002-A07 having a human IL-2 signal sequence added to the N-terminal portion thereof, and the fragment was inserted into the EcoRI-XhoI site of pcDNA3.1(+) (Invitrogen Co.) (a). Likewise, an EcoRI site and an XhoI site were added to each ends of the DNA fragment encodes H-chain peptide sequence of 002-A07 having a human IL-2 signal sequence added to the N-terminal portion thereof, and the fragment was inserted into the EcoRI-XhoI site of pcDNA3.1(+). Furthermore, the DNA fragment was amplified by PCR with the above-described plasmid incorporating the H-chain gene as the template, using the DNA primers shown below.
5' side DNA primer (a sequence containing EcoRI site portion): 5'-GGTGGAATTCCCACCATGTACAGGATGCAAC-3' (SEQ ID NO:161)
3' side DNA primer (a sequence containing XhoI site on pFUSE-CHIg-hG1 (Invitrogen Co.) and a partial sequence encoding the C-terminal region of the variable region of the H-chain of 002-A07): 5'-TGCACTCGAGACGGTGACCAGTGTACCTTGGCCCC-3' (SEQ ID NO:162)
After digestion with EcoRI and XhoI, the amplified DNA was inserted into the EcoRI-XhoI site of pFUSE-CHIg-hG1 to yield a converted-to-IgG1 H-chain expression plasmid (b). To prepare a human IgG1-type anti-CD81 antibody protein, the plasmids (a) and (b) were transiently introduced into CHO-S cells, and the cells were subjected to suspension culture. The culture supernatant was recovered, and the human IgG1-type anti-CD81 antibody was purified using a Protein A column. The nucleotide and amino acid sequences of L- and H-chains of the antibody are shown in the Sequence Listing.
L-chain:
   Nucleotide sequence: SEQ ID NO:163
   Amino acid sequence: SEQ ID NO:164
H-chain:
   Nucleotide sequence: SEQ ID NO:165
   Amino acid sequence: SEQ ID NO:166

### Experimental Example 14: Binding activity of human IgG1-type anti-CD81 antibody on cancer cells

Binding of a human IgG1-type anti-CD81 antibody to Jurkat E6.1 cells (Cat No. 88042803) derived from a human acute lymphatic leukemia patient and Ramos (RA1) cells (Cat No. EC85030802) derived from a human Burkitt's lymphoma patient was examined in the same manner as Experimental Example 1, except that human IgG (AbD Serotec Co.) was used as a control, and that a PE (phycoerythrin)-labeled anti-human Ig antibody (Beckman Coulter Co.) was used for the staining. The results of an analysis using FACS Calibur (BD Biosciences Co.) are shown in Table 16. The numerical values in the table are geometric means for FL2 in the FACS Calibur. As a result, human IgG1-type anti-CD81 antibody was confirmed as binding to Jurkat cells and Ramos cells.

**Table 16 Binding of human IgG1-type anti-CD81 antibody to Jurkat cells and Ramos cells**

| cell | Jurkat | | | | Ramos | | | |
|---|---|---|---|---|---|---|---|---|
| Antibody (µg/mL) | 10 | 1 | 0.1 | 0.01 | 10 | 1 | 0.1 | 0.01 |
| control IgG | 8 | 4 | 4 | 4 | 8 | 5 | 4 | 4 |
| human IgG1-type anti-human CD81 antibody | 2310 | 930 | 144 | 32 | 2129 | 1183 | 712 | 584 |

### Experimental Example 15: Cytotoxic effect (CDC: complement-dependent cytotoxicity) of human IgG1-type anti-CD81 antibody on cancer cells

After centrifugation at 4,000 rpm (4°C, 3 minutes), Jurkat cells and Ramos cells were recovered and suspended in CDC assay buffer (an RPMI1640 medium containing 20 mM Hepes and 0.1% bovine serum albumin). Viable cells were counted using Trypan Blue (GIBCO Co.); the cells were suspended in the CDC assay buffer to obtain a cell density of 10⁶ cells/mL. The cells suspended were dispended to a 96-well cell culture plate at 50 µL per well; the antibody shown in Table 17 was added at 50 µL per well, and the plate was incubated at 37°C for 30 minutes. A dry rabbit complement (CEDARRLANE Co.) was rehydrated with sterile distilled water and diluted 10 fold with the CDC assay buffer, after which 50 µL of the dilution was added to each well, and the plate was incubated at 37°C for 2 more hours. A 100 µL aliquot of the culture supernatant was mixed with 100 µL of the reaction liquid in an LDH assay kit (Cat. No. 744934001, Roche Co.), and they were reacted at room temperature for 30 minutes, after which absorbance at 490 nm wavelength was measured using a plate reader. CDC activity was calculated as the percent ratio to the LDH activity value obtained when the cells were completely killed by Triton X-100 treatment. As a result, the complement-dependent cytotoxic activity of the human IgG1-type anti-CD81 antibody on Jurkat cells and Ramos cells was confirmed.

**Table 17 Cytotoxic effects of human IgG1-type anti-CD81 antibody on Jurkat cells**

| cell | Jurkat | | | | | |
|---|---|---|---|---|---|---|
| antibody (ng/mL) | 10000 | 1000 | 100 | 10 | 1 | 0.1 |
| control IgG | 5.8 | 4.6 | 4.1 | 4.6 | 3.7 | 4.7 |
| human IgG1-type anti-human CD81 antibody | 84.9 | 73.3 | 42.6 | 6.4 | 5.9 | 6.3 |

**Table 18 Cytotoxic effects of human IgG1-type anti-CD81 antibody on Ramos cells**

| cell | Ramos | | | | | |
|---|---|---|---|---|---|---|
| antibody (ng/mL) | 10000 | 1000 | 100 | 10 | 1 | 0.1 |
| control IgG | 0.2 | 1.7 | 1.0 | 1.9 | 0.4 | -1.1 |
| human IgG1-type anti-human CD81 antibody | 71.7 | 71.4 | 69.4 | 49.3 | 26.0 | 9.9 |

### Industrial Applicability

The anti-human CD81 antibody of the present invention is useful for preventing, improving or treating inflammatory bowel diseases (IBD), diseases associated with T cell migration such as multiple sclerosis and psoriasis, or hematological cancer.

This application is based on a patent application No. 2010-272046 filed in Japan (filing date: December 6, 2010), the contents of which are incorporated in full herein.

## Claims

1. An anti-human CD81 antibody capable of binding to a peptide region consisting of the amino acid sequence of the amino acid numbers 80 to 175 in the amino acid sequence shown in SEQ ID NO:22.

2. The antibody of claim 1, wherein the peptide region consists of the amino acid sequence of the amino acid numbers 113 to 175.

3. The antibody of claim 1 or 2, wherein the binding affinity of the antibody to at least one human CD81 variant selected from the group consisting of the following (1) to (13) is less than 40% of that to the human CD81 having the amino acid sequence shown in SEQ ID NO:22.
(1) CD81 variant having the amino acid sequence shown in SEQ ID NO:22 wherein tyrosine at the amino acid number 127 is substituted with phenylalanine or tryptophan;
(2) CD81 variant having the amino acid sequence shown in SEQ ID NO:22 wherein alanine at the amino acid number 130 is substituted with threonine or valine;
(3) CD81 variant having the amino acid sequence shown in SEQ ID NO:22 wherein valine at the amino acid number 135 is substituted with alanine or leucine;
(4) CD81 variant having the amino acid sequence shown in SEQ ID NO:22 wherein aspartic acid at the amino acid number 137 is substituted with alanine or glutamic acid;
(5) CD81 variant having the amino acid sequence shown in SEQ ID NO:22 wherein alanine at the amino acid number 143 is substituted with threonine or valine;
(6) CD81 variant having the amino acid sequence shown in SEQ ID NO:22 wherein histidine at the amino acid number 151 is substituted with alanine or arginine;
(7) CD81 variant having the amino acid sequence shown in SEQ ID NO:22 wherein leucine at the amino acid number 154 is substituted with alanine or isoleucine;
(8) CD81 variant having the amino acid sequence shown in SEQ ID NO:22 wherein glycine at the amino acid number 158 is substituted with alanine or serine;
(9) CD81 variant having the amino acid sequence shown in SEQ ID NO:22 wherein alanine at the amino acid number 164 is substituted with threonine or valine;
(10) CD81 variant having the amino acid sequence shown in SEQ ID NO:22 wherein serine at the amino acid number 168 is substituted with alanine or threonine;
(11) CD81 variant having the amino acid sequence shown in SEQ ID NO:22 wherein valine at the amino acid number 169 is substituted with alanine or leucine;
(12) CD81 variant having the amino acid sequence shown in SEQ ID NO:22 wherein leucine at the amino acid number 170 is substituted with alanine or isoleucine; and
(13) CD81 variant having the amino acid sequence shown in SEQ ID NO:22 wherein asparagine at the amino acid number 172 is substituted with alanine or glutamine.

4. The antibody of any one of claims 1 to 3, wherein the binding affinity of the antibody to each of the above-identified human CD81 variants (9) and (11) is less than 40% of that to the human CD81 having the amino acid sequence shown in SEQ ID NO:22.

5. An antibody having a binding property equivalent to that of the antibody of any one of claims 1 to 4, or binding to the human CD81 having the amino acid sequence shown in SEQ ID NO:22 competitively with the antibody of any one of claims 1 to 4.

6. An antibody binding to the human CD81 having the amino acid sequence shown in SEQ ID NO:22 competitively with the antibody of any one of claims 1 to 4, and having a suppressive effect of T cell migration.

7. An anti-human CD81 antibody, which comprises all 6 CDRs described in any one of the following groups 1 to 24.
Group 1
(a-1) a CDR comprising the amino acid sequence shown in SEQ ID NO:1,
(b-1) a CDR comprising the amino acid sequence shown in SEQ ID NO:2,
(c-1) a CDR comprising the amino acid sequence shown in SEQ ID NO:3,
(d-1) a CDR comprising the amino acid sequence shown in SEQ ID NO:4,
(e-1) a CDR comprising the amino acid sequence shown in SEQ ID NO:5, and
(f-1) a CDR comprising the amino acid sequence shown in SEQ ID NO:6
Group 2
(a-2) a CDR comprising the amino acid sequence shown in SEQ ID NO:1,
(b-2) a CDR comprising the amino acid sequence shown in SEQ ID NO:2,
(c-2) a CDR comprising the amino acid sequence shown in SEQ ID NO:37,
(d-2) a CDR comprising the amino acid sequence shown in SEQ ID NO:4,
(e-2) a CDR comprising the amino acid sequence shown in SEQ ID NO:5, and
(f-2) a CDR comprising the amino acid sequence shown in SEQ ID NO:6
Group 3
(a-3) a CDR comprising the amino acid sequence shown in SEQ ID NO:1,
(b-3) a CDR comprising the amino acid sequence shown in SEQ ID NO:2,
(c-3) a CDR comprising the amino acid sequence shown in SEQ ID NO:40,
(d-3) a CDR comprising the amino acid sequence shown in SEQ ID NO:4,
(e-3) a CDR comprising the amino acid sequence shown in SEQ ID NO:5, and
(f-3) a CDR comprising the amino acid sequence shown in SEQ ID NO:6
Group 4
(a-4) a CDR comprising the amino acid sequence shown in SEQ ID NO:1,
(b-4) a CDR comprising the amino acid sequence shown in SEQ ID NO:2,
(c-4) a CDR comprising the amino acid sequence shown in SEQ ID NO:43,
(d-4) a CDR comprising the amino acid sequence shown in SEQ ID NO:4,
(e-4) a CDR comprising the amino acid sequence shown in SEQ ID NO:5, and
(f-4) a CDR comprising the amino acid sequence shown in SEQ ID NO:6
Group 5
(a-5) a CDR comprising the amino acid sequence shown in SEQ ID NO:1,
(b-5) a CDR comprising the amino acid sequence shown in SEQ ID NO:2,
(c-5) a CDR comprising the amino acid sequence shown in SEQ ID NO:46,
(d-5) a CDR comprising the amino acid sequence shown in SEQ ID NO:4,
(e-5) a CDR comprising the amino acid sequence shown in SEQ ID NO:5, and
(f-5) a CDR comprising the amino acid sequence shown in SEQ ID NO:6
Group 6
(a-6) a CDR comprising the amino acid sequence shown in SEQ ID NO:1,
(b-6) a CDR comprising the amino acid sequence shown in SEQ ID NO:2,
(c-6) a CDR comprising the amino acid sequence shown in SEQ ID NO:49,
(d-6) a CDR comprising the amino acid sequence shown in SEQ ID NO:4,
(e-6) a CDR comprising the amino acid sequence shown in SEQ ID NO:5, and
(f-6) a CDR comprising the amino acid sequence shown in SEQ ID NO:6
Group 7
(a-7) a CDR comprising the amino acid sequence shown in SEQ ID NO:1,
(b-7) a CDR comprising the amino acid sequence shown in SEQ ID NO:2,
(c-7) a CDR comprising the amino acid sequence shown in SEQ ID NO:52,
(d-7) a CDR comprising the amino acid sequence shown in SEQ ID NO:4,
(e-7) a CDR comprising the amino acid sequence shown in SEQ ID NO:5, and
(f-7) a CDR comprising the amino acid sequence shown in SEQ ID NO:6
Group 8
(a-8) a CDR comprising the amino acid sequence shown in SEQ ID NO:1,
(b-8) a CDR comprising the amino acid sequence shown in SEQ ID NO:2,
(c-8) a CDR comprising the amino acid sequence shown in SEQ ID NO:43,
(d-8) a CDR comprising the amino acid sequence shown in SEQ ID NO:4,
(e-8) a CDR comprising the amino acid sequence shown in SEQ ID NO:5, and
(f-8) a CDR comprising the amino acid sequence shown in SEQ ID NO:55
Group 9
(a-9) a CDR comprising the amino acid sequence shown in SEQ ID NO:60,
(b-9) a CDR comprising the amino acid sequence shown in SEQ ID NO:2,
(c-9) a CDR comprising the amino acid sequence shown in SEQ ID NO:3,
(d-9) a CDR comprising the amino acid sequence shown in SEQ ID NO:4,
(e-9) a CDR comprising the amino acid sequence shown in SEQ ID NO:5, and
(f-9) a CDR comprising the amino acid sequence shown in SEQ ID NO:61
Group 10
(a-10) a CDR comprising the amino acid sequence shown in SEQ ID NO:1,
(b-10) a CDR comprising the amino acid sequence shown in SEQ ID NO:2,
(c-10) a CDR comprising the amino acid sequence shown in SEQ ID NO:66,
(d-10) a CDR comprising the amino acid sequence shown in SEQ ID NO:4,
(e-10) a CDR comprising the amino acid sequence shown in SEQ ID NO:5, and
(f-10) a CDR comprising the amino acid sequence shown in SEQ ID NO:6
Group 11
(a-11) a CDR comprising the amino acid sequence shown in SEQ ID NO:1,
(b-11) a CDR comprising the amino acid sequence shown in SEQ ID NO:2,
(c-11) a CDR comprising the amino acid sequence shown in SEQ ID NO:3,
(d-11) a CDR comprising the amino acid sequence shown in SEQ ID NO:4,
(e-11) a CDR comprising the amino acid sequence shown in SEQ ID NO:69, and
(f-11) a CDR comprising the amino acid sequence shown in SEQ ID NO:70
Group 12
(a-12) a CDR comprising the amino acid sequence shown in SEQ ID NO:60,
(b-12) a CDR comprising the amino acid sequence shown in SEQ ID NO:2,
(c-12) a CDR comprising the amino acid sequence shown in SEQ ID NO:66,
(d-12) a CDR comprising the amino acid sequence shown in SEQ ID NO:4,
(e-12) a CDR comprising the amino acid sequence shown in SEQ ID NO:5, and
(f-12) a CDR comprising the amino acid sequence shown in SEQ ID NO:6
Group 13
(a-13) a CDR comprising the amino acid sequence shown in SEQ ID NO:1,
(b-13) a CDR comprising the amino acid sequence shown in SEQ ID NO:2,
(c-13) a CDR comprising the amino acid sequence shown in SEQ ID NO:3,
(d-13) a CDR comprising the amino acid sequence shown in SEQ ID NO:77,
(e-13) a CDR comprising the amino acid sequence shown in SEQ ID NO:5, and
(f-13) a CDR comprising the amino acid sequence shown in SEQ ID NO:6
Group 14
(a-14) a CDR comprising the amino acid sequence shown in SEQ ID NO:80,
(b-14) a CDR comprising the amino acid sequence shown in SEQ ID NO:2,
(c-14) a CDR comprising the amino acid sequence shown in SEQ ID NO:3,
(d-14) a CDR comprising the amino acid sequence shown in SEQ ID NO:4,
(e-14) a CDR comprising the amino acid sequence shown in SEQ ID NO:81, and
(f-14) a CDR comprising the amino acid sequence shown in SEQ ID NO:6
Group 15
(a-15) a CDR comprising the amino acid sequence shown in SEQ ID NO:1,
(b-15) a CDR comprising the amino acid sequence shown in SEQ ID NO:2,
(c-15) a CDR comprising the amino acid sequence shown in SEQ ID NO:66,
(d-15) a CDR comprising the amino acid sequence shown in SEQ ID NO:4,
(e-15) a CDR comprising the amino acid sequence shown in SEQ ID NO:5, and
(f-15) a CDR comprising the amino acid sequence shown in SEQ ID NO:6
Group 16
(a-16) a CDR comprising the amino acid sequence shown in SEQ ID NO:1,
(b-16) a CDR comprising the amino acid sequence shown in SEQ ID NO:2,
(c-16) a CDR comprising the amino acid sequence shown in SEQ ID NO:90,
(d-16) a CDR comprising the amino acid sequence shown in SEQ ID NO:4,
(e-16) a CDR comprising the amino acid sequence shown in SEQ ID NO:5, and
(f-16) a CDR comprising the amino acid sequence shown in SEQ ID NO:6
Group 17
(a-17) a CDR comprising the amino acid sequence shown in SEQ ID NO:1,
(b-17) a CDR comprising the amino acid sequence shown in SEQ ID NO:2,
(c-17) a CDR comprising the amino acid sequence shown in SEQ ID NO:52,
(d-17) a CDR comprising the amino acid sequence shown in SEQ ID NO:4,
(e-17) a CDR comprising the amino acid sequence shown in SEQ ID NO:5, and
(f-17) a CDR comprising the amino acid sequence shown in SEQ ID NO:93
Group 18
(a-18) a CDR comprising the amino acid sequence shown in SEQ ID NO:98,
(b-18) a CDR comprising the amino acid sequence shown in SEQ ID NO:2,
(c-18) a CDR comprising the amino acid sequence shown in SEQ ID NO:3,
(d-18) a CDR comprising the amino acid sequence shown in SEQ ID NO:4,
(e-18) a CDR comprising the amino acid sequence shown in SEQ ID NO:5, and
(f-18) a CDR comprising the amino acid sequence shown in SEQ ID NO:99
Group 19
(a-19) a CDR comprising the amino acid sequence shown in SEQ ID NO:60,
(b-19) a CDR comprising the amino acid sequence shown in SEQ ID NO:2,
(c-19) a CDR comprising the amino acid sequence shown in SEQ ID NO:3,
(d-19) a CDR comprising the amino acid sequence shown in SEQ ID NO:4,
(e-19) a CDR comprising the amino acid sequence shown in SEQ ID NO:5, and
(f-19) a CDR comprising the amino acid sequence shown in SEQ ID NO:99
Group 20
(a-20) a CDR comprising the amino acid sequence shown in SEQ ID NO:1,
(b-20) a CDR comprising the amino acid sequence shown in SEQ ID NO:2,
(c-20) a CDR comprising the amino acid sequence shown in SEQ ID NO:90,
(d-20) a CDR comprising the amino acid sequence shown in SEQ ID NO:4,
(e-20) a CDR comprising the amino acid sequence shown in SEQ ID NO:5, and
(f-20) a CDR comprising the amino acid sequence shown in SEQ ID NO:6
Group 21
(a-21) a CDR comprising the amino acid sequence shown in SEQ ID NO:1,
(b-21) a CDR comprising the amino acid sequence shown in SEQ ID NO:2,
(c-21) a CDR comprising the amino acid sequence shown in SEQ ID NO:3,
(d-21) a CDR comprising the amino acid sequence shown in SEQ ID NO:4,
(e-21) a CDR comprising the amino acid sequence shown in SEQ ID NO:5, and
(f-21) a CDR comprising the amino acid sequence shown in SEQ ID NO:55
Group 22
(a-22) a CDR comprising the amino acid sequence shown in SEQ ID NO:1,
(b-22) a CDR comprising the amino acid sequence shown in SEQ ID NO:2,
(c-22) a CDR comprising the amino acid sequence shown in SEQ ID NO:66,
(d-22) a CDR comprising the amino acid sequence shown in SEQ ID NO:110,
(e-22) a CDR comprising the amino acid sequence shown in SEQ ID NO:5, and
(f-22) a CDR comprising the amino acid sequence shown in SEQ ID NO:6
Group 23
(a-23) a CDR comprising the amino acid sequence shown in SEQ ID NO:1,
(b-23) a CDR comprising the amino acid sequence shown in SEQ ID NO:2,
(c-23) a CDR comprising the amino acid sequence shown in SEQ ID NO:3,
(d-23) a CDR comprising the amino acid sequence shown in SEQ ID NO:4,
(e-23) a CDR comprising the amino acid sequence shown in SEQ ID NO:5, and
(f-23) a CDR comprising the amino acid sequence shown in SEQ ID NO:115
Group 24
(a-24) a CDR comprising the amino acid sequence shown in SEQ ID NO:1,
(b-24) a CDR comprising the amino acid sequence shown in SEQ ID NO:2,
(c-24) a CDR comprising the amino acid sequence shown in SEQ ID NO:90,
(d-24) a CDR comprising the amino acid sequence shown in SEQ ID NO:4,
(e-24) a CDR comprising the amino acid sequence shown in SEQ ID NO:120, and
(f-24) a CDR comprising the amino acid sequence shown in SEQ ID NO:6.

8. The antibody of claim 7, which comprises the combination of the light chain variable region and the heavy chain variable region described in any one of the following groups 25 to 48.
Group 25
(g-1) a light chain variable region comprising the above-identified CDRs (a-1) to (c-1); and
(h-1) a heavy chain variable region comprising the above-identified CDRs (d-1) to (f-1),
Group 26
(g-2) a light chain variable region comprising the above-identified CDRs (a-2) to (c-2); and
(h-2) a heavy chain variable region comprising the above-identified CDRs (d-2) to (f-2),
Group 27
(g-3) a light chain variable region comprising the above-identified CDRs (a-3) to (c-3); and
(h-3) a heavy chain variable region comprising the above-identified CDRs (d-3) to (f-3),
Group 28
(g-4) a light chain variable region comprising the above-identified CDRs (a-4) to (c-4); and
(h-4) a heavy chain variable region comprising the above-identified CDRs (d-4) to (f-4),
Group 29
(g-5) a light chain variable region comprising the above-identified CDRs (a-5) to (c-5); and
(h-5) a heavy chain variable region comprising the above-identified CDRs (d-5) to (f-5),
Group 30
(g-6) a light chain variable region comprising the above-identified CDRs (a-6) to (c-6); and
(h-6) a heavy chain variable region comprising the above-identified CDRs (d-6) to (f-6),
Group 31
(g-7) a light chain variable region comprising the above-identified CDRs (a-7) to (c-7); and
(h-7) a heavy chain variable region comprising the above-identified CDRs (d-7) to (f-7),
Group 32
(g-8) a light chain variable region comprising the above-identified CDRs (a-8) to (c-8); and
(h-8) a heavy chain variable region comprising the above-identified CDRs (d-8) to (f-8),
Group 33
(g-9) a light chain variable region comprising the above-identified CDRs (a-9) to (c-9); and
(h-9) a heavy chain variable region comprising the above-identified CDRs (d-9) to (f-9),
Group 34
(g-10) a light chain variable region comprising the above-identified CDRs (a-10) to (c-10); and
(h-10) a heavy chain variable region comprising the above-identified CDRs (d-10) to (f-10),
Group 35
(g-11) a light chain variable region comprising the above-identified CDRs (a-11) to (c-11); and
(h-11) a heavy chain variable region comprising the above-identified CDRs (d-11) to (f-11),
Group 36
(g-12) a light chain variable region comprising the above-identified CDRs (a-12) to (c-12); and
(h-12) a heavy chain variable region comprising the above-identified CDRs (d-12) to (f-12),
Group 37
(g-13) a light chain variable region comprising the above-identified CDRs (a-13) to (c-13); and
(h-13) a heavy chain variable region comprising the above-identified CDRs (d-13) to (f-13),
Group 38
(g-14) a light chain variable region comprising the above-identified CDRs (a-14) to (c-14); and
(h-14) a heavy chain variable region comprising the above-identified CDRs (d-14) to (f-14),
Group 39
(g-15) a light chain variable region comprising the above-identified CDRs (a-15) to (c-15); and
(h-15) a heavy chain variable region comprising the above-identified CDRs (d-15) to (f-15),
Group 40
(g-16) a light chain variable region comprising the above-identified CDRs (a-16) to (c-16); and
(h-16) a heavy chain variable region comprising the above-identified CDRs (d-16) to (f-16),
Group 41
(g-17) a light chain variable region comprising the above-identified CDRs (a-17) to (c-17); and
(h-17) a heavy chain variable region comprising the above-identified CDRs (d-17) to (f-17),
Group 42
(g-18) a light chain variable region comprising the above-identified CDRs (a-18) to (c-18); and
(h-18) a heavy chain variable region comprising the above-identified CDRs (d-18) to (f-18),
Group 43
(g-19) a light chain variable region comprising the above-identified CDRs (a-19) to (c-19); and
(h-19) a heavy chain variable region comprising the above-identified CDRs (d-19) to (f-19),
Group 44
(g-20) a light chain variable region comprising the above-identified CDRs (a-20) to (c-20); and
(h-20) a heavy chain variable region comprising the above-identified CDRs (d-20) to (f-20),
Group 45
(g-21) a light chain variable region comprising the above-identified CDRs (a-21) to (c-21); and
(h-21) a heavy chain variable region comprising the above-identified CDRs (d-21) to (f-21),
Group 46
(g-22) a light chain variable region comprising the above-identified CDRs (a-22) to (c-22); and
(h-22) a heavy chain variable region comprising the above-identified CDRs (d-22) to (f-22),
Group 47
(g-23) a light chain variable region comprising the above-identified CDRs (a-23) to (c-23); and
(h-23) a heavy chain variable region comprising the above-identified CDRs (d-23) to (f-23),
Group 48
(g-24) a light chain variable region comprising the above-identified CDRs (a-24) to (c-24); and
(h-24) a heavy chain variable region comprising the above-identified CDRs (d-24) to (f-24).

9. The antibody of claim 8, which comprises the combination of the light chain variable region and the heavy chain variable region described in any one of the following groups 49 to 72.
Group 49
(i-1) a light chain variable region comprising the amino acid sequence shown in SEQ ID NO:8; and
(j-1) a heavy chain variable region comprising amino acid sequence shown in SEQ ID NO:10,
Group 50
(i-2) a light chain variable region comprising the amino acid sequence shown in SEQ ID NO:38; and
(j-2) a heavy chain variable region comprising amino acid sequence shown in SEQ ID NO:10,
Group 51
(i-3) a light chain variable region comprising the amino acid sequence shown in SEQ ID NO:41; and
(j-3) a heavy chain variable region comprising amino acid sequence shown in SEQ ID NO:10,
Group 52
(i-4) a light chain variable region comprising the amino acid sequence shown in SEQ ID NO:44; and
(j-4) a heavy chain variable region comprising amino acid sequence shown in SEQ ID NO:10,
Group 53
(i-5) a light chain variable region comprising the amino acid sequence shown in SEQ ID NO:47; and
(j-5) a heavy chain variable region comprising amino acid sequence shown in SEQ ID NO:10,
Group 54
(i-6) a light chain variable region comprising amino acid sequence shown in SEQ ID NO:50; and
(j-6) a heavy chain variable region comprising amino acid sequence shown in SEQ ID NO:10,
Group 55
(i-7) a light chain variable region comprising amino acid sequence shown in SEQ ID NO:53; and
(j-7) a heavy chain variable region comprising amino acid sequence shown in SEQ ID NO:10,
Group 56
(i-8) a light chain variable region comprising amino acid sequence shown in SEQ ID NO:56; and
(j-8) a heavy chain variable region comprising amino acid sequence shown in SEQ ID NO:57,
Group 57
(i-9) a light chain variable region comprising amino acid sequence shown in SEQ ID NO:62; and
(j-9) a heavy chain variable region comprising amino acid sequence shown in SEQ ID NO:63,
Group 58
(i-10) a light chain variable region comprising amino acid sequence shown in SEQ ID NO:67; and
(j-10) a heavy chain variable region comprising amino acid sequence shown in SEQ ID NO:10,
Group 59
(i-11) a light chain variable region comprising amino acid sequence shown in SEQ ID NO:71; and
(j-11) a heavy chain variable region comprising amino acid sequence shown in SEQ ID NO:72,
Group 60
(i-12) a light chain variable region comprising amino acid sequence shown in SEQ ID NO:75; and
(j-12) a heavy chain variable region comprising amino acid sequence shown in SEQ ID NO:10,
Group 61
(i-13) a light chain variable region comprising amino acid sequence shown in SEQ ID NO:8; and
(j-13) a heavy chain variable region comprising amino acid sequence shown in SEQ ID NO:78,
Group 62
(i-14) a light chain variable region comprising amino acid sequence shown in SEQ ID NO:82; and
(j-14) a heavy chain variable region comprising amino acid sequence shown in SEQ ID NO:83,
Group 63
(i-15) a light chain variable region comprising amino acid sequence shown in SEQ ID NO:86; and
(j-15) a heavy chain variable region comprising amino acid sequence shown in SEQ ID NO:87,
Group 64
(i-16) a light chain variable region comprising amino acid sequence shown in SEQ ID NO:91; and
(j-16) a heavy chain variable region comprising amino acid sequence shown in SEQ ID NO:10,
Group 65
(i-17) a light chain variable region comprising amino acid sequence shown in SEQ ID NO:94; and
(j-17) a heavy chain variable region comprising amino acid sequence shown in SEQ ID NO:95,
Group 66
(i-18) a light chain variable region comprising amino acid sequence shown in SEQ ID NO:100; and
(j-18) a heavy chain variable region comprising amino acid sequence shown in SEQ ID NO:101,
Group 67
(i-19) a light chain variable region comprising amino acid sequence shown in SEQ ID NO:104; and
(j-19) a heavy chain variable region comprising amino acid sequence shown in SEQ ID NO:101,
Group 68
(i-20) a light chain variable region comprising amino acid sequence shown in SEQ ID NO:106; and
(j-20) a heavy chain variable region comprising amino acid sequence shown in SEQ ID NO:10,
Group 69
(i-21) a light chain variable region comprising amino acid sequence shown in SEQ ID NO:108; and
(j-21) a heavy chain variable region comprising amino acid sequence shown in SEQ ID NO:57,
Group 70
(i-22) a light chain variable region comprising amino acid sequence shown in SEQ ID NO:111; and
(j-22) a heavy chain variable region comprising amino acid sequence shown in SEQ ID NO:112,
Group 71
(i-23) a light chain variable region comprising amino acid sequence shown in SEQ ID NO:116; and
(j-23) a heavy chain variable region comprising amino acid sequence shown in SEQ ID NO:117,
Group 72
(i-24) a light chain variable region comprising amino acid sequence shown in SEQ ID NO:121; and
(j-24) a heavy chain variable region comprising amino acid sequence shown in SEQ ID NO:122.

10. The antibody of claim 8 or 9, which comprises the combination of the light chain and the heavy chain described in any one of the following groups 73 to 96.
Group 73
(k-1) a light chain comprising the amino acid sequence shown in SEQ ID NO:26; and
(1-1) a heavy chain comprising the amino acid sequence shown in SEQ ID NO:28,
Group 74
(k-2) a light chain comprising the amino acid sequence shown in SEQ ID NO:39; and
(1-2) a heavy chain comprising the amino acid sequence shown in SEQ ID NO:28,
Group 75
(k-3) a light chain comprising the amino acid sequence shown in SEQ ID NO:42; and
(1-3) a heavy chain comprising the amino acid sequence shown in SEQ ID NO:28,
Group 76
(k-4) a light chain comprising the amino acid sequence shown in SEQ ID NO:45; and
(1-4) a heavy chain comprising the amino acid sequence shown in SEQ ID NO:28,
Group 77
(k-5) a light chain comprising the amino acid sequence shown in SEQ ID NO:48; and
(1-5) a heavy chain comprising the amino acid sequence shown in SEQ ID NO:28,
Group 78
(k-6) a light chain comprising the amino acid sequence shown in SEQ ID NO:51; and
(1-6) a heavy chain comprising the amino acid sequence shown in SEQ ID NO:28,
Group 79
(k-7) a light chain comprising the amino acid sequence shown in SEQ ID NO:54; and
(1-7) a heavy chain comprising the amino acid sequence shown in SEQ ID NO:28,
Group 80
(k-8) a light chain comprising the amino acid sequence shown in SEQ ID NO:58; and
(1-8) a heavy chain comprising the amino acid sequence shown in SEQ ID NO:59,
Group 81
(k-9) a light chain comprising the amino acid sequence shown in SEQ ID NO:64; and
(1-9) a heavy chain comprising the amino acid sequence shown in SEQ ID NO:65,
Group 82
(k-10) a light chain comprising the amino acid sequence shown in SEQ ID NO:68; and
(1-10) a heavy chain comprising the amino acid sequence shown in SEQ ID NO:28,
Group 83
(k-11) a light chain comprising the amino acid sequence shown in SEQ ID NO:73; and
(1-11) a heavy chain comprising the amino acid sequence shown in SEQ ID NO:74,
Group 84
(k-12) a light chain comprising the amino acid sequence shown in SEQ ID NO:76; and
(1-12) a heavy chain comprising the amino acid sequence shown in SEQ ID NO:28,
Group 85
(k-13) a light chain comprising the amino acid sequence shown in SEQ ID NO:26; and
(1-13) a heavy chain comprising the amino acid sequence shown in SEQ ID NO:79,
Group 86
(k-14) a light chain comprising the amino acid sequence shown in SEQ ID NO:84; and
(1-14) a heavy chain comprising the amino acid sequence shown in SEQ ID NO:85,
Group 87
(k-15) a light chain comprising the amino acid sequence shown in SEQ ID NO:88; and
(1-15) a heavy chain comprising the amino acid sequence shown in SEQ ID NO:89,
Group 88
(k-16) a light chain comprising the amino acid sequence shown in SEQ ID NO:92; and
(1-16) a heavy chain comprising the amino acid sequence shown in SEQ ID NO:28,
Group 89
(k-17) a light chain comprising the amino acid sequence shown in SEQ ID NO:96; and
(1-17) a heavy chain comprising the amino acid sequence shown in SEQ ID NO:97,
Group 90
(k-18) a light chain comprising the amino acid sequence shown in SEQ ID NO:102; and
(1-18) a heavy chain comprising the amino acid sequence shown in SEQ ID NO:103,
Group 91
(k-19) a light chain comprising the amino acid sequence shown in SEQ ID NO:105; and
(1-19) a heavy chain comprising the amino acid sequence shown in SEQ ID NO:103,
Group 92
(k-20) a light chain comprising the amino acid sequence shown in SEQ ID NO:107; and
(1-20) a heavy chain comprising the amino acid sequence shown in SEQ ID NO:28,
Group 93
(k-21) a light chain comprising the amino acid sequence shown in SEQ ID NO:109; and
(1-21) a heavy chain comprising the amino acid sequence shown in SEQ ID NO:59,
Group 94
(k-22) a light chain comprising the amino acid sequence shown in SEQ ID NO:113; and
(1-22) a heavy chain comprising the amino acid sequence shown in SEQ ID NO:114,
Group 95
(k-23) a light chain comprising the amino acid sequence shown in SEQ ID NO:118; and
(1-23) a heavy chain comprising the amino acid sequence shown in SEQ ID NO:119,
Group 96
(k-24) a light chain comprising the amino acid sequence shown in SEQ ID NO:123; and
(1-24) a heavy chain comprising the amino acid sequence shown in SEQ ID NO:124.

11. An antibody binding to the human CD81 having the amino acid sequence shown in SEQ ID NO:22 competitively with the antibody of any one of claims 7 to 10.

12. The antibody of claim 11, which has a suppressive effect of T cell migration.

13. An anti-human CD81 antibody, wherein the antibody comprises one or more of CDRs described in any one of the groups 1 to 24 in claim 7 and binds to the human CD81 having the amino acid sequence shown in SEQ ID NO:22 competitively with the antibody comprising all 6 CDRs described in said group.

14. An anti-human CD81 antibody, wherein the antibody has a 90% sequence homology with any one of antibodies of claim 7 and binds to the human CD81 having the amino acid sequence shown in SEQ ID NO:22 competitively with said antibody.

15. An anti-human CD81 antibody comprising:
(a-25) a CDR comprising the amino acid sequence shown in SEQ ID NO:11;
(b-25) a CDR comprising the amino acid sequence shown in SEQ ID NO:12;
(c-25) a CDR comprising the amino acid sequence shown in SEQ ID NO:13;
(d-25) a CDR comprising the amino acid sequence shown in SEQ ID NO:14;
(e-25) a CDR comprising the amino acid sequence shown in SEQ ID NO:15; and
(f-25) a CDR comprising the amino acid sequence shown in SEQ ID NO:16.

16. The antibody of claim 15, which comprises:
(g-25) a light chain variable region comprising the above-identified CDRs (a-25) to (c-25); and
(h-25) a heavy chain variable region comprising the above-identified CDRs (d-25) to (f-25).

17. The antibody of claim 16, which comprises:
(i-25) a light chain variable region comprising the amino acid sequence shown in SEQ ID NO:18; and
(j-25) a heavy chain variable region comprising the amino acid sequence shown in SEQ ID NO:20.

18. The antibody of claim 16 or 17, which comprises:
(k-25) a light chain comprising the amino acid sequence shown in SEQ ID NO:30; and
(1-25) a heavy chain comprising the amino acid sequence shown in SEQ ID NO:32

19. An antibody binding to the human CD81 having the amino acid sequence shown in SEQ ID NO:22 competitively with the antibody of any one of claims 15 to 18.

20. The antibody of claim 19, which has a suppressive effect of T cell migration.

21. An anti-human CD81 antibody, wherein the antibody comprises one or more of CDRs in claim 15 and binds to the human CD81 having the amino acid sequence shown in SEQ ID NO:22 competitively with the antibody described in claim 15.

22. An anti-human CD81 antibody, wherein the antibody has a 90% sequence homology with the antibody described in claim 15 and binds to the human CD81 having the amino acid sequence shown in SEQ ID NO:22 competitively with said antibody.

23. The antibody of any one of claims 1 to 6, 11 to 14 and 19 to 22 which is a humanized or human antibody.

24. The antibody of any one of claims 7 to 10 and 15 to 18 which is a humanized or human antibody.

25. The polynucleotide comprising a nucleotide sequence that encodes a heavy chain variable region and a light chain variable region of the antibody of any one of claims 1 to 24.

26. A combination of the polynucleotide comprising a nucleotide sequence that encodes a heavy chain variable region of the antibody of any one of claims 1 to 6, 11 to 14 and 19 to 23, and the polynucleotide comprising a nucleotide sequence that encodes a light chain variable region of the antibody of any one of claims 1 to 6, 11 to 14 and 19 to 23.

27. A combination of the polynucleotide comprising a nucleotide sequence that encodes a heavy chain variable region of the antibody of any one of claims 7 to 10, 15 to 18 and 24, and the polynucleotide comprising a nucleotide sequence that encodes the corresponding light chain variable region of the antibody of any one of claims 7 to 10, 15 to 18 and 24.

28. The polynucleotide comprising a nucleotide sequence that encodes a heavy chain and a light chain of the antibody of any one of claims 1 to 24.

29. A combination of the polynucleotide comprising a nucleotide sequence that encodes a heavy chain of the antibody of any one of claims 1 to 6, 11 to 14 and 19 to 23, and the polynucleotide comprising a nucleotide sequence that encodes a light chain of the antibody of any one of claims 1 to 6, 11 to 14 and 19 to 23.

30. A combination of the polynucleotide comprising a nucleotide sequence that encodes a heavy chain of the antibody of any one of claims 7 to 10, 15 to 18 and 24, and the polynucleotide comprising a nucleotide sequence that encodes the corresponding light chain of the antibody of any one of claims 7 to 10, 15 to 18 and 24.

31. An expression vector comprising the polynucleotide of claim 25 or 28.

32. A recombinant cell transformed with the expression vector of claim 31.

33. A recombinant cell transformed with the expression vector comprising the polynucleotide comprising a nucleotide sequence that encodes a heavy chain of the antibody of any one of claims 1 to 6, 11 to 14 and 19 to 23, and with the expression vector comprising the polynucleotide comprising a nucleotide sequence that encodes a light chain of the antibody of any one of claims 1 to 6, 11 to 14 and 19 to 23.

34. A recombinant cell transformed with the expression vector comprising the polynucleotide comprising a nucleotide sequence that encodes the heavy chain of the antibody of any one of claims 7 to 10, 15 to 18 and 24, and with the expression vector comprising the polynucleotide comprising a nucleotide sequence that encodes the corresponding light chain of the antibody of any one of claims 7 to 10, 15 to 18 and 24.

35. A method of producing an anti-human CD81 antibody, comprising culturing the recombinant cell of any one of claims 32 to 34, and recovering the antibody from the culture obtained.

36. A pharmaceutical composition comprising the antibody of any one of claims 1 to 24.

37. **38.** An agent for the prophylaxis, improvement or treatment of a disease selected from inflammatory bowel disease, multiple sclerosis, psoriasis and hematological cancer comprising the antibody of any one of claims 1 to 24.
